# FASCICULE DE BREVET EUROPEEN

(11) **EP 1 972 328 B1**
(45) Date de publication et mention de la délivrance du brevet: **09.06.2010**
(21) Numéro de dépôt: 08152086.8
(22) Date de dépôt: 28.02.2008
(51) Int. Cl.: A61K 8/49, A61Q 5/06

(54) **Utilisation pour la coloration des fibres kératiniques d'une composition comprenant un composé halochromique et / ou le colorant correspondant à ce composé**
Verwendung einer Zusammensetzung für die Färbung von Keratinfasern, die einen halochromen Bestandteil und/oder den Farbstoff enthält, der diesem Bestandteil entspricht
Use of a composition for dyeing keratinous fibres comprising a halochromic compound and/or the dye corresponding to this compound

(30) Priorité: 20.03.2007 FR 0753924
(43) Date de publication de la demande: 24.09.2008
(73) Titulaire: L'Oréal, 75008 Paris (FR)
(72) Inventeur: Lagrange, Alain, 77700 Coupvray (FR); Guerin, Frédéric, 75010 Paris (FR)
(74) Mandataire: Prevel, Estelle Nicole

(56) Documents cités:
- EP-A- 0 091 402
- FR-A- 2 862 530
- US-A- 4 754 034
- DATABASE CA [Online] CHEMICAL ABSTRACTS SERVICE, COLUMBUS, OHIO, US; 12 mai 1984 (1984-05-12), KAMIO, TAKAYOSHI ET AL: "Imaging materials containing pyrazolochromen derivative type color formers" XP002493753 extrait de STN Database accession no. 1980:119745 & JP 54 126114 A (FUJI PHOTO FILM CO., LTD., JAPAN) 1 octobre 1979 (1979-10-01)
- GABBUTT ET AL: "Oxidation and ring cleavage reactions of 3-benzhydrylchromones. Generation of triarylmethine cations from methylidenechroman-4-ones and benzopyrano[4,3-c]pyrazoles" TETRAHEDRON, ELSEVIER SCIENCE PUBLISHERS, AMSTERDAM, NL, vol. 62, no. 47, 20 novembre 2006 (2006-11-20), pages 10945-10953, XP005703305 ISSN: 0040-4020
- DATABASE CA [Online] CHEMICAL ABSTRACTS SERVICE, COLUMBUS, OHIO, US; 16 décembre 2001 (2001-12-16), ZAL'KIND, YU. S. ET AL: "Condensation of tetraphenylbutynediol with phenol" XP002493754 extrait de STN Database accession no. 1946:23981 & ZHURNAL OBSHCHEI KHIMII , 15, 488-98 CODEN: ZOKHA4; ISSN: 0044-460X, 1945,

## Description

La présente invention a pour objet l'utilisation pour la coloration des fibres kératiniques, et en particulier des fibres kératiniques humaines telles que les cheveux, d'une composition comprenant au moins un composé halochromique convenablement sélectionné et / ou le colorant correspondant à ce composé.

Il est connu de teindre les fibres kératiniques, et en particulier les fibres kératiniques humaines telles que les cheveux, avec des compositions tinctoriales contenant des précurseurs de colorant d'oxydation, appelés généralement bases d'oxydation, tels que des ortho- ou para-phénylènediamines, des ortho- ou para-aminophénols et des composés hétérocycliques tels que des dérivés de diaminopyrazole. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques, et en particulier les fibres kératiniques humaines telles que les cheveux, avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, azoïque cationique, xanthénique, acridinique azinique, triarylméthane, benzénique nitré ou des colorants naturels.

L'utilisation des colorants directs est très répandue car ils présentent certains avantages par rapport aux précurseurs de colorants d'oxydation et, notamment, une diminution des risques potentiels d'allergie, l'absence de sensibilisation du cheveu due au processus oxydatif et des temps de pose moins longs.

Les colorations obtenues sont cependant temporaires ou semi-permanentes, car la nature des interactions qui lient les colorants directs à la fibre kératinique, et leur désorption de la surface et / ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

L'utilisateur peut choisir le mode de coloration lui permettant d'obtenir des nuances adaptées à ses besoins en terme de reflets et de ténacité. Cependant, pour effacer les couleurs ainsi obtenues, il doit utiliser des compositions de décoloration comprenant un ou plusieurs agents oxydants ou des réducteurs de type réductone, thiol ou sulfite. Parmi les agents oxydants classiquement utilisés, on peut citer le peroxyde d'hydrogène ou des composés susceptibles de produire du peroxyde d'hydrogène par hydrolyse, comme par exemple le peroxyde d'urée ou les persels tels que les perborates, les persulfates, les percarbonates. L'utilisation de ces agents oxydants présente l'inconvénient d'entraîner une dégradation non négligeable des fibres kératiniques et d'altérer leurs propriétés cosmétiques. Les cheveux ont tendance à devenir rèches, plus difficilement démêlables et plus fragiles. Les réducteurs de type réductone, thiol ou sulfite présentent quant à eux l'inconvénient de ne pas convenir à tous les types de colorants. Si ils permettent d'effacer efficacement et sans endommager la fibre kératinique les colorants azoïques, ils sont inopérants avec les colorants dérivés des anthraquinones.

Par ailleurs, l'utilisation pour la coloration des fibres kératiniques de composés halochromiques particuliers est connue de l'art antérieur, notamment de la demande FR 2 862 530 qui décrit des composés comportant un cycle pouvant subir une ouverture en formant un groupement acide, de la demande FR 2 862 531 qui décrit des composés comportant un cycle lactone, et de la demande FR 2 862 532 qui décrit des dimères de composés comportant un cycle lactone. Ces composés permettent d'obtenir des compositions pour la coloration des fibres kératiniques qui permettent de pallier partiellement les inconvénients indiqués ci-dessus mais qui sont encore insuffisamment performants.

Le but de la présente invention est de fournir de nouveaux composés halochromiques pour la coloration des fibres kératiniques qui permettent d'obtenir des compositions améliorées pour la coloration des fibres kératiniques notamment vis-à-vis des inconvénients mentionnés ci-dessus. En particulier, le but de la présente invention est de fournir de nouveaux composés halochromiques pour la coloration des fibres kératiniques permettant d'obtenir rapidement des colorations avec des reflets intenses et tenaces qui peuvent s'effacer grâce à un agent extérieur qui n'altère pas les fibres kératiniques comme un agent de pH ou la chaleur par exemple.

Ce but est atteint avec la présente invention qui a pour objet l'utilisation pour la coloration des fibres kératiniques, et en particulier des fibres kératiniques humaines telles que les cheveux, d'une composition comprenant, dans un milieu approprié pour la teinture, au moins un composé choisi parmi les composés de formule (I) comportant un groupement cyclique G incluant un cycle H susceptible de s'ouvrir, les colorants correspondant aux composés de formule (I) dont le cycle H est ouvert et leurs sels d'addition : 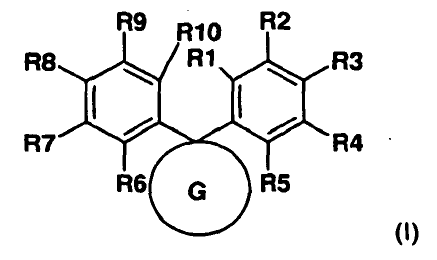 dans laquelle :
- **R₁**, **R₂**, **R₃**, **R₄**, **R₅**, **R₆**, **R₇**, **R₈**, **R₉ et R₁₀** représentent, indépendamment l'un de l'autre :
   - un atome d'hydrogène ;
   - un atome d'halogène ;
   - un radical hydroxyle ;
   - un radical nitro ;
   - un radical amino ;
   - un radical carboxy ;
   - un radical aminocarbonyle ;
   - un radical cyano ;
   - un radical issu d'une chaîne hydrocarbonée comportant de 1 à 100 atomes de carbone, de préférence de 1 à 50, linéaire ou ramifiée, saturée ou insaturée, acyclique ou monocyclique dans laquelle le cycle est aromatique ou non aromatique ou polycyclique dans laquelle les cycles sont condensés ou non condensés et aromatiques ou non aromatiques, pouvant être interrompue ou terminée à l'une de ses extrémités par un ou plusieurs hétéroatomes choisis parmi les atomes d'oxygène et de soufre ou par un ou plusieurs groupements choisis parmi les groupements carbonyle, SO, SO₂ et NH et pouvant être terminée à son autre extrémité par un groupement hydrogénocarbonyle ou par un groupement contenant un ou plusieurs hétéroatomes choisis parmi les atomes d'azote, d'oxygène et de soufre, ladite chaîne hydrocarbonée pouvant être substituée par un ou plusieurs groupements choisis parmi les radicaux hydroxyle, halogéno, carboxy, carboxyalkyle en C₁-C₉, cyano, amino, amino substitué par un ou deux groupements alkyle en C₁-C₄, alcoxy en C₁-C₆, aryle en C₆-C₁₆, aryloxy dont le groupement aryle est en C₆-C₁₈, acyloxy en C₂-C₉ ;
   deux des radicaux **R₁**, **R₂**, **R₃**, **R₄**, **R₅**, **R₆**, **R₇**, **R₈**, **R₉** et **R₁₀**, portés par deux atomes de carbone adjacents, pouvant former ensemble et avec les atomes de carbone auxquels ils sont attachés un groupement monocarbocyclique dans lequel le cycle est aromatique ou non aromatique ou polycarbocyclique dans lequel les cycles sont condensés ou non condensés et aromatiques ou non aromatiques, comprenant de 5 à 20 chaînons, un ou plusieurs atomes de carbone pouvant être remplacés par un atome d'oxygène, d'azote, de soufre ou de phosphore, le groupement mono ou polycarbocyclique étant non substitué ou substitué par un ou plusieurs substituants choisis parmi un atome d'halogène, un radical hydroxyle, un radical amino, un radical carboxy, un radical aryle en C₆-C₁₈, un radical cyano, un radical alkyle en C₁-C₉ ; un radical alcoxy en C₁-C₉, un radical alcoxy(C₁-C₉)carbonylalkyl(C₁-C₉)amino, un radical α-naphtylalkylamino ;
- **G** représente un radical divalent ayant l'une des formules suivantes : 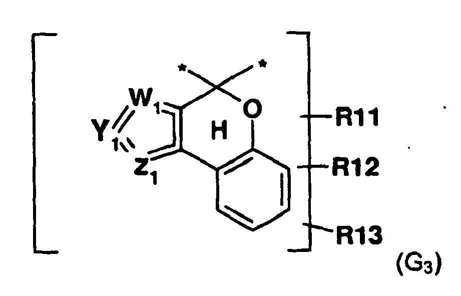 ou 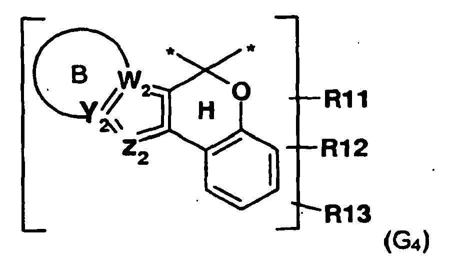 dans lesquelles :
   - **Y₁**, **W₁** et **Z₁** d'une part, et **Y₂, W₂** et **Z₂** d'autre part, représentent, indépendamment les uns des autres, un atome de soufre, un atome de carbone, un atome d'azote ou un groupement divalent CR₁₅ ou NR₁₅ ;
   - **R₁₁**, **R₁₂**, **R₁₃** et **R₁₅** ont, indépendamment les uns des autres, les mêmes définitions que les radicaux **R₁** à **R₁₀** ;
   deux des radicaux **R₁₁**, **R₁₂** et **R₁₃**, portés par deux atomes de carbone adjacents, pouvant former ensemble et avec les atomes de carbone auxquels ils sont attachés un groupement monocarbocyclique dans lequel le cycle est aromatique ou non aromatique ou polycarbocyclique dans lequel les cycles sont condensés ou non condensés et aromatiques ou non aromatiques, comprenant de 5 à 20 chaînons, un ou plusieurs atomes de carbone pouvant être remplacés par un atome d'oxygène, d'azote, de soufre ou de phosphore, le groupement mono ou polycarbocyclique étant non substitué ou substitué par un ou plusieurs substituants choisis parmi un atome d'halogène, un radical hydroxyle, un radical amino, un radical carboxy, un radical aryle en C₆-C₁₈, un radical cyano, un radical alkyle en C₁-C₉, un radical alcoxy en C₁-C₉ ;
- **B** représente un groupement aryle en C₆-C₁₈ ou un groupement hétérocyclique comportant de 5 à 12 chaînons, saturé ou insaturé, substitué ou non substitué ;
les radicaux amino étant non substitués ou substitués par un ou deux radicaux, identiques ou différents, choisis parmi un radical alkyle en C₁-C₉ ; un radical hydroxyalkyle en C₁-C₉ ; un radical alcényle en C₂-C₉ ; un radical cycloalkyle en C₅-C₁₂, un radical aryl(C₆-C₁₈)carbonyle ; un radical cycloalkyl(C₅-C₁₂)alkyle en C₁-C₉ ; un radical alkyl(C₁-C₉)carbonyle ; un radical alcoxy(C₁-C₉)carbonylalkyl(C₁-C₉) ; un radical α-naphtylalkyle ; un radical halogénoalkyle en C₁-C₉ ; un radical alkyl(C₁-C₉)carbonyloxyalkyl(C₁-C₉) ; un radical cyanoalkyle en C₁-C₉ ; un radical acyle en C₂-C₁₅ ; un radical alcoxy(C₁-C₉)carbonyle ; un radical aryloxy(C₆-C₁₈)carbonyle ; un radical aryloxy(C₆-C₁₈)alkyl(C₁-C₉)carbonyle ; un radical aryl(C₆-C₁₈)alcoxy(C₁-C₉)carbonyle : un radical alcoxy(C₁-C₉)aryl(C₆-C₁₈)carbonyle ; un radical dialkyl(C₁-C₉)aminocarbonyle ; un radical dialkyl(C₁-C₉)aminosulfonyle; un radical alkyl(C₁-C₉)aryl(C₆-C₁₈)sulfonyle ; un radical alkyl(C₁-C₉)sulfonyle ; un radical dialkyl(C₁-C₉)aminoalkyle en C₁-C₉ ; un radical alcoxy(C₁-C₉)alkyl(C₁-C₉) ; un radical aryle en C₆-C₁₈ ou un radical aryl(C₆-C₁₈)alkyl(C₁-C₉) éventuellement substitué sur le noyau aryle par un ou plusieurs substituants choisis parmi un atome d'halogène, un radical alkyle en C₁-C₉, un radical nitro, un radical dialkyl(C₁-C₉)amino, un radical alcoxy en C₁-C₉ ; les deux radicaux pouvant former ensemble et avec l'atome d'azote du groupement amino un cycle éventuellement porteur d'un autre hétéroatome de 5 à 12 chaînons, ledit cycle pouvant être substitué par un radical alkyle en C₁-C₉.

La présente invention a également pour objet un procédé de traitement des fibres kératiniques, et en particulier des fibres kératiniques humaines telles que les cheveux, mettant en oeuvre la composition conforme à l'invention.

La présente invention permet en particulier d'obtenir rapidement une coloration des fibres kératiniques avec des reflets intenses et tenaces pouvant être effacée puis reformée tout aussi rapidement. Le procédé d'effaçage et de reformation de la couleur peut être reproduit au moins une fois sans perte substantielle de couleur à l'aide d'un agent de pH ou en agissant sur la température.

Les composés de formule (I) comportent un cycle H pouvant subir une ouverture en formant un groupement acide en présence de protons. Ces composés sont incolores ou faiblement colorés et les composés correspondant à l'ouverture du cycle H sont des espèces colorées et colorantes. En milieu aqueux, il s'établit un équilibre entre les espèces colorées et les espèces non colorées qui dépend du pH et de la température.

Lorsque la composition comprend plusieurs composés choisis parmi les composés de formule (I), les colorants correspondant aux composés de formule (I) dont le cycle H est ouvert et leurs sels d'addition, la coloration des fibres kératiniques peut aussi être modifiée à l'aide d'un agent de pH ou en agissant sur la température.

Dans le cadre de la présente invention, les symboles dans les formules (G3) et (G4) indiquent les liaisons par lesquelles le radical divalent G se rattache aux noyaux aromatiques substitués par les radicaux R₁ à R₁₀ dans la formule (I).

Dans le cadre de la présente invention, on entend par radical alkyle (alk) un radical linéaire ou ramifié, par exemple un radical méthyle, éthyle, n-propyle, iso-propyle, n-butyle ou ter-butyle. Un radical alcoxy est un radical alk-O-, un radical alkylthio est un radical alk-S-, un radical mono ou dialkylamino est un radical -N(alk)ₙ avec n = 1 ou 2, un radical alkylcarbonyle ou un radical acyle est un radical alk-CO-, un radical alcoxycarbonyle est un adical alk-O-CO-, un radical alkylcarbonylalkyle est un radical alk-CO-alk-, un radical alkylcarbonylamino est un radical alk-CO-NH-, dans chacune de ces définitions le radical alkyle étant tel que défini précédemment.

On entend par radical alcényle, un alkyle de 2 à 10 atomes de carbone et comprenant une ou plusieurs doubles et / ou triples liaisons, conjuguées ou non.

On entend par radical cycloalkyle un radical alkyle dans lequel les atomes de carbone forment un cycle, par exemple un radical cyclohexyle. Un radical mono ou dicycloalkylamino est un radical amino substitué par un ou deux radicaux cycloalkyle.

On entend par radical aryle (ar) un radical carboné dérivé des composés benzéniques condensés ou non condensés, par exemple phényle, anthracényle ou naphtyle. Un radical mono ou diarylamino est un radical amino substitué par un ou deux radicaux aryle. Un radical mono ou di(arylalkyl)amino est un radical amino substitué par un ou deux radicaux arylalkyle. Un radical arylalkyle est un radical alkyle substitué par un radical aryle. Un radical arylalcoxy est un radical alcoxy substitué par un radical aryle. Un radical arylcarbonyle est un radical ar-CO-, avec ar étant tel que défini précédemment.

On entend par radical hétéroaryle un radical aryle comportant un ou plusieurs hétéroatomes, par exemple un cycle pyridinique.

Un radical halogéno désigne un atome d'halogène choisi parmi le chlore, le brome, l'iode et le fluor.

Dans le cadre de la présente invention, le terme "condensé" signifie au moins deux cycles accolés présentant au moins deux atomes communs.

Un groupement mono ou polyhétérocyclique, condensé ou non condensé, aromatique ou non aromatique, comprenant de 5 à 50 chaînons, peut être par exemple un cycle thiophène, benzofurane, benzothiophène, indole, bispyridine, benzopyrane, quinoline, pyrazole, pyridine, pyrrole, furane, imidazole, benzimidazole.

Un groupement mono ou polycarbocyclique aromatique, condensé ou non condensé, comprenant de 5 à 50 chaînons, un ou plusieurs atomes de carbone pouvant être remplacés par un atome d'oxygène, d'azote, de soufre ou de phosphore peut être par exemple un cycle benzène, naphtalène, anthracène, pyridine, quinoline, thiofène, pyrimidine.

Un radical imino est un radical HN=C.

Un radical arylimino peut être par exemple un radical phénylimino.

Un radical alcoxycarbonylalkylamino est un radical amino substitué par un radical alcoxycarbonylalkyle.

Un radical α-naphtylalkylamino est un radical amino substitué par un radical α-naphtylalkyle, qui est un radical alkyle substitué par un radical α-naphtyle.

Selon un mode de réalisation particulier de l'invention, lorsque A et B sont substitués alors ils sont substitués par un ou plusieurs radicaux R₁₆, identiques ou différents, ayant la même définition que les radicaux R₁ à R₁₀.

Selon un mode de réalisation particulier de l'invention, le composé de formule (I) est tel que :
- **R₅** représente un atome d'hydrogène ;
- **R₁** représente :
   - un atome d'hydrogène ;
   - un atome d'halogène ;
   - un radical alkyle en C₁-C₉ ;
   - un radical alcoxy en C₁-C₉ ;
   - un radical nitro ;
   - un radical amino ;
   - un radical alkyl(C₁-C₉)thio ;
   - un radical acyloxy(C₂-C₉)alcoxy(C₁-C₉) ;
- **R₂** représente un atome d'hydrogène ; un radical alkyle en C₁-C₉ ; un radical alcoxy en C₁-C₉ ; un atome d'halogène ; un radical amino ;
- **R₃** représente un atome d'hydrogène ; un radical amino ; un atome d'halogène; un radical aryloxy(C₆-C₁₈)alcoxy(C₁-C₉) ; un radical nitro ; un radical alcoxy en C₁-C₉ ;
- **R₄** représente un atome d'hydrogène ; un atome d'halogène ; un radical alkyle en C₁-C₉ ; un radical alcoxy en C₁-C₉ ; un radical alkyl(C₁-C₉)thio ;
- **R₆** représente un atome d'hydrogène ; un atome d'halogène ; un radical alkyle en C₁-C₉ ; un radical alcoxy en C₁-C₉ ; un radical nitro ; un radical amino ; un radical acyloxy(C₂-C₉)alcoxy(C₁-C₉) ;
- **R₇** représente un atome d'hydrogène ; un atome d'halogène ; un radical alkyle en C₁-C₉ ; un radical alcoxy en C₁-C₉ ;
- **R₈** représente un atome d'hydrogène ; un atome d'halogène ; un radical alkyle en C₁-C₉ ; un radical alcoxy en C₁-C₉ ; un radical nitro ; un radical amino ;
- **R₉** représente un atome d'hydrogène ; un radical alkyle en C₁-C₉ ; un radical alcoxy en C₁-C₉ ;
- **R₁₀** représente un atome d'hydrogène ; un radical alcoxy en C₁-C₉ ;
- **R₁₁** représente un atome d'hydrogène ; un atome d'halogène ; un radical alkyle en C₁-C₉ ; un radical alcoxy en C₁-C₉ ; un radical aryle en C₆-C₁₈ ; un radical amino ; un radical acyle en C₂-C₁₈ ;un radical aryl(C₆-C₁₈)sulfonyle ;
- **R₁₂** représente un atome d'hydrogène ; un radical alkyle en C₁-C₉ ; un radical alcoxy en C₁-C₉ ; un radical amino ;
- **R₁₃** représente un atome d'hydrogène ;
- **R₁** et **R₂** et / ou **R₂** et **R₃** et / ou **R₆** et **R₇** et / ou **R₇** et **R₈** pouvant former ensemble et avec les atomes de carbone auxquels ils sont attachés un groupement monocarbocyclique ou polycarbocyclique dans lequel les cycles sont aromatiques, condensés ou non condensés, comprenant de 5 à 20 chaînons, un ou plusieurs atomes de carbone pouvant être remplacés par un atome d'oxygène, d'azote, de soufre ou de phosphore, non substitué ou substitué, les substituants étant de préférence choisis parmi les atomes d'halogène, les radicaux hydroxyle, amino, carboxy, alcoxy(C₁-C₉)carbonylalkyl(C₁-C₉)amino, α-naphtylalkylamino.

Selon un mode de réalisation particulier de l'invention, les composés de formule (I) sont choisis parmi les composés de formule (I₁) suivante : 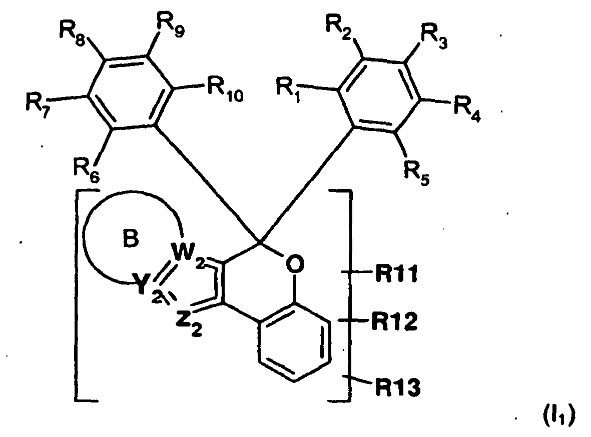 dans laquelle de préférence :
- **Y₂**, **W₂** et **Z₂** représentent, indépendamment l'un de l'autre, un atome de carbone ou d'azote ou de soufre ou un groupement divalent CR₁₅ ou NR₁₅ ;
- **B** présente un groupement aryle en C₆-C₁₈ ou un groupement hétérocyclique de 5 à 12 chaînons saturé ou insaturé, substitué ou non substitué ;
- **R₁** représente un atome d'hydrogène ; un atome d'halogène ; un radical alcoxy en C₁-C₉; un radical alkyle en C₁-C₉; un radical nitro ; un radical alkyl(C₁-C₉)thio ;
- **R₂** représente un atome d'hydrogène ; un atome d'halogène ; un radical alkyle en C₁-C₉ ; un radical alcoxy en C₁-C₉ ;
- **R₃** représente un atome d'hydrogène ; un atome d'halogène ; un radical nitro ; un radical amino ; un radical alcoxy en C₁-C₉ ;
- **R₄** représente un atome d'hydrogène ; un radical alcoxy en C₁-C₉ ; un radical alkyle en C₁-C₉ ; un radical alkyl(C₁-C₉)thio ;
- **R₅**, **R₁₀** et **R₁₃** représentent un atome d'hydrogène ;
- **R₆** représente un atome d'hydrogène ; un atome d'halogène ; un radical nitro ; un radical alcoxy en C₁-C₉ ; un radical alkyle en C₁-C₉ ; un radical amino ; un radical acyloxy(C₂-C₉)alcoxy(C₁-C₉) ;
- **R₇** représente un atome d'hydrogène ; un radical alcoxy en C₁-C₉ ;
- **R₈** représente un atome d'hydrogène ; un atome d'halogène ; un radical alcoxy en C₁-C₉ ; un radical alkyle en C₁-C₉ ; un radical amino ;
- **R₉** représente un atome d'hydrogène ; un radical alcoxy en C₁-C₉ ; un radical alkyle en C₁-C₉ ;
- **R₁₁** représente un atome d'hydrogène ; un atome d'halogène ; un radical amino ; un radical alkyle en C₁-C₉ ; un radical dialkyl(C₁-C₉)aminocarbonyle ; un radical aryl(C₆-C₁₈)carbonyle ; un radical aryl(C₆-C₁₈)sulfonyle ; un radical acyle en C₂-C₉ ;
- **R₁₂** représente un atome d'hydrogène ; un radical alkyle en C₁-C₉ ; un radical aryl(C₆-C₁₈)aminocarbonyle ; un radical acyle en C₂-C₂₀ ; un radical alcoxy(C₁-C₉)carbonyle ;
**R₁** et **R₂** d'une part, et **R₆** et **R₇** d'autre part, pouvant former ensemble et avec les atomes de carbone auxquels ils sont attachés un cycle aromatique en C₆-C₁₈.

De préférence, **W₂** représente un atome de carbone, un atome d'azote ou un atome de soufre, **Y₂** représente un atome de carbone, un atome d'azote ou un groupe divalent CR₁₅, et **Z₂** représente un atome d'azote ou un groupe divalent CR₁₅ ou NR₁₅.

Selon un mode de réalisation particulier de l'invention, les composés de formule (I) sont choisis parmi l'une des formules (I₂) à (I₆) suivantes : 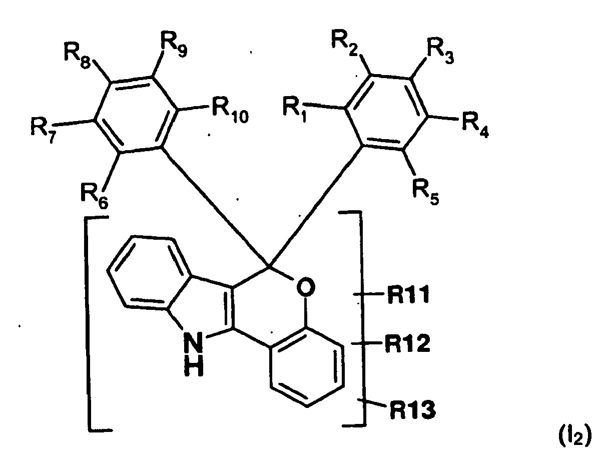 ou 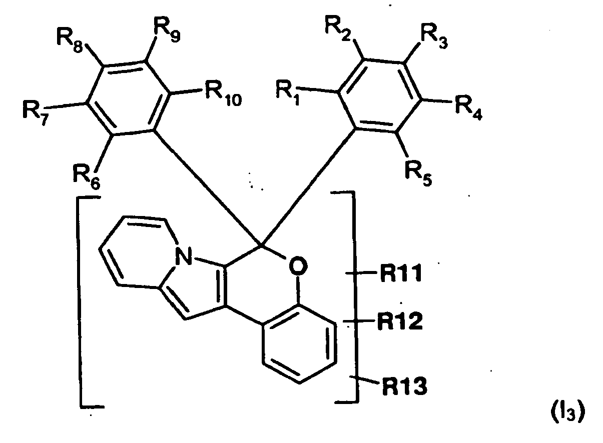 ou 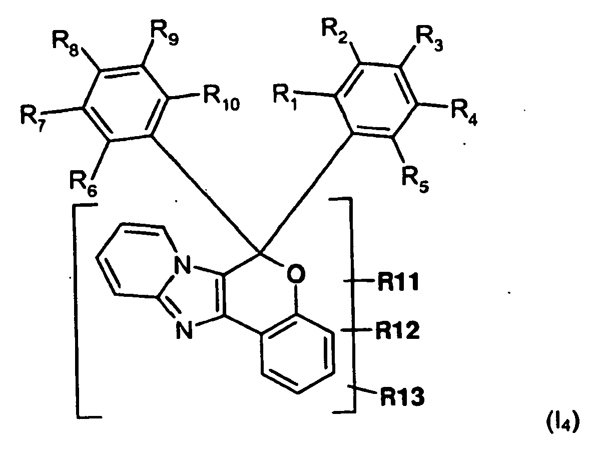 ou 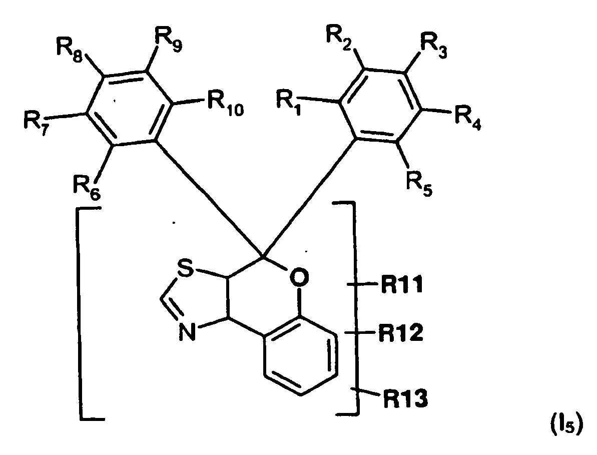 ou 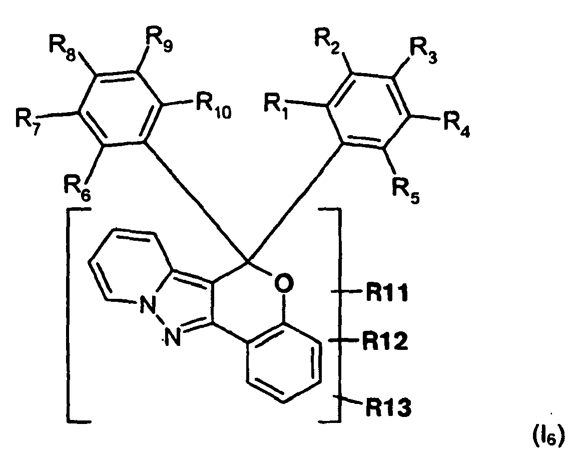 dans lesquelles de préférence :
- **R₁** représente un atome d'hydrogène ; un atome d'halogène ; un radical alcoxy en C₁-C₉ ; un radical alkyle en C₁-C₉ ; un radical nitro ; un radical alkyl(C₁-C₉)thio ;
- **R₂** représente un atome d'hydrogène ; un atome d'halogène ; un radical alkyle en C₁-C₉ ; un radical alcoxy en C₁-C₉ ;
- **R₃** représente un atome d'hydrogène ; un atome d'halogène ; un radical nitro ; un radical amino ; un radical alcoxy en C₁-C₉ ;
- **R₄** représente un atome d'hydrogène ; un radical alcoxy en C₁-C₉ ; un radical alkyle en C₁-C₉ ; un radical alkyl(C₁-C₉)thio ;
- **R₅**, **R₁₀** et **R₁₃** représentent un atome d'hydrogène ;
- **R₆** représente un atome d'hydrogène ; un atome d'halogène ; un radical nitro ; un radical alcoxy en C₁-C₉ ; un radical alkyle en C₁-C₉ ; un radical amino ; un radical acyloxy(C₂-C₉)alcoxy(C₁-C₉) ;
- **R₇** représente un atome d'hydrogène; un radical alcoxy en C₁-C₉ ;
- **R₈** représente un atome d'hydrogène ; un atome d'halogène ; un radical alcoxy en C₁-C₉ ; un radical alkyle en C₁-C₉ ; un radical amino ;
- **R₉** représente un atome d'hydrogène ; un radical alcoxy en C₁-C₉ ; un radical alkyle en C₁-C₉ ;
- **R₁₁** représente un atome d'hydrogène ; un atome d'halogène ; un radical amino ; un radical alkyle en C₁-C₉ ; un radical dialkyl(C₁-C₉)aminocarbonyle ; un radical aryl(C₆-C₁₈)carbonyle ; un radical aryl(C₆-C₁₈)sulfonyle ; un radical acyle en C₂-C₉ ;
- **R₁₂** représente un atome d'hydrogène ; un radical alkyle en C₁-C₉ ; un radical aryl(C₆-C₁₈)aminocarbonyle ; un radical acyle en C₂-C₂₀ ; un radical alcoxy(C₁-C₉)carbonyle ;
**R₁** et **R₂** d'une part, et **R₆** et **R₇** d'autre part, pouvant former ensemble et avec les atomes de carbone auxquels ils sont attachés un cycle aromatique en C₆-C₁₈.

Pour les formules (I₁) à (I₆), par radical amino, il faut entendre un radical amino non substitué ou substitué par un ou deux radicaux, identiques ou différents, choisis parmi un radical alkyle en C₁-C₉ ; un radical hydroxyalkyle en C₁-C₉ ; un radical alcényle en C₂-C₉ ; un radical cycloalkyle en C₅-C₁₂ ; un radical aryl(C₆-C₁₈)carbonyle ; un radical cycloalkyl(C₅-C₁₂)alkyle en C₁-C₉ ; un radical alkyl(C₁-C₉)carbonyle ; un radical alcoxy(C₁-C₉)carbonylalkyl(C₁-C₉) ; un radical α-naphtylalkyle ; un radical halogénoalkyle en C₁-C₉ ; un radical alkyl(C₁-C₉)carbonyloxyalkyl(C₁-C₉) ; un radical cyanoalkyle en C₁-C₉ ; un radical acyle en C₂-C₁₅ ; un radical alcoxy(C₁-C₉)carbonyle ; un radical aryloxy(C₆-C₁₈)carbonyle; un radical aryloxy(C₆-C₁₈)alkyl(C₁-C₉)carbonyle ; un radical aryl(C₆-C₁₈)alcoxy(C₁-C₉)carbonyle ; un radical alcoxy(C₁-C₉)aryl(C₆-C₁₈)carbonyle ; un radical dialkyl(C₁-C₉)aminocarbonyle ; un radical dialky)(C₁-C₉)aminosulfonyle ; un radical alkyl(C₁-C₉)aryl(C₆-C₁₈)sulfonyle ; un radical alkyl(C₁-C₉)sulfonyle ; un radical dialkyl(C₁-C₉)aminoalkyle en C₁-C₉ ; un radical alcoxy(C₁-C₉)alkyl(C₁-C₉) ; un radical aryle en C₆-C₁₈ ou un radical aryl(C₆-C₁₈)alkyl(C₁-C₉) éventuellement substitué sur le noyau aryle par un ou plusieurs substituants choisis parmi un atome d'halogène, un radical alkyle en C₁-C₉, un radical nitro, un radical dialkyl(C₁-C₉)amino, un radical alcoxy en C₁-C₉ ; les deux radicaux pouvant former ensemble et avec l'atome d'azote du groupement amino un cycle éventuellement porteur d'un autre hétéroatome de 5 à 12 chaînons, ledit cycle pouvant être substitué par un radical alkyle en C₁-C₉.

Pour les formules (I₁) à (I₆), par radical aryle, il faut entendre un radical aryle non substitué ou substitué par un ou plusieurs groupements choisis parmi les radicaux hydroxyle, alkyle en C₁-C₉, halogéno, carboxy, cyano, amino.

Pour les formules (I₁) à (I₆), par radical alkyle, il faut entendre un radical alkyle non substitué ou substitué par un atome d'halogène, un radical hydroxyle, un radical amino, un radical amino substitué par un ou deux groupement alkyle en C₁-C₄, un radical carboxy, un radical aryle en C₆-C₁₈, un radical cyano ; un radical alcoxy en C₁-C₉, aryle en C₆-C₁₈, aryloxy dont le groupement aryle est en C₆-C₁₈, acyloxy en C₂-C₉.

A titre d'exemples de composés de formule (1), on peut citer les composés suivants :

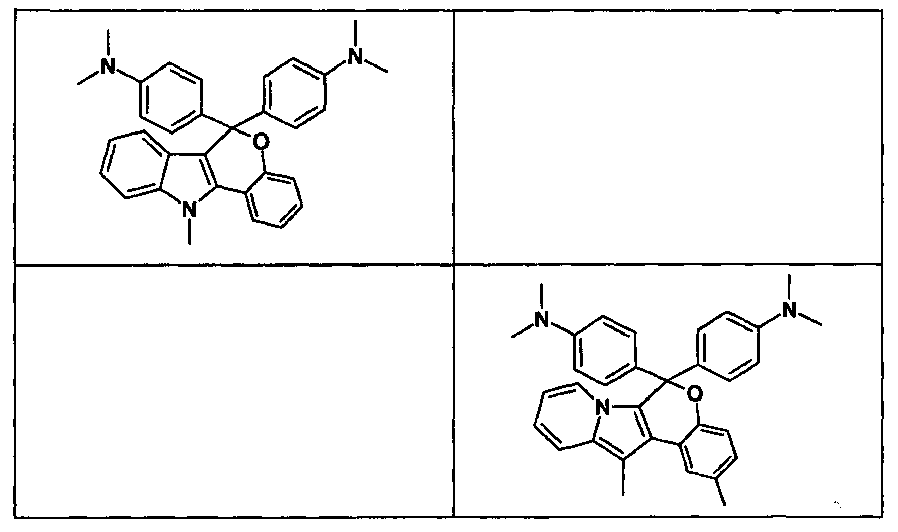

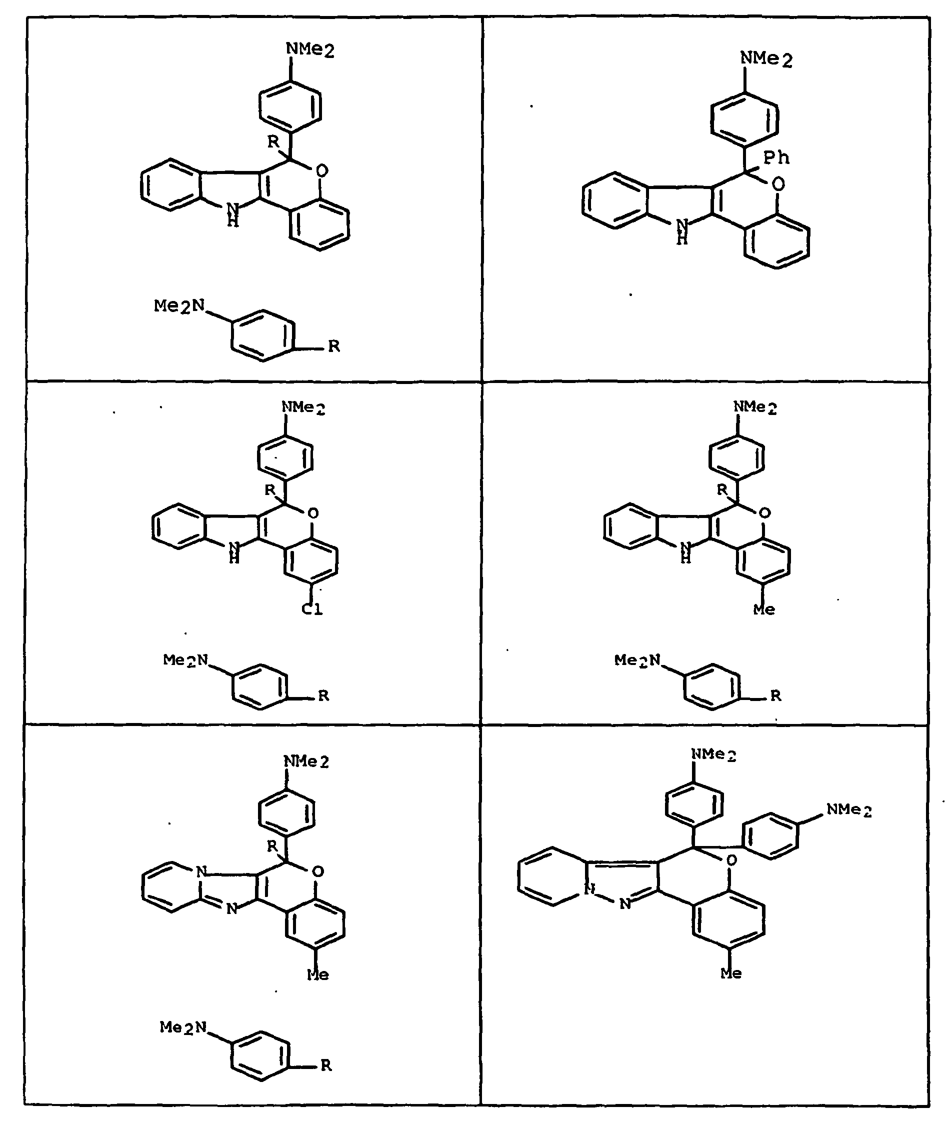

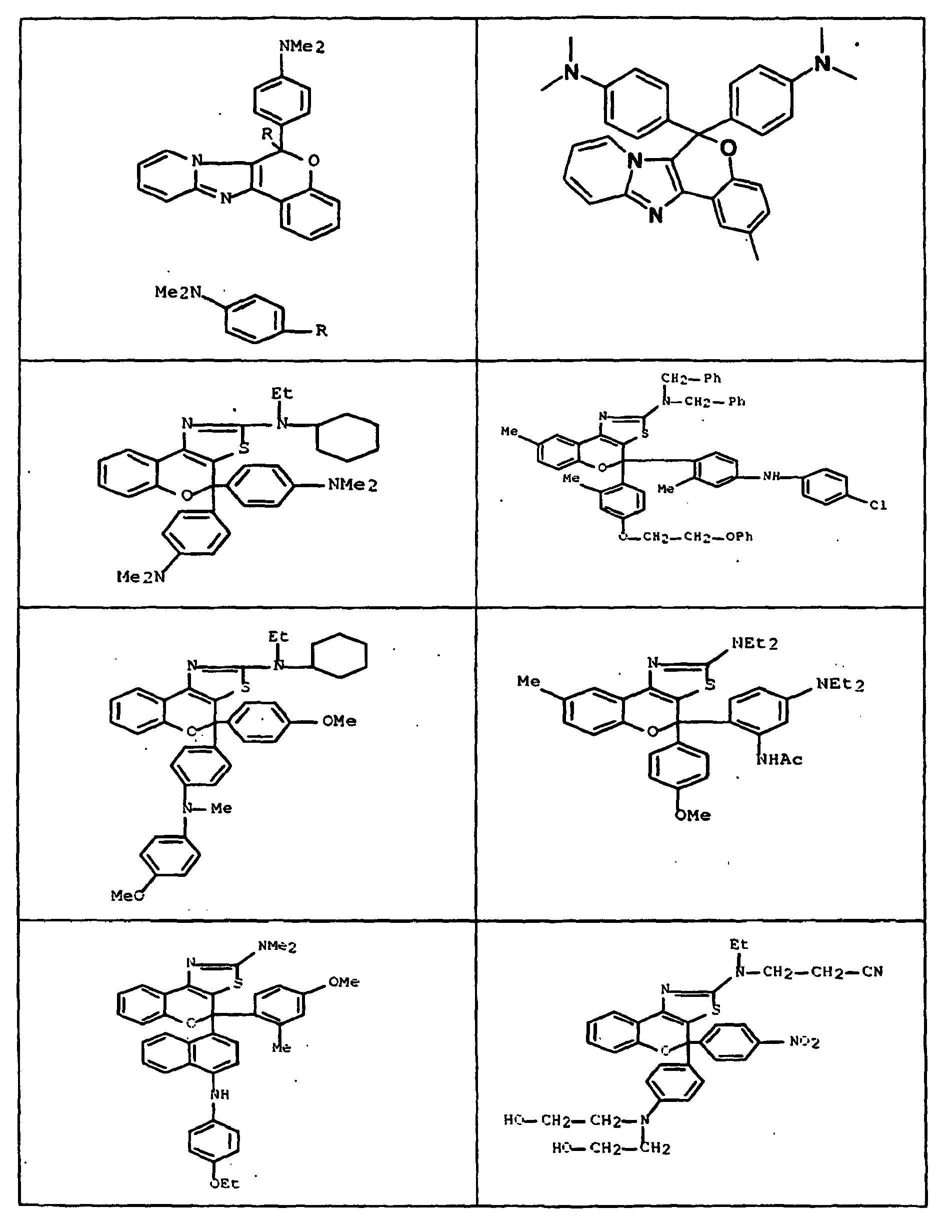

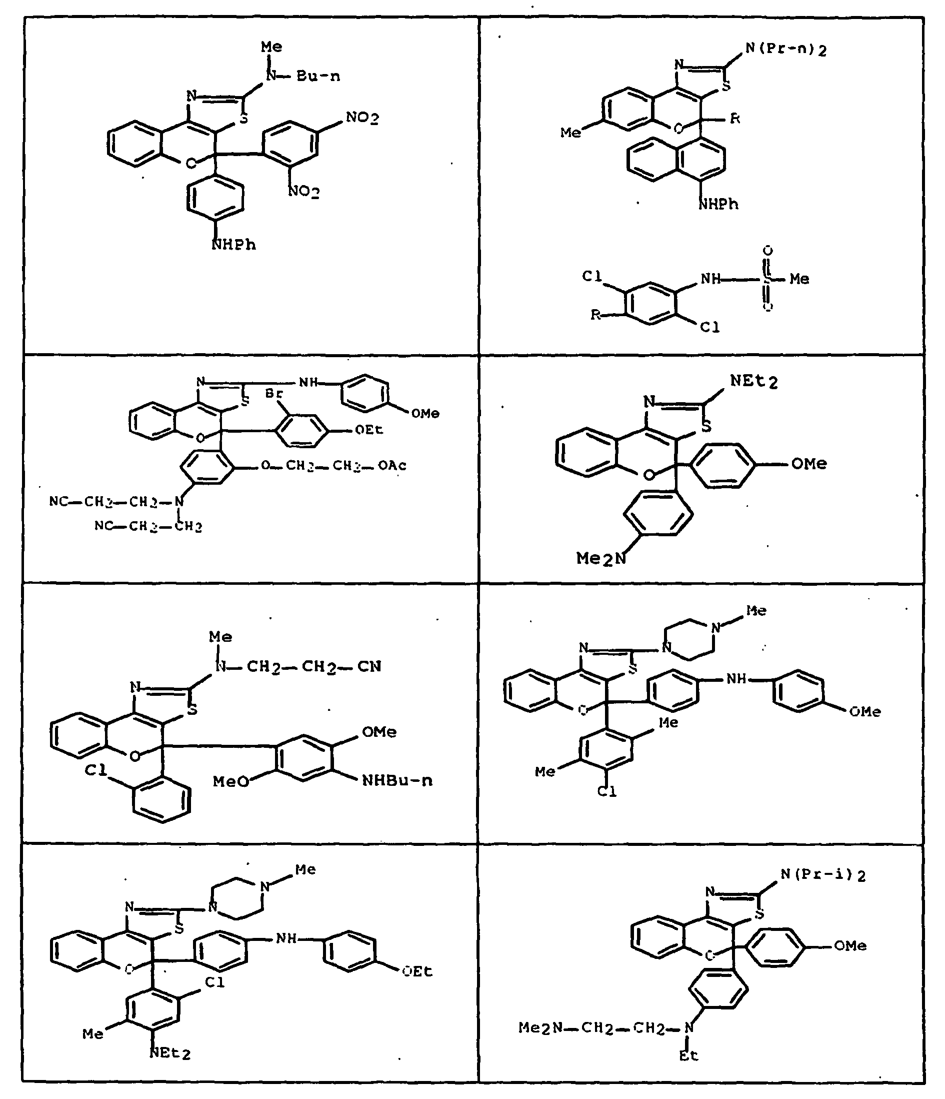

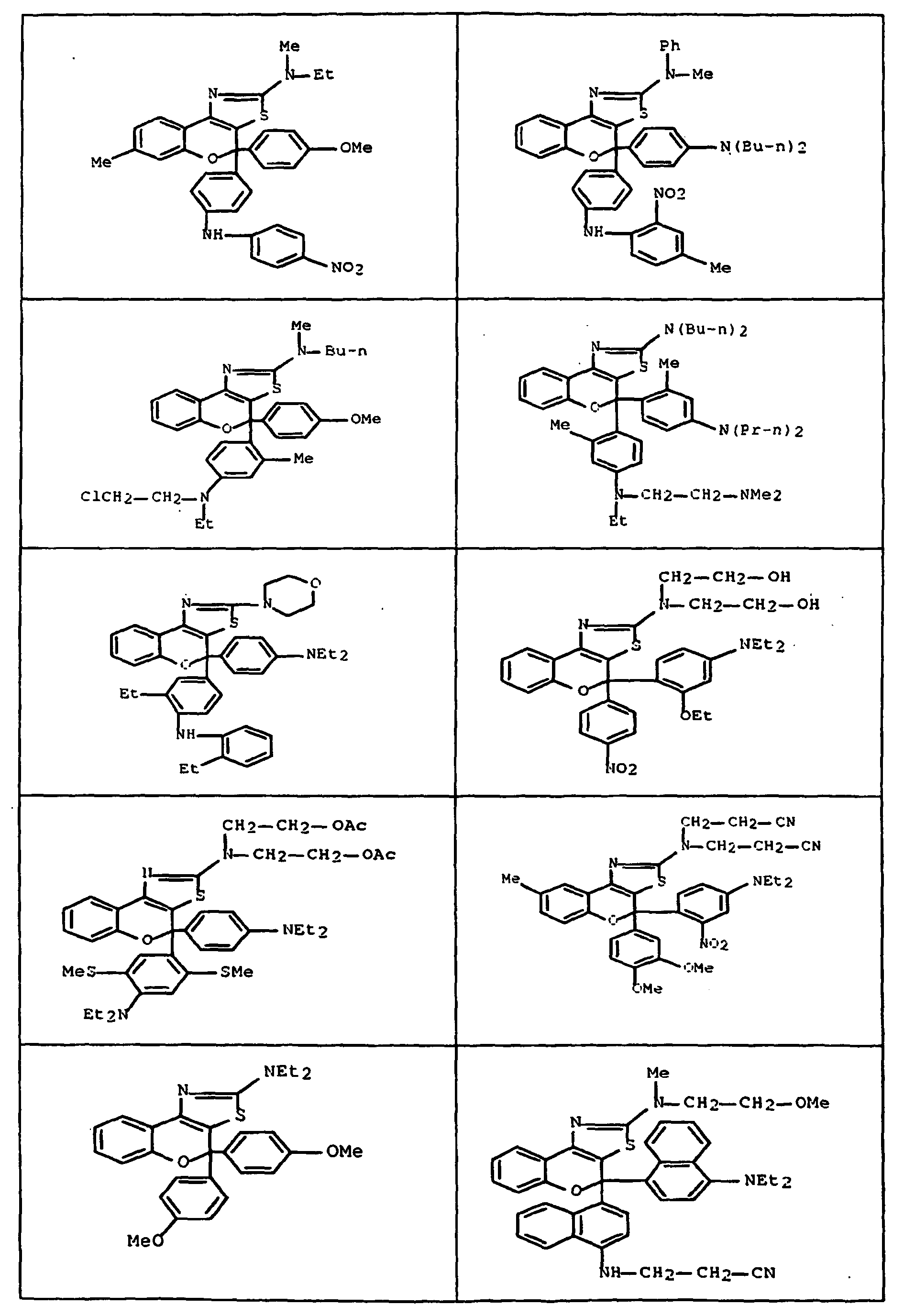

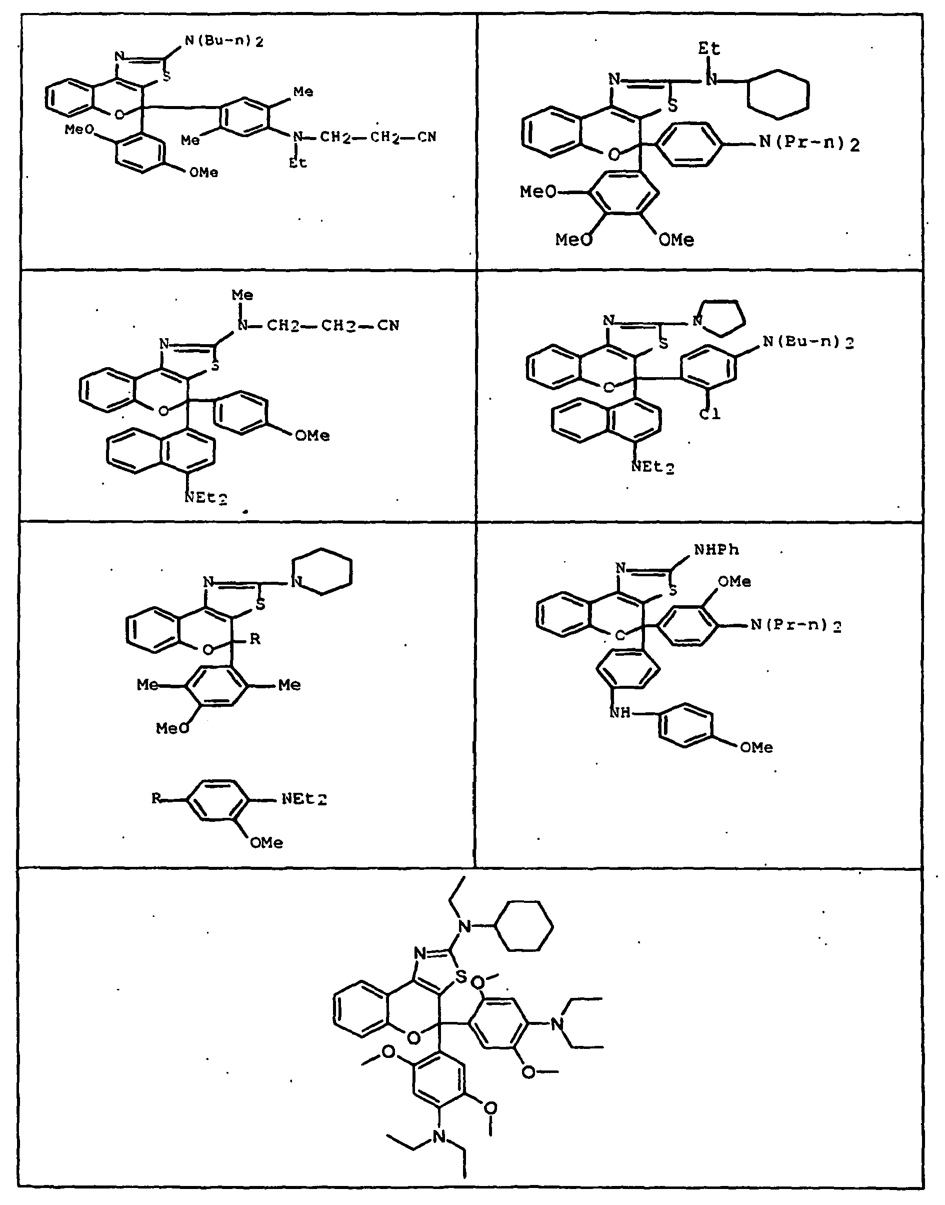

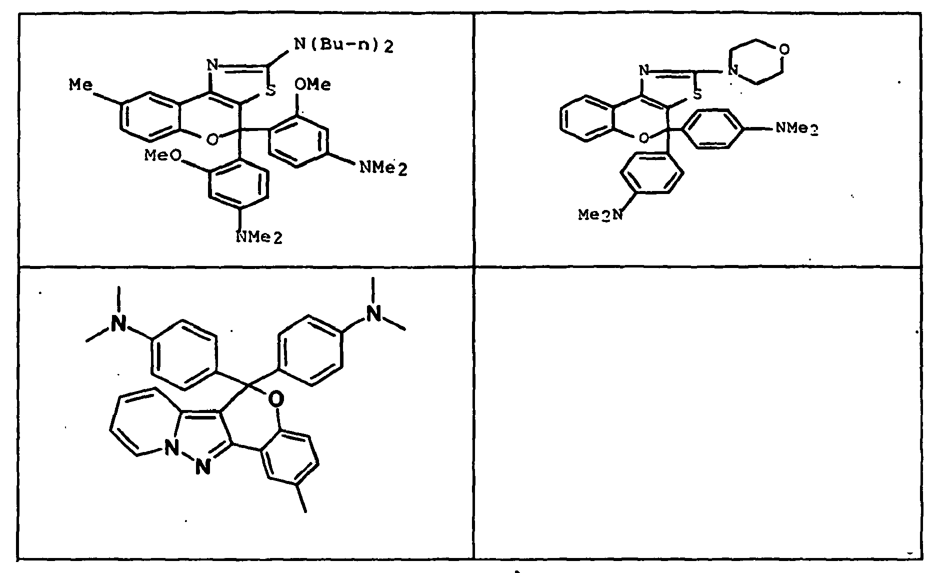

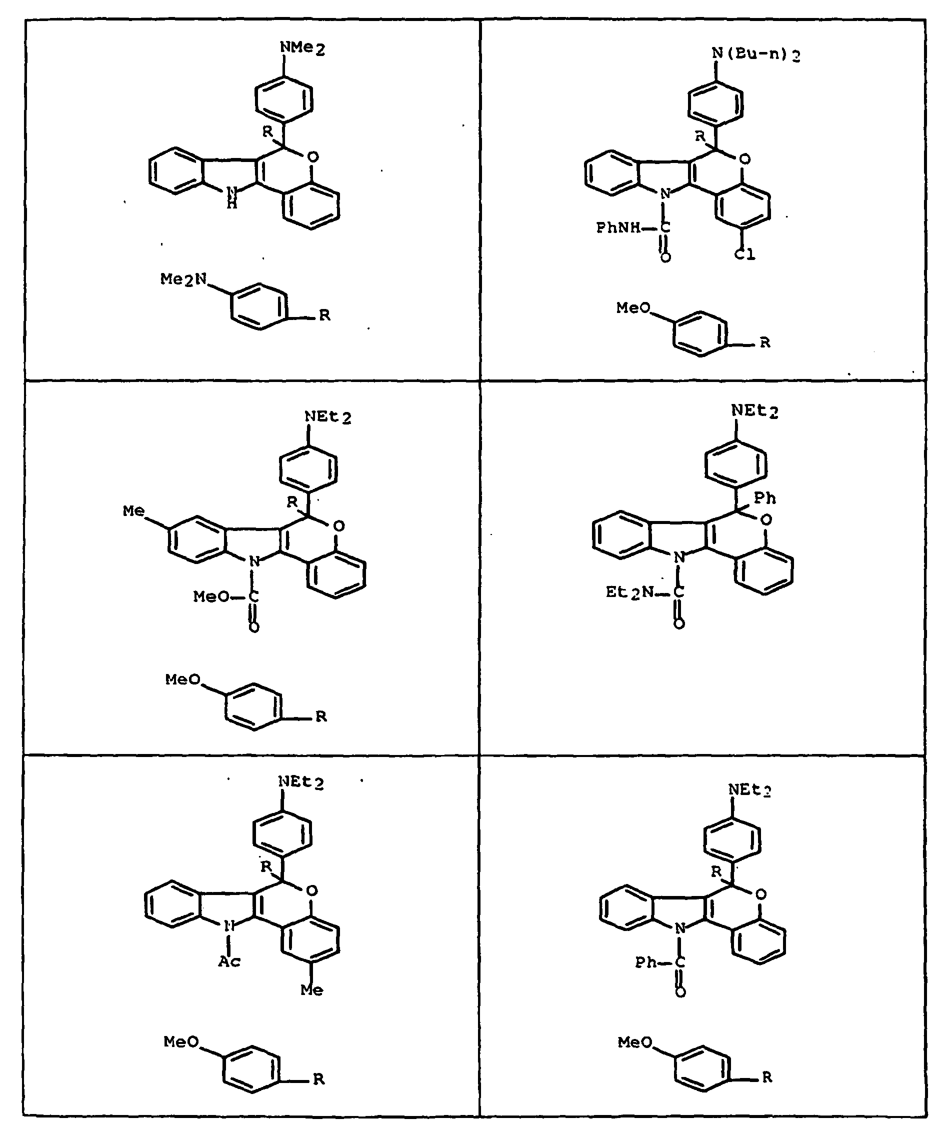

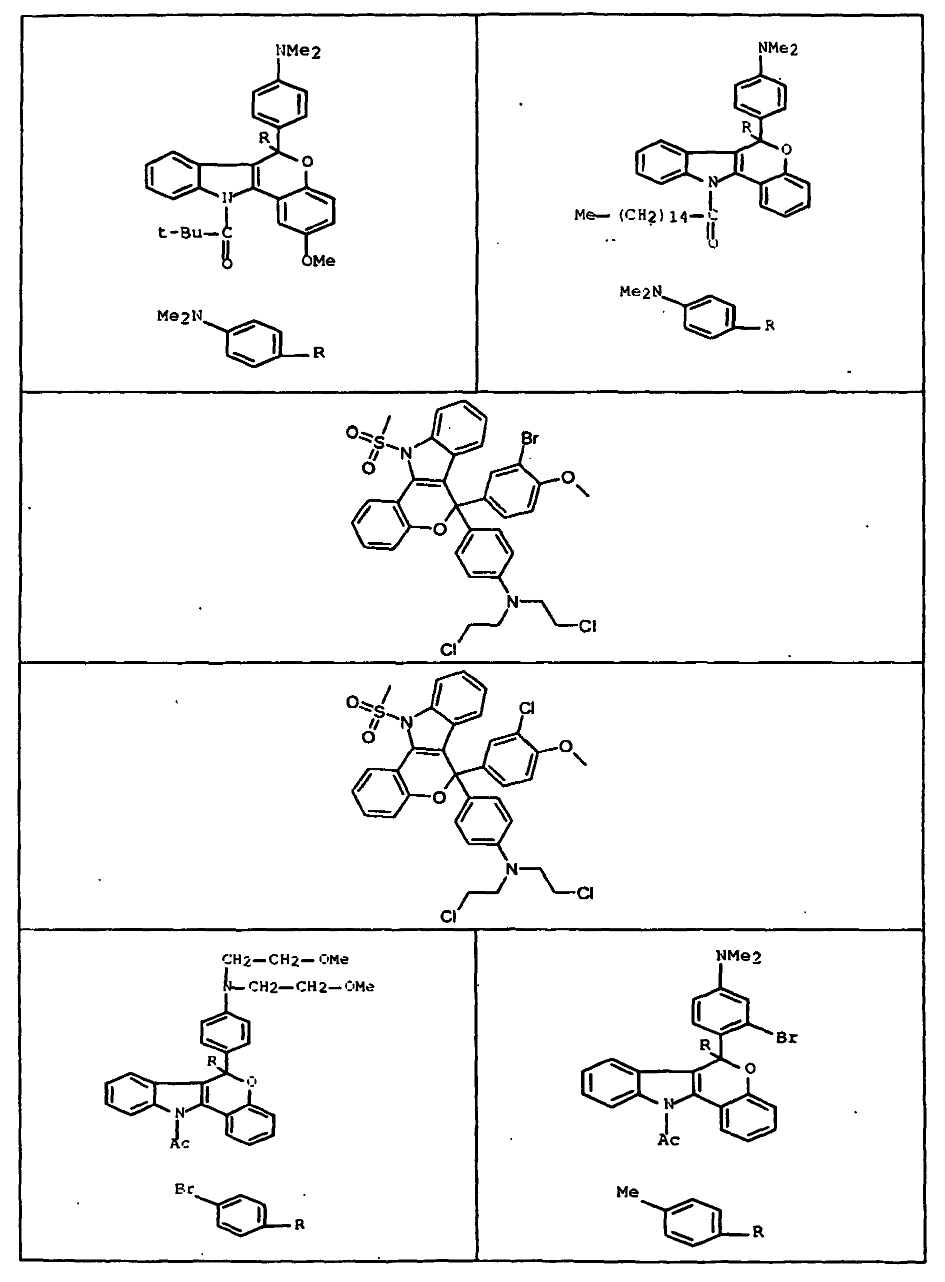

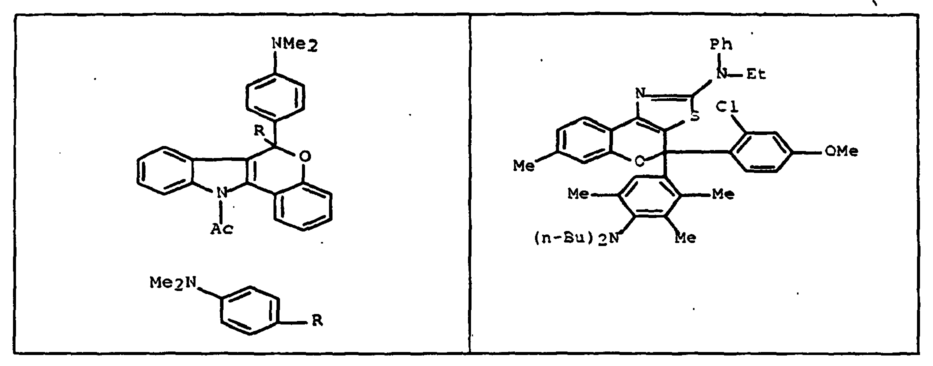

Les composés choisis parmi les composés de formule (I), les colorants correspondant aux composés de formule (I) dont le cycle H est ouvert et leurs sels d'addition représentent en général de 0,001 à 10 % en poids, de préférence de 0,005 à 5 % en poids par rapport au poids total de la composition tinctoriale conforme à l'invention.

D'une manière générale, les sels d'addition des composés de formule (I) et des colorants correspondant aux composés de formule (I) dont le cycle H est ouvert utilisables dans le cadre de l'invention sont notamment choisis parmi les sels d'addition avec un acide tels que les chlorhydrates, les bromhydrates, les sulfates, les méthosulfates, les gluconates, les citrates, les succinates, les tartrates, les lactates, les tosylates, les benzènesulfonates, les phosphates et les acétates et les sels d'addition avec une base telles que la soude, la potasse, l'ammoniaque, les amines ou les alcanolamines.

La composition conforme à l'invention peut en outre contenir un ou plusieurs colorants directs pouvant notamment être choisis parmi les colorants nitrés de la série benzénique, les colorants directs azoïques, les colorants directs méthiniques et les colorants naturels. Ces colorants directs peuvent être de nature non ionique, anionique ou cationique.

En présence de colorants directs additionnels dans la composition conforme à l'invention, ceux-ci représentent en général de 0,001 à 20 % en poids par rapport au poids total de la composition tinctoriale conforme à l'invention, de préférence de 0,01 à 10 %.

La composition de la présente invention peut en outre comprendre une ou plusieurs colorants d'oxydation choisis parmi les bases d'oxydation et les coupleurs classiquement utilisées en coloration d'oxydation.

En présence de colorants d'oxydation dans la composition conforme à l'invention, ceux-ci représentent en général de 0,001 à 20 % en poids par rapport au poids total de la composition tinctoriale conforme à l'invention, de préférence de 0,01 à 10 %.

Le milieu approprié pour la teinture, appelé aussi support de teinture, est généralement constitué par de l'eau ou par au moins un solvant organique ou par un mélange d'eau et d'au moins un solvant organique. A titre de solvant organique, on peut par exemple citer les cétones tels que l'acétone ; les alcanols en C₁-C₄, tels que l'éthanol et l'isopropanol ; les polyols comme le propylène glycol, la glycérine, l'hexylène glycol, et les éthers de polyols comme le 2-butoxyéthanol, le monométhyléther de propylèneglycol, le monoéthyléther et le monométhyléther du diéthylèneglycol, et leurs mélanges.

Les solvants sont généralement présents dans des proportions comprises entre 1 et 40 % en poids environ par rapport au poids total de la composition tinctoriale, et encore plus préférentiellement entre 5 et 30 % en poids environ.

Les composés choisis parmi les composés de formule (1), les colorants correspondant aux composés de formule (1) dont le cycle H est ouvert et leurs sels d'addition sont soit solubles, soit en dispersion, dans le support de teinture.

La composition conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des agents dispersants, des agents de conditionnement tels que par exemple des polymères cationiques ou amphotères, des cations, des silicones volatiles ou non volatiles, modifiées ou non modifiées, des chitosanes ou des dérivés de chitosane, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Lorsque les solvants sont constitués par de l'eau ou par un mélange d'eau et d'au moins un solvant organique, le pH de la composition tinctoriale conforme à l'invention est généralement compris entre 3 et 12, de préférence entre 4 et 11, encore plus préférentiellement entre 6 et 8,5. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux comme par exemple l'acide chlorhydrique, l'acide nitrique, l'acide sulfurique ou les acides organiques comme par exemple les composés comprenant au moins une fonction acide carboxylique comme l'acide acétique, l'acide tartrique, l'acide citrique, l'acide lactique, une fonction acide sulfonique, une fonction acide phosphonique ou une fonction acide phosphorique.

Parmi les agents alcalinisants on peut citer, à titre d'exemple :
- les acides aminés basiques ;
- les carbonates ou les bicarbonates de métaux alcalins ou alcalino-terreux ;
- les silicates ou les métasilicates ;
- les composés de formule (II) suivante :

   X(OH)ₙ (II)

   dans laquelle :
   - X représente un ion potassium, lithium, sodium, ammonium N⁺R₁₇R₁₈R₁₉R₂₀ avec R₁₇, R₁₈, R₁₉ et R₂₀, identiques ou différents, désignant un radical alkyle en C₂-C₄ lorsque n est égal à 1 ;
   - X représente un atome de magnésium ou de calcium lorsque n est égal à 2;
- les composés de formule (III) suivante : 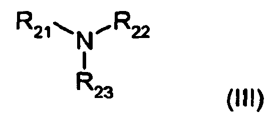 dans laquelle :
   - R₂₁ représente un atome d'hydrogène ; un radical alkyle en C₁-C₆, un radical monohydroxyalkyle en C₁-C₆ ; un radical polyhydroxyalkyle en C₂-C₆ ;
   - R₂₂ et R₂₃, identiques ou différents, représentent un atome d'hydrogène, un radical alkyle en C₁-C₆, un radical monohydroxyalkyle en C₁-C₆ un radical polyhydroxyalkyle en C₂-C₆
- les composés de formule (IV) suivante : 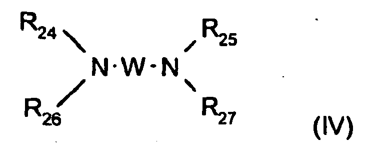 dans laquelle :
   - W est un reste propylène éventuellement substitué par un groupement hydroxyle ou un radical alkyle en C₁-C₄ ;
   - R₂₄, R₂₅, R₂₆ et R₂₇, identiques ou différents, représentent un atome d'hydrogène, un radical alkyle en C₁-C₄ ou hydroxyalkyle en C₁-C₄.

Au sens de la présente invention, on entend par "acide aminé basique", soit (i) un acide aminé présentant en plus de la fonction amine positionnée en α du groupement carboxyle, un groupement cationique (ou basique) supplémentaire; soit (ii) un acide aminé présentant une chaîne latérale (hydrophile) cationique (ou basique); soit (iii) un acide aminé portant une chaîne latérale constituée d'une base azotée. Ces définitions sont généralement connues et publiées dans les ouvrages de biochimie générale tels que J.H. WEIL (1983) pages 5 et suivantes, Lubert STRYER (1995) page 22, A. LEHNINGER (1993) pages 115-116, DE BOECK-WESMAEL (1994) pages 57-59.

Les acides aminés basiques conformes à l'invention sont choisis de préférence parmi ceux répondant à la formule (D) suivante : 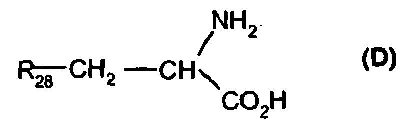 où R₂₈ désigne un groupe choisi parmi :

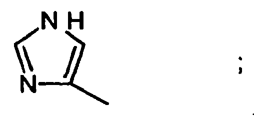

-(CH₂)₃NH₂ ;

-(CH₂)₂NH₂ ;

-(CH₂)₂NHCONH₂ ;

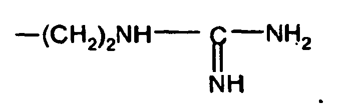

Parmi les composés de formule (D), on peut citer à titre d'exemple l'histidine, la lysine, l'ornithine, la citrulline, l'arginine.

La composition selon l'invention peut se présenter sous des formes diverses, telles que sous forme de liquides, de crèmes, de gels, ou sous toute autre forme appropriée pour réaliser une coloration des fibres kératiniques, et notamment des cheveux humains.

Le procédé de traitement des fibres kératiniques conforme à l'invention permet d'obtenir une coloration intense et tenace dont les reflets peuvent varier en fonction du pH ou de la température et qui peut être effacée et reformée au moins une fois sans perte substantielle de couleur. De préférence, la coloration est modifiée, effacée ou reformée en ajustant le pH à l'aide d'un agent acidifiant ou d'un agent alcalinisant.

Dans le sens de la présente invention, la coloration est effacée lorsque les fibres kératiniques ont retrouvé leur couleur d'origine. La coloration est modifiée lorsque la coloration obtenue est différente de celle obtenue au cours de l'étape précédente. La coloration est reformée lorsque la coloration des fibres kératiniques obtenue est sensiblement la même que celle qui avait été obtenue au cours d'une étape précédente et qui avait ensuite été modifiée.

La coloration obtenue dépend des composés de formule (I) qui sont appliqués sur les fibres kératiniques. Lorsque la totalité de ces composés ont leur cycle H ouvert, la coloration est intense. En jouant sur le pH ou la température, il est possible d'effacer cette coloration en passant de la totalité des composés de formule (I) dont le cycle H est ouvert à la totalité des composés de formule (I) dont le cycle H est fermé, puis de la reformer en passant de la totalité des composés de formule (I) dont le cycle H est fermé à la totalité des composés de formule (I) dont le cycle H est ouvert. Il est également possible de faire varier le rapport entre la concentration en composés de formule (I) dont le cycle H est ouvert et la concentration en composés de formule (I) dont le cycle H est fermé. La coloration est alors modifiée en intensité ou en couleur suivant qu'on applique sur les fibres kératiniques un ou plusieurs composés de formule (I) dont le cycle H est fermé ou un ou plusieurs composés de formule (I) dont le cycle H est ouvert et suivant leur sensibilité relative au pH ou à la température.

Un procédé préféré de traitement des fibres kératiniques, et en particulier des fibres kératiniques humaines telles que les cheveux, est caractérisé en ce que les étapes suivantes sont mises en oeuvre :
a) application d'une composition tinctoriale selon l'invention sur les fibres kératiniques pendant un temps de pose suffisant, le pH étant ajusté à l'aide d'un agent acidifiant ou d'un agent alcalinisant en fonction de la coloration souhaitée
b) éventuellement modification de la coloration des fibres kératiniques à l'aide d'un agent acidifiant ou d'un agent alcalinisant appliqué sur les fibres kératiniques.

L'application de la composition tinctoriale selon l'invention dans l'étape a) peut être ou non suivie d'un rinçage.

Selon un mode de réalisation particulier de l'invention, dans la première étape, l'agent acidifiant ou l'agent alcalinisant est mélangée à la composition tinctoriale avant application sur les fibres kératiniques.

Selon un autre mode de réalisation particulier de l'invention, dans la première étape, l'agent acidifiant ou l'agent alcalinisant est appliqué avant ou après la composition tinctoriale. Entre l'application de l'agent acidifiant ou de l'agent alcalinisant et l'application de la composition tinctoriale, on peut avoir un écart de 5 à 30 minutes. L'agent acidifiant ou l'agent alcalinisant est de préférence appliqué après la composition tinctoriale.

Le temps de pose de la composition conforme à l'invention est généralement compris entre 3 et 60 minutes, de préférence entre 5 et 40 minutes, encore plus préférentiellement entre 10 et 20 minutes.

La température d'application est généralement fixée entre la température ambiante et 250 °C, de préférence entre 25 et 55 °C.

Les fibres kératiniques peuvent être ou non rincées après application de l'agent acidifiant ou l'agent alcalinisant.

Les agents acidifiants ou alcalinisants sont choisis parmi ceux qui sont décrits ci-dessus.

La présente invention a également pour objet un dispositif à plusieurs compartiments ou kit permettant de mettre en oeuvre le procédé de coloration des fibres kératiniques décrit ci-dessus.

Le dispositif à plusieurs compartiments de l'invention contient dans un premier compartiment une composition conforme à l'invention comprenant au moins un tensioactif et au moins un agent épaississant et dans un deuxième compartiment un agent acidifiant ou un agent alcalinisant. Dans une autre version, le dispositif à plusieurs compartiments de l'invention contient dans le deuxième compartiment un agent acidifiant et dans un troisième compartiment un agent alcalinisant.

La présente invention a également pour objet une composition pour la coloration des fibres kératiniques, et en particulier des fibres kératiniques humaines telles que les cheveux telle que définie ci-dessus comprenant de plus au moins un tensioactif et / ou au moins un polymère, de préférence un polymère épaississant associatif ou non associatif.

Le ou les tensio-actifs peuvent être choisis parmi les tensio-actifs anioniques, cationiques, non-ioniques, amphotères, zwittérioniques ou leurs mélanges.

Par polymère associatif, on entend au sens de la présente invention tout polymère comportant au moins une chaîne grasse en C₈-C₃₀.

Par polymère épaississant, on entend au sens de la présente invention tout polymère qui, introduit dans la composition sans polymère à 25 °C, permet d'en accroître la viscosité, et en particulier tout polymère qui, introduit à 1 % en matière active dans la composition sans polymère, permet d'en accroître la viscosité d'au moins 100 cps à la température de 25 °C et à un taux de cisaillement de 1 s⁻¹. Cette viscosité est mesurable par exemple à l'aide d'un viscosimètre de type cone / plan.

On peut citer à titre d'exemple de polymère épaississant associatif les dérivés de polyuréthanes associatifs, les dérivés de celluloses associatifs, les dérivés de polyvinyllactames associatifs et les dérivés de polyacides insaturés associatifs.

On peut citer à titre d'exemple de polymère épaississant non associatif les homopolymères d'acide acrylique réticulés ; les homopolymères réticulés d'acide 2-acrylamido-2-méthyl-propane sulfonique et leurs copolymères réticulés d'acrylamide partiellement ou totalement neutralisés ; les homopolymères d'acrylate d'ammonium ou les copolymères d'acrylate d'ammonium et d'acrylamide ; les homopolymères de diméthylaminoéthylméthacrylate quaternisé par le chlorure de méthyle ou les copolymères de diméthylamino-éthylméthacrylate quaternisé par le chlorure de méthyle et d'acrylamide ; les gommes de guar non ioniques ; les gommes de biopolysaccharides d'origine microbienne telles que les gommes de scléroglucane ou de xanthane ; les gommes issues d'exudats végétaux telles que les gommes arabiques, gommes Ghatti, gommes Karaya et Tragacanthe ; les hydroxypropyl ou carboxyméthyl celluloses; les pectines ; les alginates.

L'exemple qui suit sert à illustrer l'invention sans toutefois présenter un caractère limitatif.

### EXEMPLE

### Colorant :

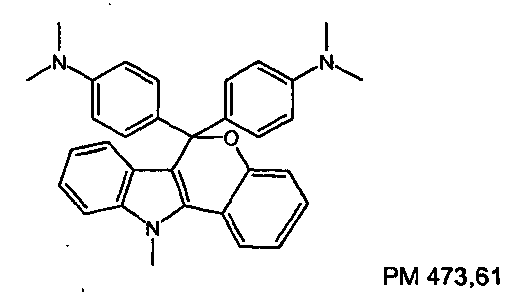

### Composition selon l'invention :

| | **Composition** |
|---|---|
| Colorant | 2g |
| Alcool benzylique | 4g |
| Polyéthylèneglycol 60E | 6g |
| Acide méthane sulfonique | qsp pH3 |
| Eau déminéralisée | qsp 100 g |

Cette composition est appliquée sur des mèches de cheveux gris à 90 % de cheveux blancs naturels et permanentés, à raison de 5 g pour 1 g de cheveux, à température ambiante pendant 30 minutes. A l'issue du temps de pose, la mèche est séchée.
La coloration capillaire est évaluée de manière visuelle. La nuance obtenue est un bleu.
Les mèches ainsi colorées peuvent être décolorées très rapidement par application d'une solution de soude 0,1 M. Les mèches retrouvent leurs reflets d'origine.

## Revendications

1. Utilisation pour la opération des fibres kératiniques d'une composition comprenant, dans un milieu approprié pour la teinture, au moins un composé choisi parmi les composés de formule (I) comportant un groupement cyclique G incluant un cycle H susceptible de s'ouvrir, les colorants correspondant aux composés de formule (I) dont le cycle H est ouvert et leurs sels d'addition: 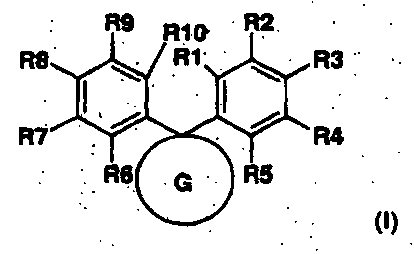 dans laquelle :
• **R₁**, **R₂**, **R_{3,} R₄, R₅**, **R₆**, **R₇, R₈**, **R₉** et **R₁₀** représentent, indépendamment l'un de l'autre :
- un atome d'hydrogène ;
- un atome d'halogène ;
- un radical hydroxyle ;
- un radical nitro ;
- un radical amino ;
- un radical carboxy ;
- un radical aminocarbonyle ;
- un radical cyano ;
- un radical issu d'une chaîne hydrocarbonée comportant de 1 à 100 atomes de carbone, linéaire ou ramifiée, saturée ou insaturée,acyclique ou monocyclique dans laquelle le cycle est aromatique ou non aromatique ou polycyclique dans laquelle les cycles sont condensés ou non condensés et aromatiques ou non aromatiques, pouvant être interrompue ou terminée à l'une de ses extrémités par un ou plusieurs hétéroatomes choisis parmi les atomes d'oxygène et de soufre ou par un ou plusieurs groupements choisis parmi les groupements carbonyle, SO, SO₂ et NH et pouvant être terminée à son autre extrémité par un groupement hydrogénocarbonyle ou par un groupement contenant un ou plusieurs hétéroatomes choisis parmi les atomes d'azote, d'oxygène et de soufre, ladite chaîne hydrocarbonée pouvant être substituée par un ou plusieurs groupements choisis parmi les radicaux hydroxyle, halogéno, carboxyl, carboxyalkyle en C₁-C₉, cyano, amino, amino substitué par un ou deux groupements alkyle en C₁-C₄, alcoxy en C₁-C₆, aryle en C₆-C₁₈, aryloxy dont le groupement aryle est en C₆-C₁₈, acyloxy en C₂-C₉ ;
deux des radicaux **R₁**, **R₂, R₃, R₄, R₅**, **R₆**, **R₇**, **R₈**, **R₉** et **R₁₀**, portés par deux atomes de carbone adjacents, pouvant former ensemble et avec les atomes de carbone auxquels ils sont attachés un groupement monocarbocyclique dans lequel le cycle est aromatique ou non aromatique ou polycarbocyclique dans lequel les cycles sont condensés ou non condensés et aromatiques ou non aromatiques, comprenant de 5 à 20 chaînons, un ou plusieurs atomes de carbone pouvant être remplacés par un atome d'oxygène, d'azote, de soufre ou de phosphore, le groupement mono ou polycarbocyclique étant non substitué ou substitué par un ou plusieurs substituants choisis parmi un atome d'halogène, un radical hydroxyle, un radical amino, un radical carboxy, un radical aryle en C₆-C₁₈, un radical cyano, un radical alkyle en C₁-C₉ ; un radical alcoxy en C₁-C₉, un radical alcoxy(C₁-C₉)carbonylalkyl(C₁-C₉)amino, un radical α-naphtylalkylamino ;
• **G** représente un radical divalent ayant l'une des formules suivantes : 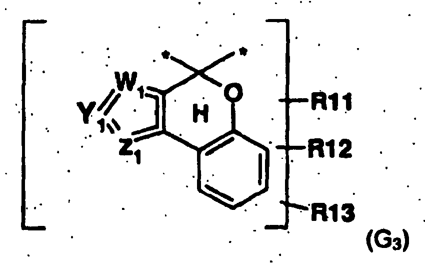 ou 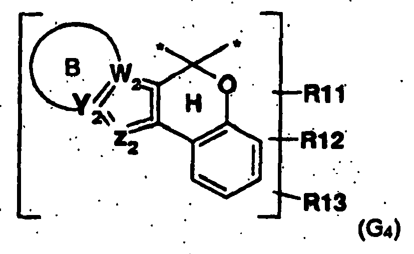 dans lesquelles
• **Y₁**, **W₁** et **Z₁** d'une part, et **Y₂**, **W₂** et **Z₂** d'autre part, représentent indépendamment les uns des autres, un atome de soufre, un atome de carbone, un atome d'azote ou un groupement divalent CR₁₅ ou NR₁₅;
• **R₁₁**, **R₁₂**, **R₁₃** et **R₁₅** ont, indépendamment les uns des autres, les mêmes définitions que les radicaux **R₁** à **R₁₀**;
deux des radicaux **R₁₁**, **R₁₂** et **R₁₃,** portés par deux atomes de carbone adjacents, pouvant former ensemble et avec les atomes de carbone auxquels ils sont attachés un groupement monocarbocyclique dans lequel le cycle est aromatique ou non aromatique ou polycarbocyclique dans lequel les cycles sont condensés ou non condensés et aromatiques ou non aromatiques, comprenant de 5 à 20 chaînons, un ou plusieurs atomes de carbone pouvant être remplacés par un atome d'oxygène, d'azote, de soufre ou de phosphore, le groupement mono ou polycarbocyclique étant non substitué ou substitué par un ou plusieurs substituants choisis parmi un atome d'halogène, un radical hydroxyle, un radical amino, un radical carboxy, un radical aryle en C₆-C₁₈, un radical cyano, un radical alkyle en C₁-C₉, un radical alcoxy en C₁-C₉ ;
• B représente un groupement aryle en C₆-C₁₈ ou un groupement hétérocyclique comportant de 5 à 12 chaînons, saturé ou insaturé, substitué ou non substitué;
les radicaux amino étant non substitués ou substitués par un ou deux radicaux, identiques ou différents, choisis parmi un radical alkyle en C₁-C₉ ; un radical hydroxyalkyle en C₁-C₉ ; un radical alcényle en C₂-C₉ , un radical cycloalkyle en C₅-C₁₂; un radical aryl(C₆-C₁₈)carbonyle ; un radical cycloalkyl(C₅-C₁₂)alkyle en C₁-C₉ ; un radical alkyl(C₁-C₉)carbonyle. ; un radical alcoxy(C₁-C₉)carbonylalkyl(C₁-C₈) ; un radical α-naphtylalkyle ; un radical halogénoalkyle en C₁-C₉ ; un radical alkyl(C₁-C₈)carbonyloxyalkyl(C₁-C₉) ; un radical cyanoalkyle en C₁-C₉ ; un radical acyle en C₂-C₁₅ ; un radical alcoxy(C₁-C₉)carbonyle ; un radical aryloxy(C₆-C₁₈)carbonyle;, un radical aryloxy(C₈-C₁₈)alkyl(C₁-C₉)carbonyle ; un radical aryl(C₆-C₁₈)alcoxy(C₁-C₉)carbonyle ; un radical alcoxy(C₁-C6₉)aryl(C₆-C₁₈)carbonyle; un radical dialkyl(C₁-C₉)aminocarbonyle ; un radical dialkyl(C₁-C₉)aminosulfonyle ; un radical alkyl(C₁-C₉)aryl(C₈-C₁₈)sulfonyle ; un radical alkyl(C₁-C₉)sulfonyle ; un radical dialkyl(C₁-C₉)aminoalkyle en C₁-C₉ ; un radical alcoxy(C₁-C₉)alkyl(C₁-C₉) ; un radical aryle en C₆-C₁₈ ou un radical aryl(C₆-C₁₈)alkyl(C₁-C₉) éventuellement substitué sur le noyau aryle par un ou plusieurs substituants choisis parmi un atome d'halogène, un radical alkyle en C₁-C₉, un radical nitro, un radical dialkyl(C₁-C₉)amino, un radical alcoxy en C₁-C₉ ; les deux radicaux pouvant former ensemble et avec l'atome d'azote du groupement amino un cycle éventuellement porteur d'un autre hétéroatome de 5 à 12 chaînons, ledit cycle pouvant être substitué par un radical alkyle en C₁-C₉.

2. Utilisation selon la revendication 1 dans laquelle le ou les composés de formule (I) sont tels que
• **R₅** représente un atome d'hydrogène ;
• **R₁** représente :
- un atome d'hydrogène ;
- un atome d'halogène ;
- un radical alkyle en C₁-C₉ ;
- un radical alcoxy en C₁-C₉ ;
- un radical nitro ;
- un radical amino ;
- un radical alkyl(C₁-C₉)thio ;
- un radical acyloxy(C₂-C₉)alcoxy(C₁-C₉) ;
• **R₂** représente un atome d'hydrogène ; un radical alkyle en C₁-C₉ ; un radical alcoxy en C₁-C₉: un atome d'hàlogène ; un radical amino ;
• **R₃** représente un atome d'hydrogène ; un radical amino ; un atome d'halogène ; un radical aryloxy(C₆-C₁₈)alcoxy(C₁-C₉) ; un radical nitro ; un radical-alcoky en C₁-C₉;
• **R₄** représente un atome d'hydrogène ; un atome d'halogène ; un radical alkyle en C₁-C₉ ; un radical àlcoxy en C₁-C₉ ; un radical alkyl(C₁-C₉)thio ;
• **R₆** représente un atome d'hydrogène ; un atome d'halogène ; un radical alkyle en C₁-C₉; un radical alcoxy en C₁-C₉ ; un radical nitro ; un radical amino ; un radical acyloxy(C₂-C₉)alcoxy(C₁-C₉) ;
• **R₇** représente un atome d'hydrogène ; un atome d'halogène ; un radical alkyle en C₁-C₉ ; un radical alcoxy en C₁-C₉ ;
• **R₈** représente un atome d'hydrogène ; un atome d'halogène; un radical alkyle en C₁-C₉ ; un radical alcoxy en C₁-C₉ ; un radical nitro ; un radical amino ;
• **R₉** représente un atome d'hydrogène ; un radical alkyle en C₁-C₉ ; un radical alcoxy en C₁-C₉ :
• **R₁₀** représente un atome d'hydrogène ; un radical alcoxy en C₁-C₉;
• **R₁₁** représente un atome d'hydrogène ; un atome d'halogène ; un radical alkyle en C₁-C₉ ; un radical alcoxy en C₁-C₉ ; un radical aryle en C₆-C₁₈; un radical amino ; un radical acyle en C₂-C₁₈ ; un radical aryl(C₆-C₁₈)sulfonyle;
• **R₁₂** représente un atome d'hydrogène ; un radical alkyle en C₁-C₉ ; un radical alcoxy en C₁-C₉ ; un radical amino ;
• **R₁₃** représente un atome d'hydrogène ;
• **R₁** et **R₂** et / ou **R₂** et **R₃** et / ou **R₆** et **R₇** et / ou **R₇** et **R₈** pouvant former ensemble et avec les atomes de carbone auxquels ils sont attachés un groupement monocarbocyclique ou polycarbocyclique dans lequel les cycles sont aromatiques, condensés ou non condensés, comprenant de 5 à 20 chaînons, un ou plusieurs atomes de carbone pouvant être remplacés par un atome d'oxygène, d'azote, de soufre ou de phosphore, non substitué ou substitué.

3. Utilisation selon la revendication 1 dans laquelle le ou les composés de formule (I) sont choisis parmi les composés de formule (I₁) suivante : 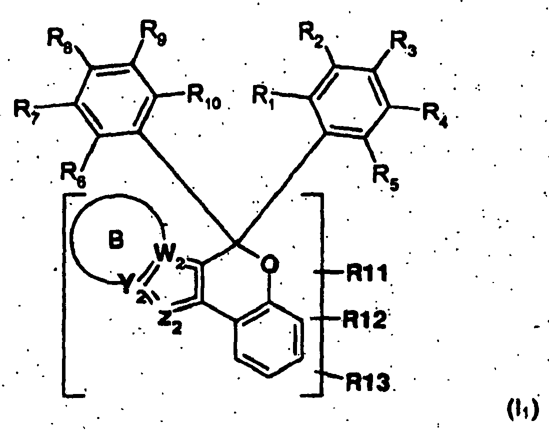 dans laquelle :
• **Y₂, W₂** et **Z₂** représentent, indépendamment l'un de l'autre, un atome de carbone ou d'azote ou de soufre ou un groupement divalent CR₁₅ ou NR₁₅ ;
• **B** présente un groupement aryle en C₆-C₁₈ ou un groupement hétérocyclique de 5 à 12 chaînons, saturé ou insaturé, substitué ou non substitué ;
• **R₁** représente un atome d'hydrogène ; un atome d'halogène ; un radical alcoxy en C₁-C₉ ; un radical alkyle en C₁-C₉ ; un radical nitro ; un radical alkyl(C₁-C₉)thio ;
• **R₂** représente un atome d'hydrogène ; un atome d'halogène ; un radical alkyle en C₁-C₉ ; un radical alcoxy en C₁-C₉ ;
• **R₃** représente un atome d'hydrogène ; un atome d'halogène un radical nitro ; un radical amino ; un radical alcoxy en C₁-C₉ ;
• **R₄** représente un atome d'hydrogène ; un radical alcoxy en C₁-C₉ ; un radical alkyle en C₁-C₉ ; un radical alkyl(C₁-C₉)thio ;
• **R₅**, **R₁₀** et **R₁₃** représentent un atome d'hydrogène ;
• **R₆** représente un atome d'hydrogène ; un atome d'halogène ; un radical nitro ; un radical alcoxy en C₁-C₉ ; un radical alkyle en C₁-C₉ ; un radical amino ; un radical acyloxy(C₂-C₉)alcoxy(C₁-C₉) ;
• **R₇** représente un atome d'hydrogène ; un radical alcoxy en C₁-C₉ ;
• **R₈** représente un atome d'hydrogène ; un atome d'halogène ; un radical alcoxy en C₁-C₉ ; un radical alkyle en C₁-C₉ ; un radical amino ;
• **R₉** représente un atome d'hydrogène; un radical alcoxy en C₁-C₉; un radical alkyle en C₁-C₉ ;
• **R₁₁** représente un atome d'hydrogène ; un atome d'halogène ; un radical amino ; un radical alkyle en C₁-C₉ ; un radical dialkyl(C₁-C₉)aminocarbonyle ; un radical aryl(C₆-C₁₈)carbonyle ; un radical aryl(C₆-C₁₈)sulfonyle ; un radical acyle en C₂-C₉ ;
• **R₁₂** représente un atome d'hydrogène ; un radical alkyle en C₁-C₉ ; un radical arylC₆-C₁₈)aminocarbonyle ; un radical acyle en C₂-C₂₀ ; un radical alcoxy(C₁-C₉)carbonyle ;
**R₁** et **R₂** d'une part, et **R₆** et **R₇** d'autre part, pouvant former ensemble et avec les atomes de carbone auxquels ils sont attachés un cycle aromatique en C₆-C₁₈.

4. Utilisation selon la revendication 1 dans laquelle le ou les composés de formule (I) sont choisis parmi l'une des formules (I₂) à (I₆) suivantes : 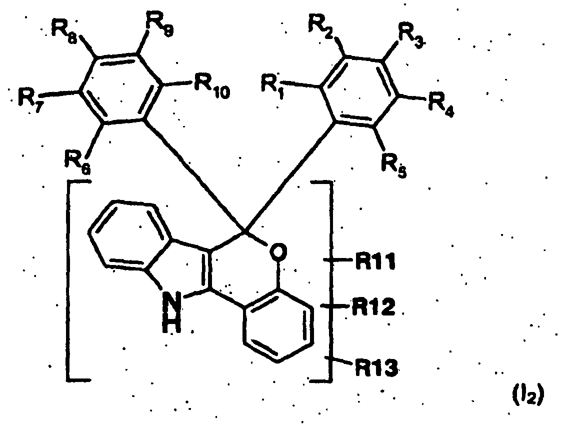 ou 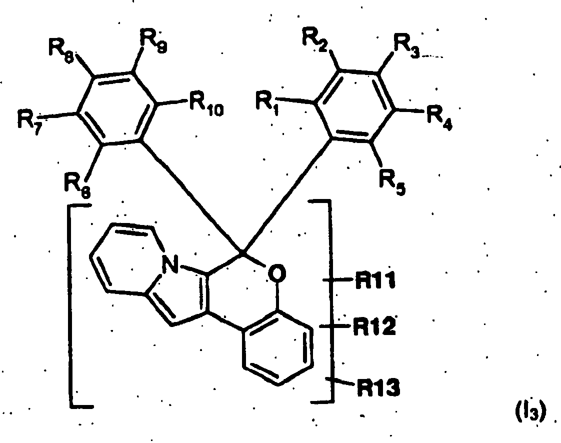 ou 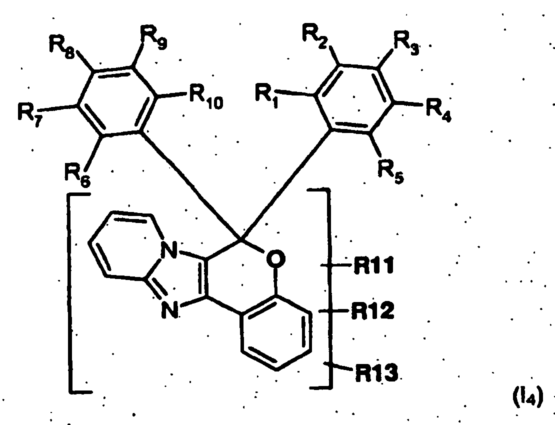 ou 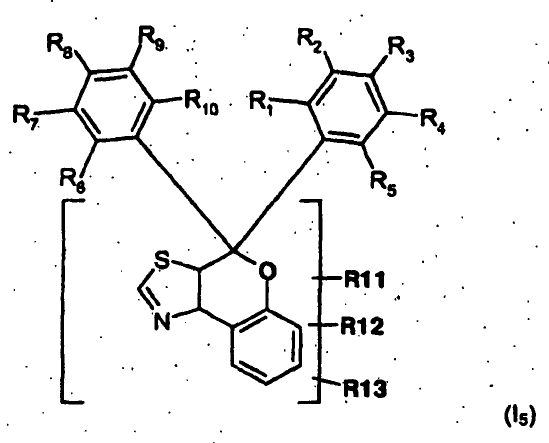 ou 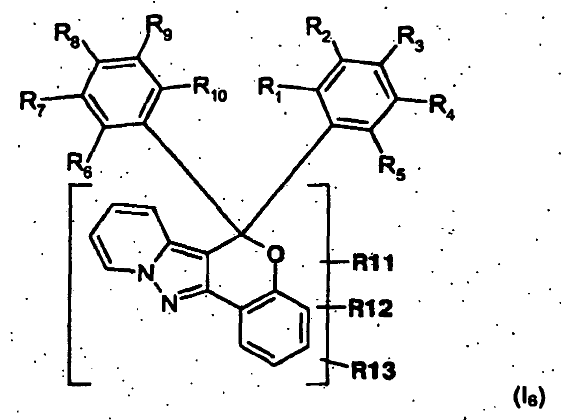 dans lesquelles :
• **R₁** représente un atome d'hydrogène ; un atome d'halogène ; un radical alcoxy en C₁-C₉ ; un radical alkyle en C₁-C₉ ; un radical nitro ; un radical alkyl(C₁-C₉)thio ;
• **R₂** représente un atome d'hydrogène ; un atome d'halogène ; un radical alkyle en C₁-C₉ ; un radical alcoxy en C₁-C₉ ;
• **R₃** représente un atome d'hydrogène ; un atome d'halogène ; un radical nitro ; un radical amino ; un radical alcoxy en C₁-C₉;
• **R₄** représente un atome d'hydrogène ; un radical alcoxy en C₁-C₉ ; un radical alkyle en C₁-C₉ ; un radical alkyl(C₁-C₉)thio ;
• **R₅**, **R₁₀** et **R₁₃** représentent un atome d'hydrogène ;
• **R₆** représente un atome d'hydrogène ; un atome d'halogène ; un radical nitro ; un radical alcoxy en C₁-C₉ ; un radical alkyle en C₁-C₉ ; un radical amino ; un radical acyloxy(C₂-C₉)alcoxy(C₁-C₉) ;
• **R⁷** représente un atome d'hydrogène ; un radical alcoxy en C₁-C₉ ;
• **R₈** représente un atome d'hydrogène ; un atome d'halogène ; un radical alcoxy en C₁-C₉ ; un radical alkyle en C₁-C₉ ; un radical amino ;
• **R₉** représente un atome d'hydrogène ; radical alcoxy en C₁-C₉ ; un radical alkyle en C₁-C₉ ;
• **R₁₁** représente un atome d'hydrogène ; un atome d'halogène ; un radical amino ; un radical alkyle en C₁-C₉ ; un radical dialkyl(C₁-C₉)aminocarbonyle ; un radical aryl(C₆-C₁₈)carbonyle; un radical aryl(C₆-C₁₈)sulfonyle ; un radical acyle en C₂-C₉ ;
• **R₁₂** représente un atome d'hydrogène ; un radical alkyle en C₁-C₉ ; un radical aryl(C₆-C₁₈)aminocarbonyle ; un radical acyle en C₂-C₂₀ ; un radical alcoxy(C₁-C₉)carbonyle ;
**R₁** et **R₂** d'une part, et **R₆** et **R₇** d'autre part, pouvant former ensemble et avec les atomes de carbone auxquels ils sont attachés un cycle aromatique en C₆-C₁₈.

5. Utilisation selon l'une quelconque dés revendications précédentes dans laquelle le composé de formule (I) est choisi parmi les composés suivants :

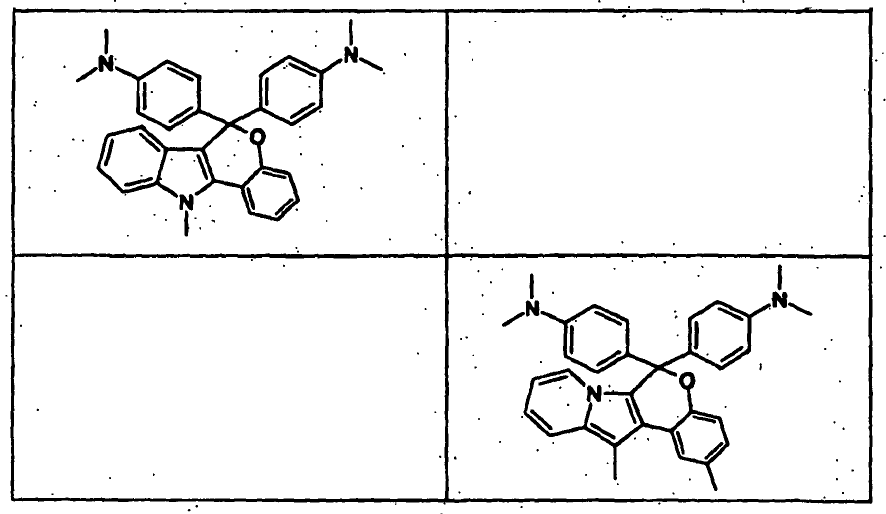

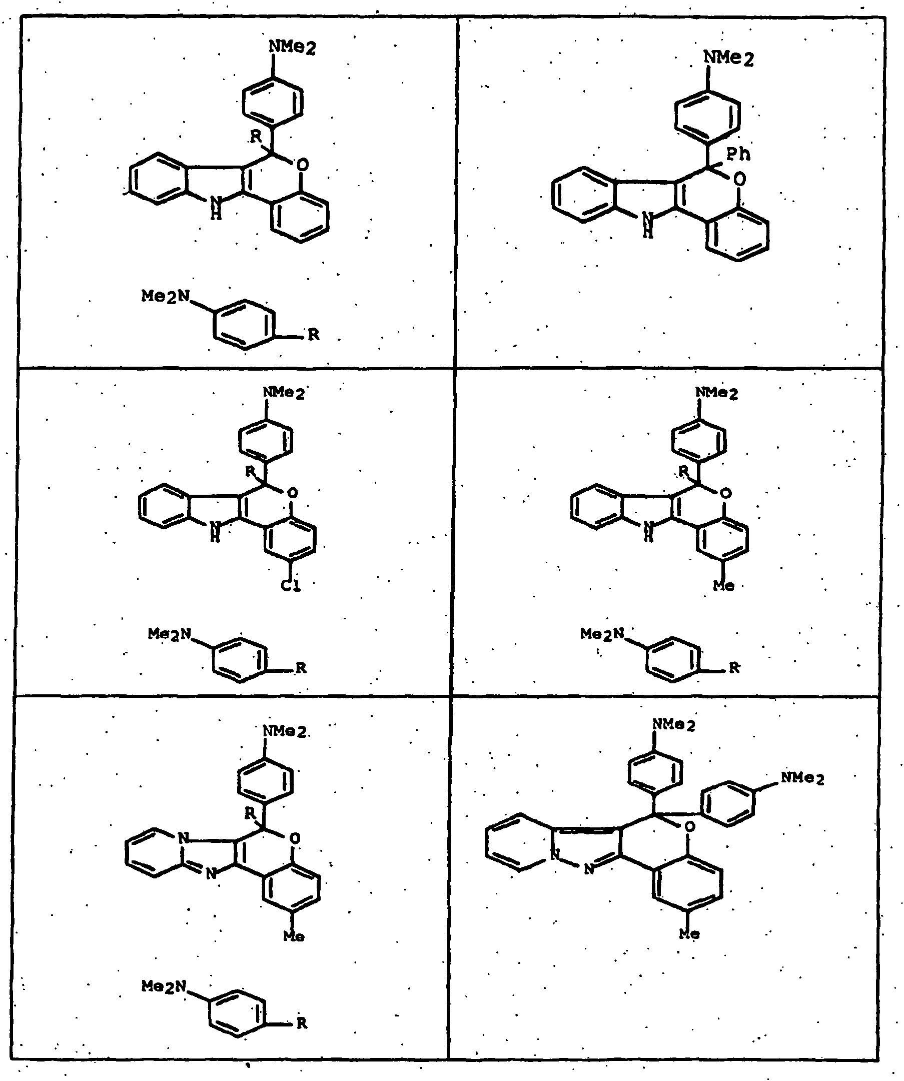

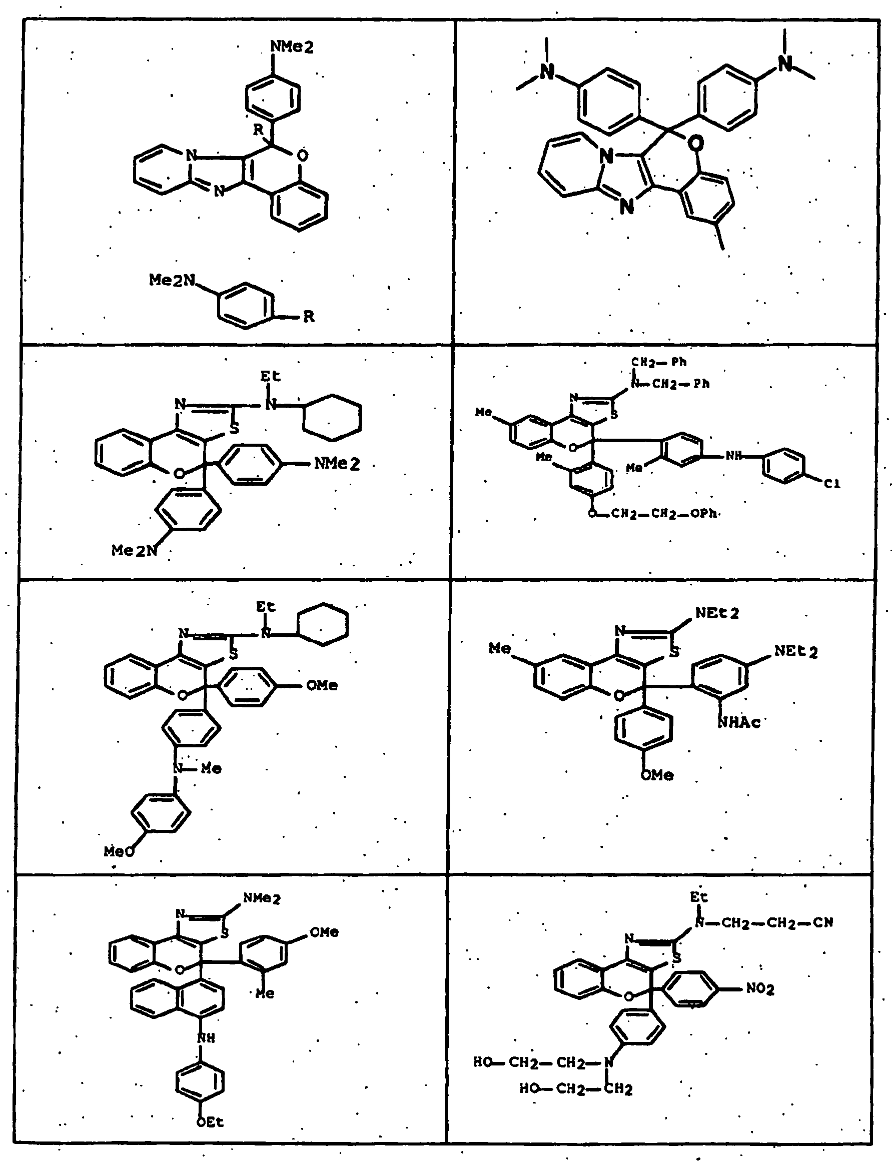

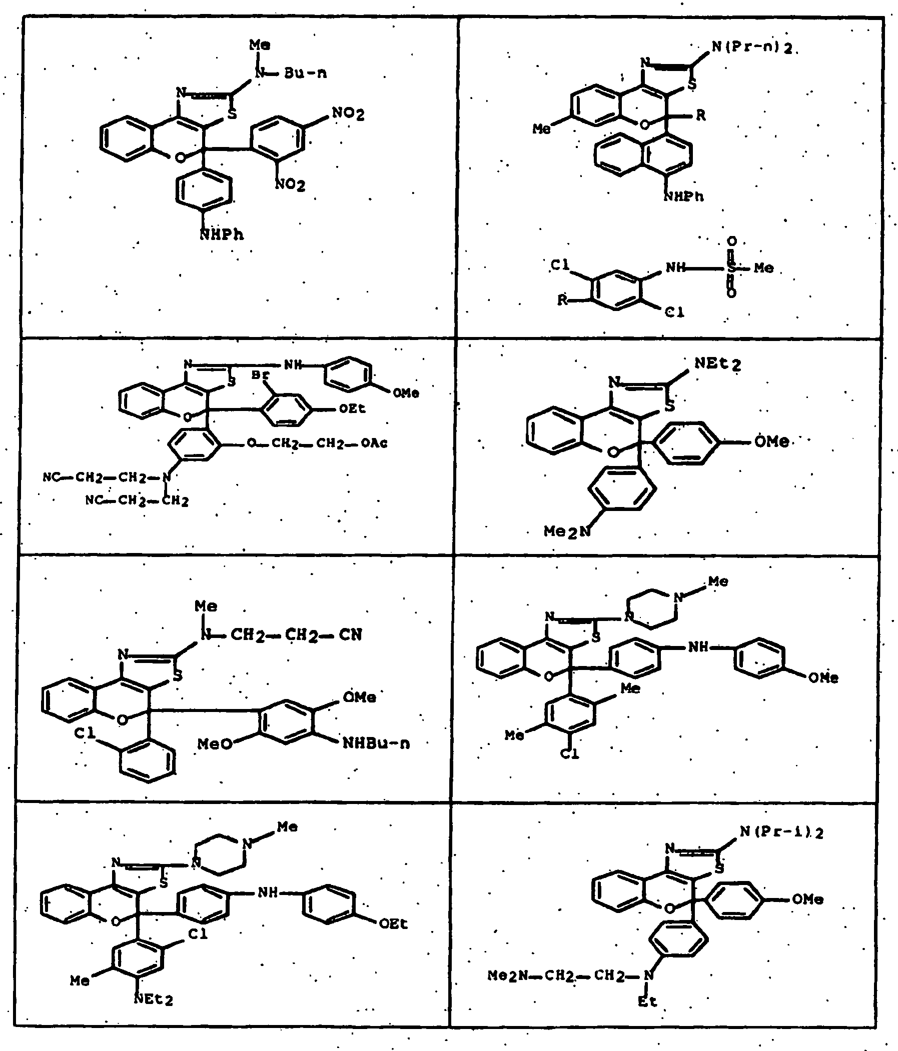

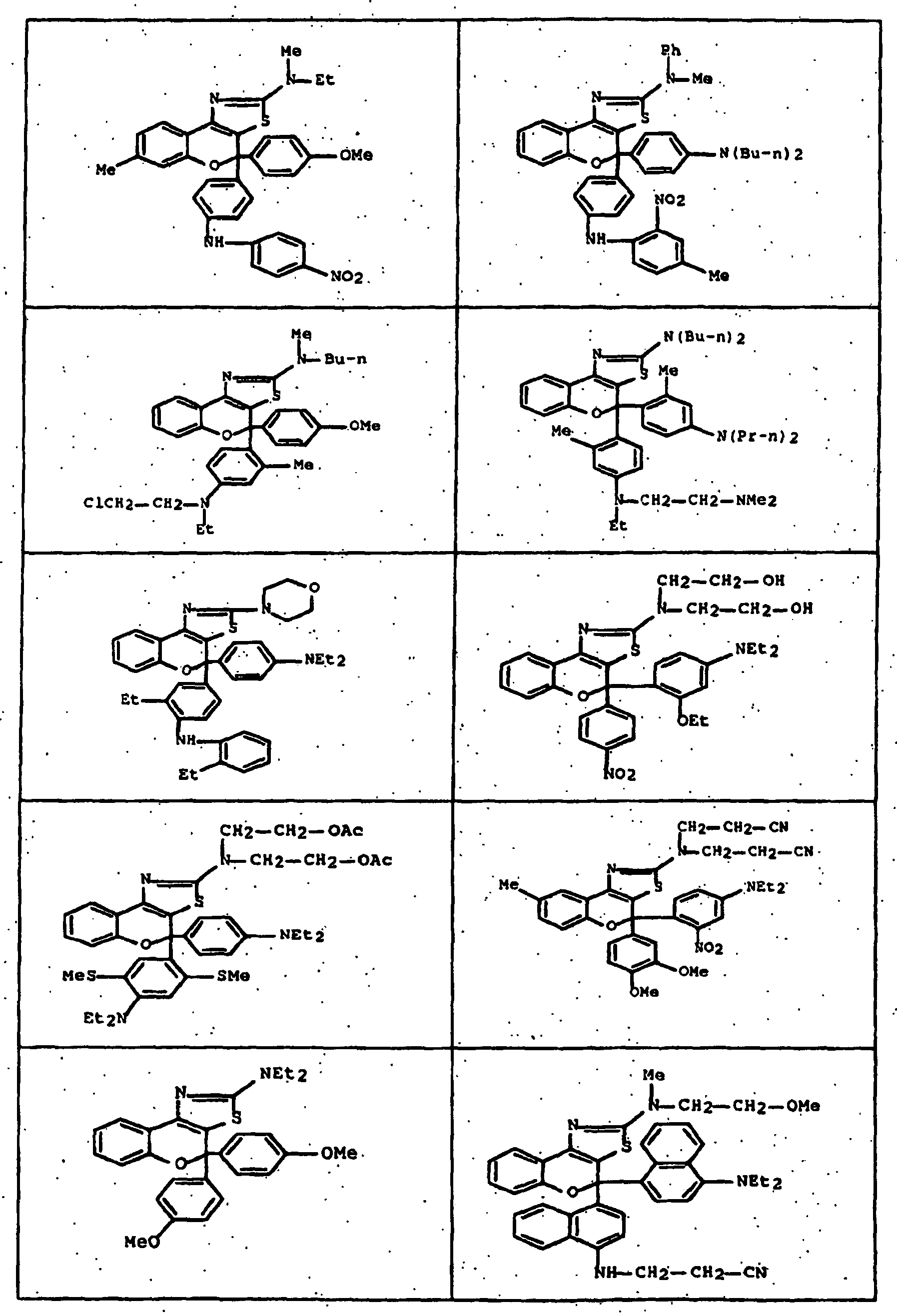

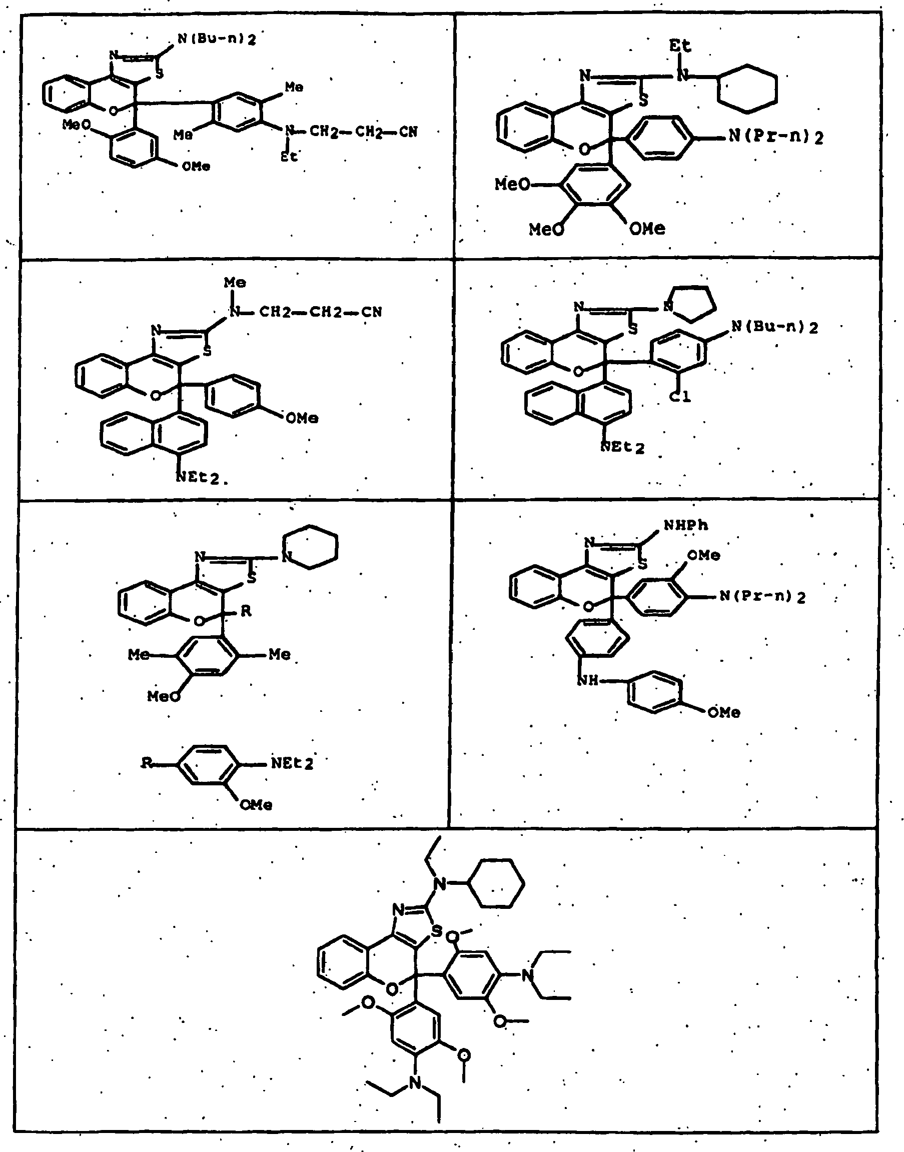

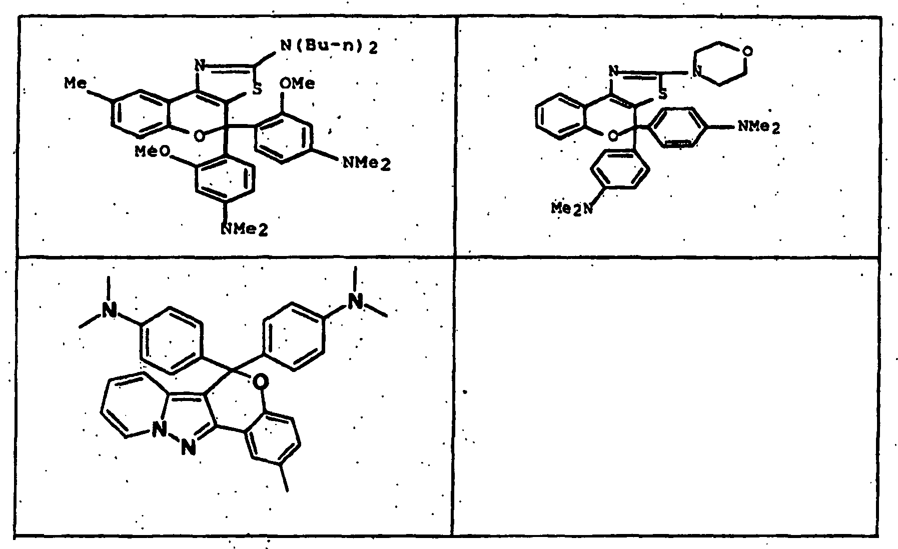

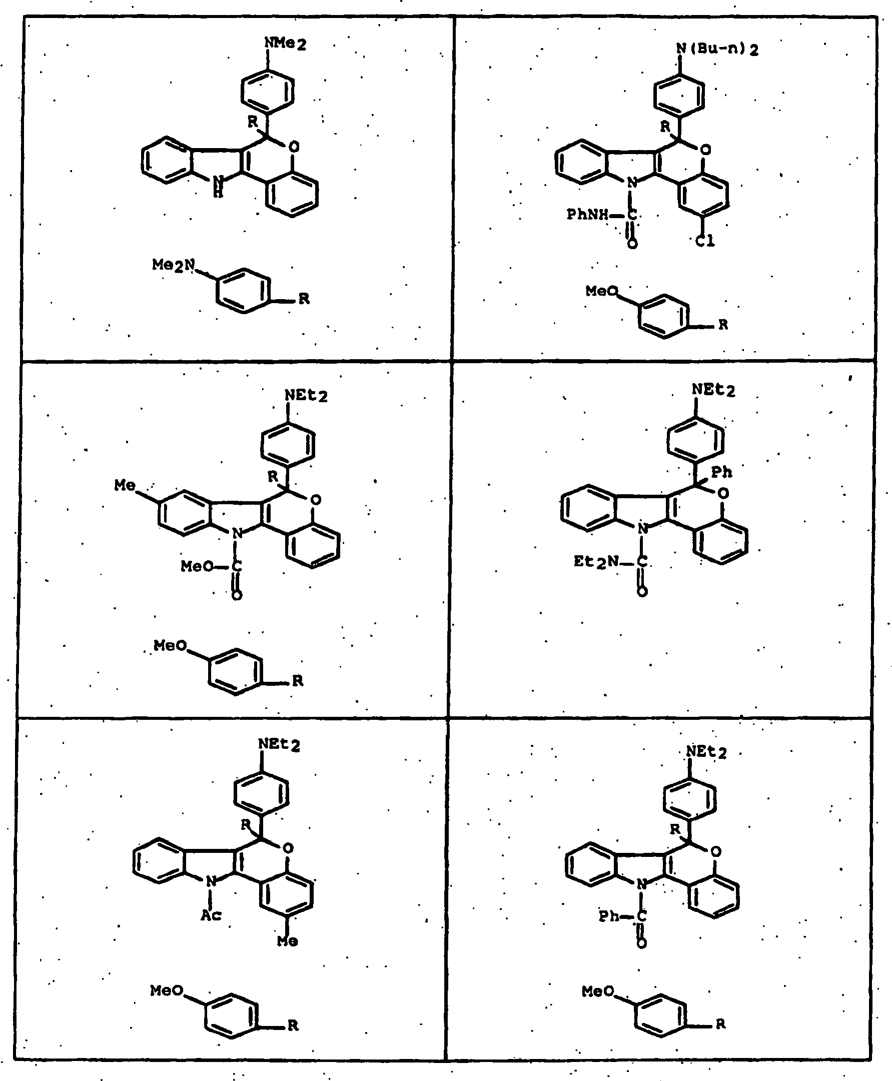

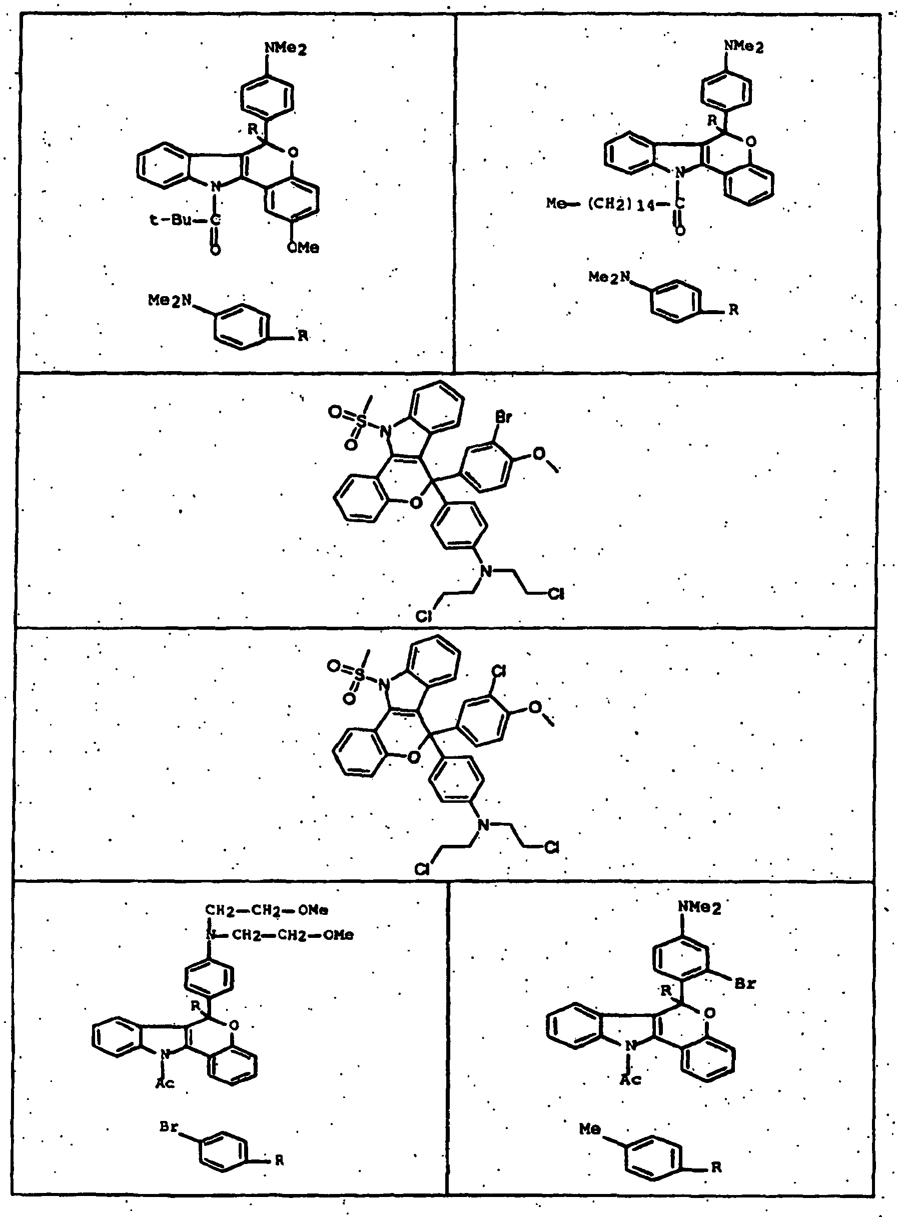

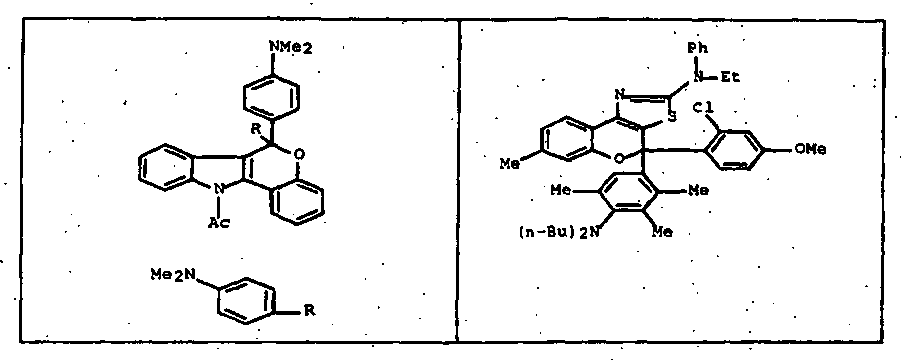

6. Utilisation selon l'une quelconque des revendications précédentes dans laquelle les composés choisis parmi les composés de formule (I), les colorants correspondant aux composés de formule (I) dont le cycle H est ouvert et leurs sels d'addition représentent de 0,001 à 10 % en poids, rapporté au poids total de la composition.

7. Utilisation selon l'une quelconque des revendications précédentes dans laquelle le milieu approprié pour la teinture est constitué par de l'eau ou par au moins un solvant organique ou par un mélange d'eau et d'au moins un solvant organique.

8. Utilisation selon la revendication 7 dans laquelle le milieu approprié pour la teinture est constitué par de l'eau ou par un mélange d'eau et d'au moins un solvant organique et le pH est compris entre 3 et 12.

9. Procédés de traitement des fibres kératiniques dans lequel les étapes suivantes sont mises en oeuvre :
a) application d'une composition tinctoriale telle que définie à l'une quelconque des revendications 1 à 18 pendant un temps de pose suffisant, le pH étant ajusté à l'aide d'un agent acidifiant ou d'un agent alcalinisant en fonction de la coloration souhaitée ;
b) éventuellement modification de la coloration des fibres kératiniques à l'aide d'un agent acidifiant ou d'un agent alcalinisant appliqué sur les fibres kératiniques.

10. Procédé selon la revendication 9 dans lequel, dans la première étape, l'agent acidifiant ou l'agent alcalinisant est mélangée à la composition tinctoriale avant application sur les fibres kératiniques.

11. Procédé selon la revendication 9 dans lequel, dans la première étape, l'agent acidifiant ou l'agent alcalinisant est appliqué avant ou après la composition tinctoriale.

12. Procédé selon l'une quelconque des revendications 9 à 11 dans lequel l'agent acidifiant est choisi parmi les acides minéraux tels que l'acide chlorhydrique, l'acide nitrique, l'acide sulfurique ou les acides organiques tels que les composés comprenant au moins une fonction acide carboxylique, une fonction acide sulfonique, une fonction acide phosphonique ou une fonction acide phosphorique.

13. Procédé selon l'une quelconque des revendications 9 à 12 dans lequel l'agent alcalinisant est choisi parmi:
- les acides aminés basiques ;
- les carbonates ou les bicarbonates de métaux alcalins ou alcalino-terreux ;
- les silicates ou les métasilicates ;
- les composés de formule (II) suivante :
X(OH)ₙ (II)
dans laquelle :
• X représente un ion potassium, lithium, sodium, ammonium N⁺R₁₇R₁₈R₁₉R₂₀ avec R₁₇, R₁₈, R₁₉ et R_{20,} identiques ou différents, désignant un radical alkyle en C₂-C₄ lorsque n est égal à 1 ;
• X représente un atome de magnésium ou de calcium lorsque n est égal à 2 ;
- les composés de formule (III) suivante : 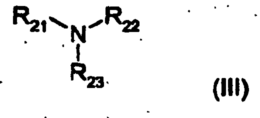 dans laquelle :
• R₂₁ représente un atome d'hydrogène ; une radical alkyle en C₁-C₆, une radical monohydroxyalkyle en C₁-C₆ ; une radical polyhydroxyalkyle en C₂-C₆ ;
• R₂₂ et R₂₃, identiques ou différents, représentent un atome d'hydrogène ; un radical alkyle en C₁-C₆ ; un radical monohydroxyalkyle en C₁-C₆ ; un radical polyhydroxyalkyle en C₂-C₆
- les composés de formule (IV) suivante: 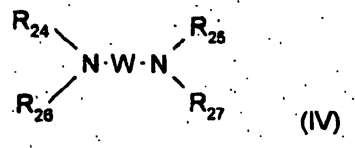 dans laquelle :
• W est un reste propylène éventuellement substitué par un groupement hydroxyle ou un radical alkyle en C₁-C₄ ;
• R₂₄, R₂₅, R₂₆ et R₂₇, identiques ou différents, représentent un atome d'hydrogène, un radical alkyle en C₁-C₄ ou hydroxyalkyle en C₁-C₄.

14. Composition telle que définie à l'une quelconque des revendications 1 à 8 comprenant de plus au moins un tensioactif et au moins un agent épaississant.

15. Composition selon la revendication 14 dans laquelle le ou les tensioactifs sont anioniques, cationiques, non-ioniques,amphotères, zwittérioniques ou leurs mélanges.

16. Composition selon la revendication 14 ou 15 dans laquelle le ou les agents épaississants sont des épaississants associatifs ou non associatifs.

17. Dispositif à plusieurs compartiments contenant dans un premier compartiment une composition telle que définie à l'une quelconque des revendications 14 à 16 et dans un deuxième compartiment un argent acidifiant ou un agent alcalinisant.

18. Dispositif selon la revendication 17 contenant dans le deuxième compartiment un agent acidifiant et dans un troisième compartiment un agent alcalinisant.

## Claims

1. Use in the colouring of keratinous fibres of a composition comprising, in a medium appropriate for dyeing, at least one compound chosen from the compounds of formula (I) comprising a cyclic group G including a ring H capable of opening, the dyes corresponding to the compounds of formula (I) in which the ring H is open and their addition salts: 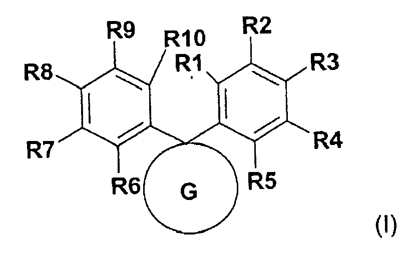 in which:
• **R₁**, **R₂**, **R₃**, **R₄**, **R₅**, **R₆**, **R₇**, **R₈**, **R₉** and **R₁₀** represent, independently of one another:
- a hydrogen atom;
- a halogen atom;
- a hydroxyl radical;
- a nitro radical;
- an amino radical;
- a carboxyl radical;
- an aminocarbonyl radical;
- a cyano radical;
- a radical resulting from a hydrocarbon chain which comprises from 1 to 100 carbon atoms, which is linear or branched, saturated or unsaturated and acyclic or monocyclic, in which the ring is aromatic or nonaromatic, or polycyclic, in which the rings are fused or unfused and aromatic or nonaromatic, which can be interrupted or terminated at one of its ends by one or more heteroatoms chosen from oxygen and sulphur atoms or by one or more groups chosen from carbonyl, SO, SO₂ and NH groups and which can be terminated at its other end by a hydrocarbonyl group or by a group comprising one or more heteroatoms chosen from nitrogen, oxygen and sulphur atoms, it being possible for the said hydrocarbon chain to be substituted by one or more groups chosen from the following radicals: hydroxyl, halo, carboxyl, carboxy(C₁-C₉) alkyl, cyano, amino, amino substituted by one or two C₁-C₄ alkyl groups, C₁-C₆ alkoxy, C₆-C₁₈ aryl, aryloxy, the aryl group of which is a C₆-C₁₈ group, or C₂-C₉ acyloxy;
it being possible for two of the **R₁**, **R₂**, **R₃**, **R₄**, **R₅**, **R₆**, **R₇**, **R₈**, **R₉** and **R₁₀** radicals carried by two adjacent carbon atoms to form, together and with the carbon atoms to which they are attached, a monocarbocyclic group in which the ring is aromatic or nonaromatic or a polycarbocyclic group in which the rings are fused or unfused and aromatic or nonaromatic, comprising from 5 to 20 ring members, it being possible for one or more carbon atoms to be replaced by an oxygen, nitrogen, sulphur or phosphorus atom, the mono- or polycarbocyclic group being unsubstituted or substituted by one or more substituents chosen from a halogen atom, a hydroxyl radical, an amino radical, a carboxyl radical, a C₆-C₁₈ aryl radical, a cyano radical, a C₁-C₉ alkyl radical, a C₁-C₉ alkoxy radical, a (C₁-C₉) alkoxycarbonyl (C₁-C₉)alkylamino radical or an α-naphthylalkylamino radical;
• **G** represents a divalent radical having one of the following formulae: 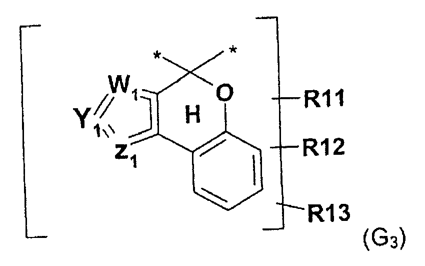 or 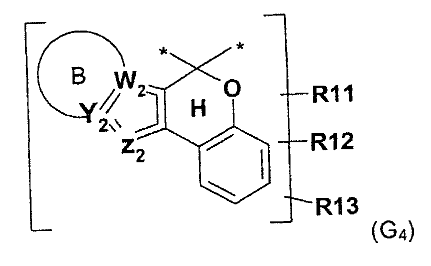 in which:
■ **Y₁**, **W₁** and **Z₁**, on the one hand, and **Y₂**, **W₂** and **Z₂**, on the other hand, represent, independently of one another, a sulphur atom, a carbon atom, a nitrogen atom or a divalent group CR₁₅ or NR₁₅;
■ **R₁₁**, **R₁₂**, **R₁₃** and **R₁₅** have, independently of one another, the same definitions as the **R₁** to **R₁₀** radicals;
it being possible for two of the **R₁₁**, **R₁₂** and **R₁₃** radicals carried by two adjacent carbon atoms to form, together and with the carbon atoms to which they are attached, a monocarbocyclic group in which the ring is aromatic or nonaromatic or a polycarbocyclic group in which the rings are fused or unfused and aromatic or nonaromatic, comprising from 5 to 20 ring members, it being possible for one or more carbon atoms to be replaced by an oxygen, nitrogen, sulphur or phosphorus atom, the mono- or polycarbocyclic group being unsubstituted or substituted by one or more substituents chosen from a halogen atom, a hydroxyl radical, an amino radical, a carboxyl radical, a C₆-C₁₈ aryl radical, a cyano radical, a C₁-C₉ alkyl radical or a C₁-C₉ alkoxy radical;
• **B** represents a C₆-C₁₈ aryl group or a heterocyclic group comprising from 5 to 12 ring members which is saturated or unsaturated and substituted or unsubstituted;
the amino radicals being unsubstituted or substituted by one or two identical or different radicals chosen from a C₁-C₉ alkyl radical; a C₁-C₉ hydroxyalkyl radical; a C₂-C₉ alkenyl radical; a C₅-C₁₂ cycloalkyl radical; a (C₆-C₁₈) arylcarbonyl radical; a cyclo(C₅-C₁₂)alkyl(C₁-C₉)alkyl radical; a (C₁-C₉) alkylcarbonyl radical; a (C₁-C₉)alkoxycarbonyl(C₁-C₉)alkyl radical; an α-naphthylalkyl radical; a C₁-C₉ haloalkyl radical; a (C₁-C₉)alkylcarbonyloxy(C₁-C₉)alkyl radical; a C₁-C₉ cyanoalkyl radical; a C₂-C₁₅ acyl radical; a (C₁-C₉) alkoxycarbonyl radical; a (C₆-C₁₈)aryloxycarbonyl radical; a (C₆-C₁₈) aryloxy (C₁-C₉) alkylcarbonyl radical; a (C₆-C₁₈) aryl (C₁-C₉) alkoxycarbonyl radical; a (C₁-C₉) alkoxy (C₆-C₁₈) arylcarbonyl radical; a di(C₁-C₉)alkylaminocarbonyl radical; a di(C₁-C₉)alkylaminosulphonyl radical; a (C₁-C₉) alkyl (C₆-C₁₈) arylsulphonyl radical; a (C₁-C₉) alkylsulphonyl radical; a di(C₁-C₉)alkylamino (C₁-C₉) alkyl radical; a (C₁-C₉) alkoxy (C₁-C₉) alkyl radical; a C₆-C₁₈ aryl radical and a (C₆-C₁₈)aryl(C₁-C₉)alkyl radical optionally substituted on the aryl nucleus by one or more substituents chosen from a halogen atom, a C₁-C₉ alkyl radical, a nitro radical, a di(C₁-C₉)alkylamino radical and a C₁-C₉ alkoxy radical; it being possible for the two radicals to form, together and with the nitrogen atom of the amino group, a 5- to 12- membered ring optionally carrying another heteroatom, it being possible for the said ring to be substituted by a C₁-C₉ alkyl radical.

2. Use according to Claim 1, in which the compound or compounds of formula (I) are such that:
• **R₅** represents a hydrogen atom;
• **R₁** represents:
- a hydrogen atom;
- a halogen atom;
- a C₁-C₉ alkyl radical;
- a C₁-C₉ alkoxy radical;
- a nitro radical;
- an amino radical;
- a (C₁-C₉)alkylthio radical;
- a (C₂-C₉)acyloxy(C₁-C₉)alkoxy radical;
• **R₂** represents a hydrogen atom; a C₁-C₉ alkyl radical; a C₁-C₉ alkoxy radical; a halogen atom; an amino radical;
• **R₃** represents a hydrogen atom; an amino radical; a halogen atom; a (C₆-C₁₈)aryloxy(C₁-C₉)alkoxy radical; a nitro radical; a C₁-C₉ alkoxy radical;
• **R₄** represents a hydrogen atom; a halogen atom; a C₁-C₉ alkyl radical; a C₁-C₉ alkoxy radical; a (C₁-C₉)alkylthio radical;
• **R₆** represents a hydrogen atom; a halogen atom; a C₁-C₉ alkyl radical; a C₁-C₉ alkoxy radical; a nitro radical; an amino radical; a (C₂-C₉)acyloxy(C₁-C₉)alkoxy radical;
• **R₇** represents a hydrogen atom; a halogen atom; a C₁-C₉ alkyl radical; a C₁-C₉ alkoxy radical;
• **R₈** represents a hydrogen atom; a halogen atom; a C₁-C₉ alkyl radical; a C₁-C₉ alkoxy radical; a nitro radical; an amino radical;
• **R₉** represents a hydrogen atom; a C₁-C₉ alkyl radical; a C₁-C₉ alkoxy radical;
• **R₁₀** represents a hydrogen atom; a C₁-C₉ alkoxy radical;
• **R₁₁** represents a hydrogen atom; a halogen atom; a C₁-C₉ alkyl radical; a C₁-C₉ alkoxy radical; a C₆-C₁₈ aryl radical; an amino radical; a C₂-C₁₈ acyl radical; a (C₆-C₁₈)arylsulphonyl radical;
• **R₁₂** represents a hydrogen atom; a C₁-C₉ alkyl radical; a C₁-C₉ alkoxy radical; an amino radical;
• **R₁₃** represents a hydrogen atom;
• it being possible for **R₁** and **R₂** and/or **R₂** and **R₃** and/or **R₆** and **R₇** and/or **R₇** and **R₈** to form, together and with the carbon atoms to which they are attached, a monocarbocyclic or polycarbocyclic group in which the rings are aromatic and fused or unfused, which comprises from 5 to 20 ring members, it being possible for one or more carbon atoms to be replaced by an oxygen, nitrogen, sulphur or phosphorus atom, and which is unsubstituted or substituted.

3. Use according to Claim 1, in which the compound or compounds of formula (I) are chosen from the compounds of following formula (I₁): 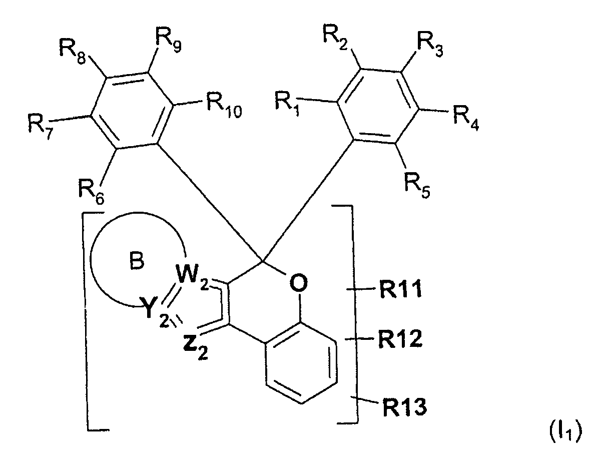 in which:
**Y₂**, **W₂** and **Z₂** represent, independently of one another, a carbon or nitrogen or sulphur atom or a divalent group CR₁₅ or NR₁₅;
B represents a C₆-C₁₈ aryl group or a 5- to 12- membered heterocyclic group which is saturated or unsaturated and substituted or unsubstituted;
• **R₁** represents a hydrogen atom; a halogen atom; a C₁-C₉ alkoxy radical; a C₁-C₉ alkyl radical; a nitro radical; a (C₁-C₉)alkylthio radical;
• **R₂** represents a hydrogen atom; a halogen atom; a C₁-C₉ alkyl radical; a C₁-C₉ alkoxy radical;
• **R₃** represents a hydrogen atom; a halogen atom; a nitro radical; an amino radical; a C₁-C₉ alkoxy radical;
• **R₄** represents a hydrogen atom; a C₁-C₉ alkoxy radical; a C₁-C₉ alkyl radical; a (C₁-C₉)alkylthio radical;
• **R₅**, **R₁₀** and **R₁₃** represent a hydrogen atom;
• **R₆** represents a hydrogen atom; a halogen atom; a nitro radical; a C₁-C₉ alkoxy radical; a C₁-C₉ alkyl radical; an amino radical; a (C₂-C₉)acyloxy(C₁-C₉)alkoxy radical;
• **R₇** represents a hydrogen atom; a C₁-C₉ alkoxy radical;
• **R₈** represents a hydrogen atom; a halogen atom; a C₁-C₉ alkoxy radical; a C₁-C₉ alkyl radical; an amino radical;
• **R₉** represents a hydrogen atom; a C₁-C₉ alkoxy radical; a C₁-C₉ alkyl radical;
• **R₁₁** represents a hydrogen atom; a halogen atom; an amino radical; a C₁-C₉ alkyl radical; a di(C₁-C₉)alkylaminocarbonyl radical; a (C₆-C₁₈) arylcarbonyl radical; a (C₆-C₁₈) arylsulphonyl radical; a C₂-C₉ acyl radical;
• **R₁₂** represents a hydrogen atom; a C₁-C₉ alkyl radical; a (C₆-C₁₈)arylaminocarbonyl radical; a C₂-C₂₀ acyl radical; a (C₁-C₉)alkoxycarbonyl radical;
it being possible for **R₁** and **R₂**, on the one hand, and **R₆** and **R₇**, on the other hand, to form, together and with the carbon atoms to which they are attached, an aromatic C₆-C₁₈ ring.

4. Use according to Claim 1, in which the compound or compounds of formula (I) are chosen from one of the following formulae (I₂) to (I₆): 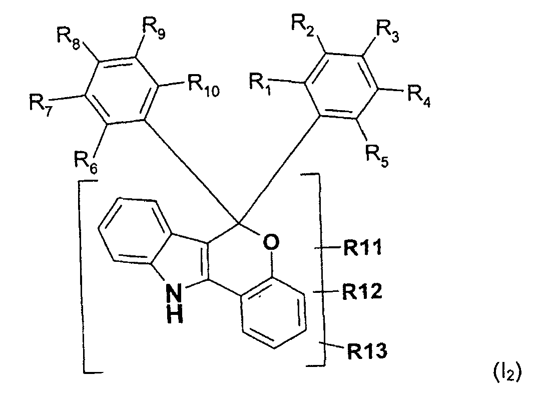 or 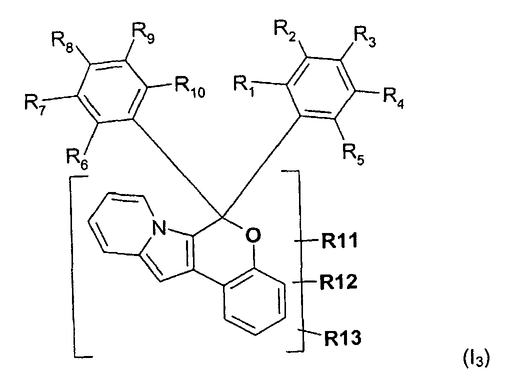 or 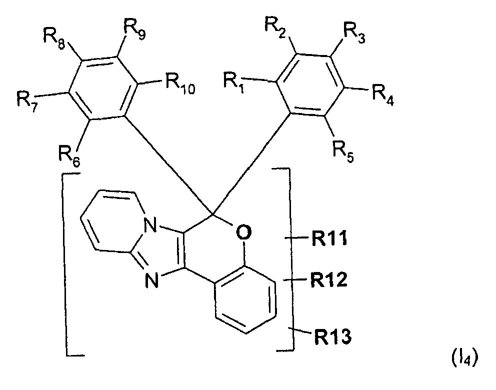 or 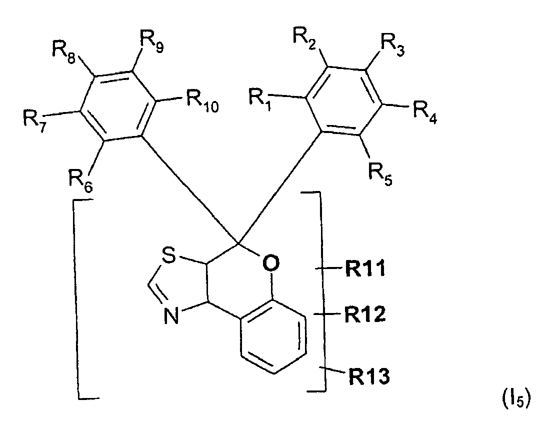 or 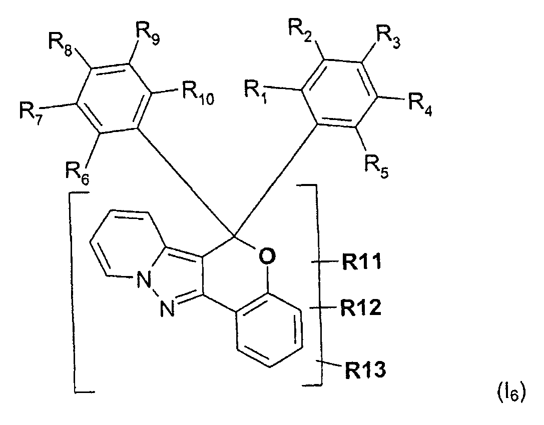 in which:
• **R₁** represents a hydrogen atom; a halogen atom; a C₁-C₉ alkoxy radical; a C₁-C₉ alkyl radical; a nitro radical; a (C₁-C₉)alkylthio radical;
• **R₂** represents a hydrogen atom; a halogen atom; a C₁-C₉ alkyl radical; a C₁-C₉ alkoxy radical;
• **R₃** represents a hydrogen atom; a halogen atom; a nitro radical; an amino radical; a C₁-C₉ alkoxy radical;
• **R₄** represents a hydrogen atom; a C₁-C₉ alkoxy radical; a C₁-C₉ alkyl radical; a (C₁-C₉) alkylthio radical;
• **R₅**, **R₁₀** and **R₁₃** represent a hydrogen atom;
• **R₆** represents a hydrogen atom; a halogen atom; a nitro radical; a C₁-C₉ alkoxy radical; a C₁-C₉ alkyl radical; an amino radical; a (C₂-C₉)acyloxy(C₁-C₉)alkoxy radical;
• **R₇** represents a hydrogen atom; a C₁-C₉ alkoxy radical;
• **R₈** represents a hydrogen atom; a halogen atom; a C₁-C₉ alkoxy radical; a C₁-C₉ alkyl radical; an amino radical;
• **R₉** represents a hydrogen atom; a C₁-C₉ alkoxy radical; a C₁-C₉ alkyl radical;
• **R₁₁** represents a hydrogen atom; a halogen atom; an amino radical; a C₁-C₉ alkyl radical; a di(C₁-C₉)alkylaminocarbonyl radical; a (C₆-C₁₈) arylcarbonyl radical; a (C₆-C₁₈) arylsulphonyl radical; a C₂-C₉ acyl radical;
• **R₁₂** represents a hydrogen atom; a C₁-C₉ alkyl radical; a (C₆-C₁₈)arylaminocarbonyl radical; a C₂-C₂₀ acyl radical; a (C₁-C₉) alkoxycarbonyl radical;
it being possible for **R₁** and **R₂**, on the one hand, and **R₆** and **R₇**, on the other hand, to form, together and with the carbon atoms to which they are attached, an aromatic C₆-C₁₈ ring.

5. Use according to any one of the preceding claims, in which the compound of formula (I) is chosen from the following compounds:

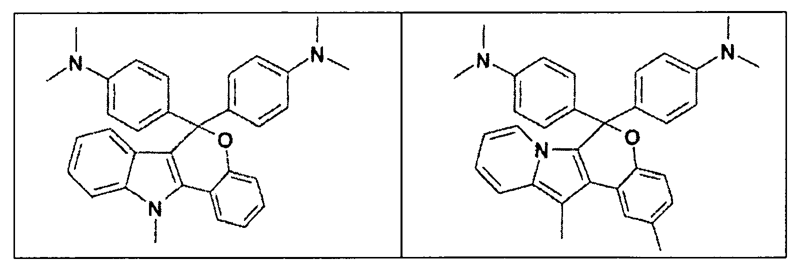

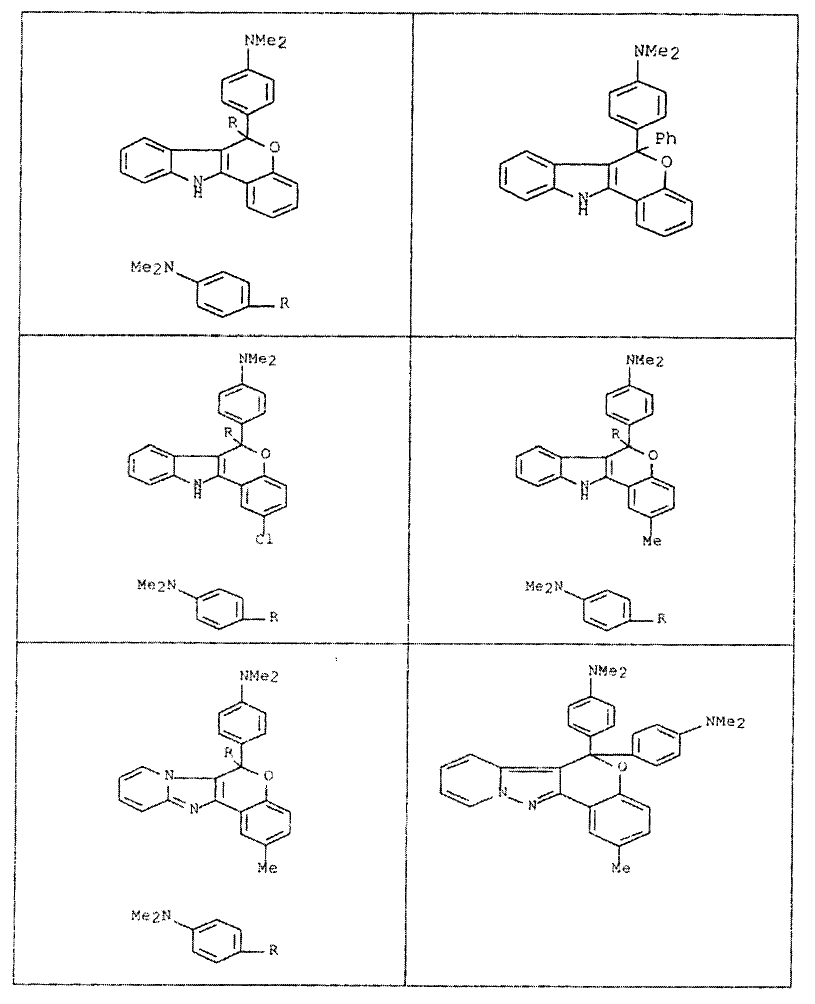

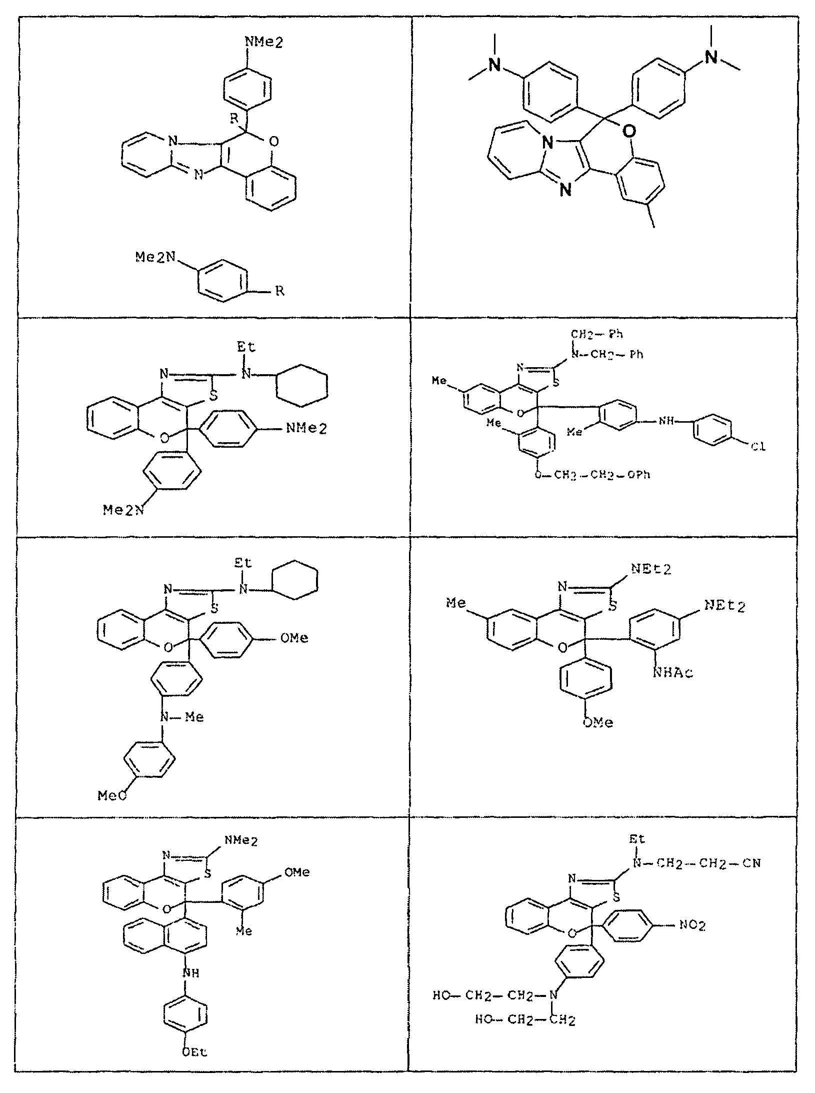

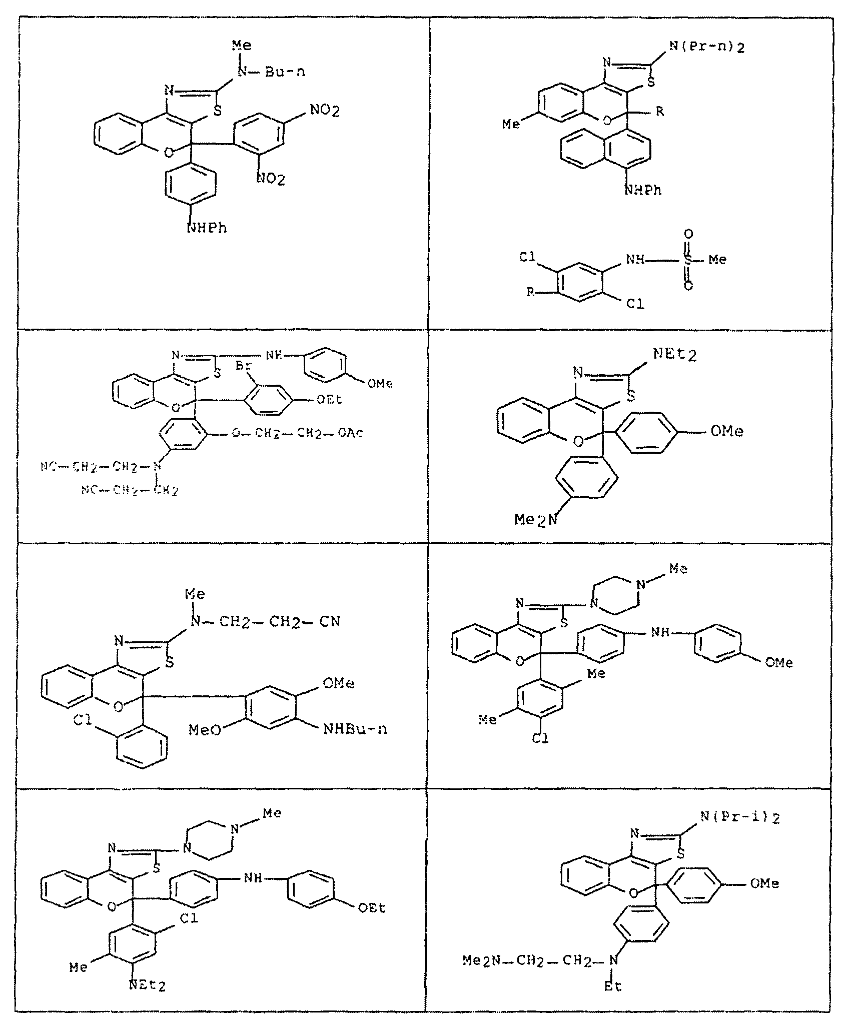

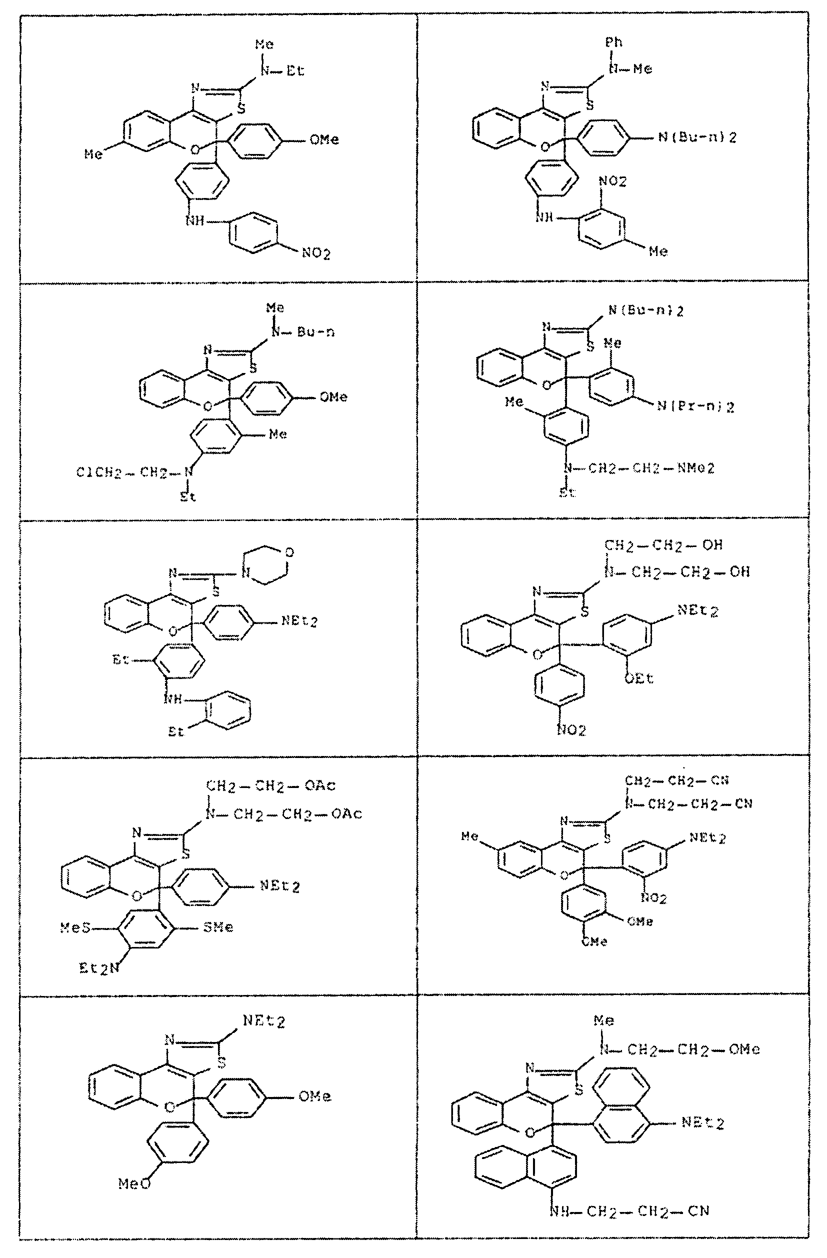

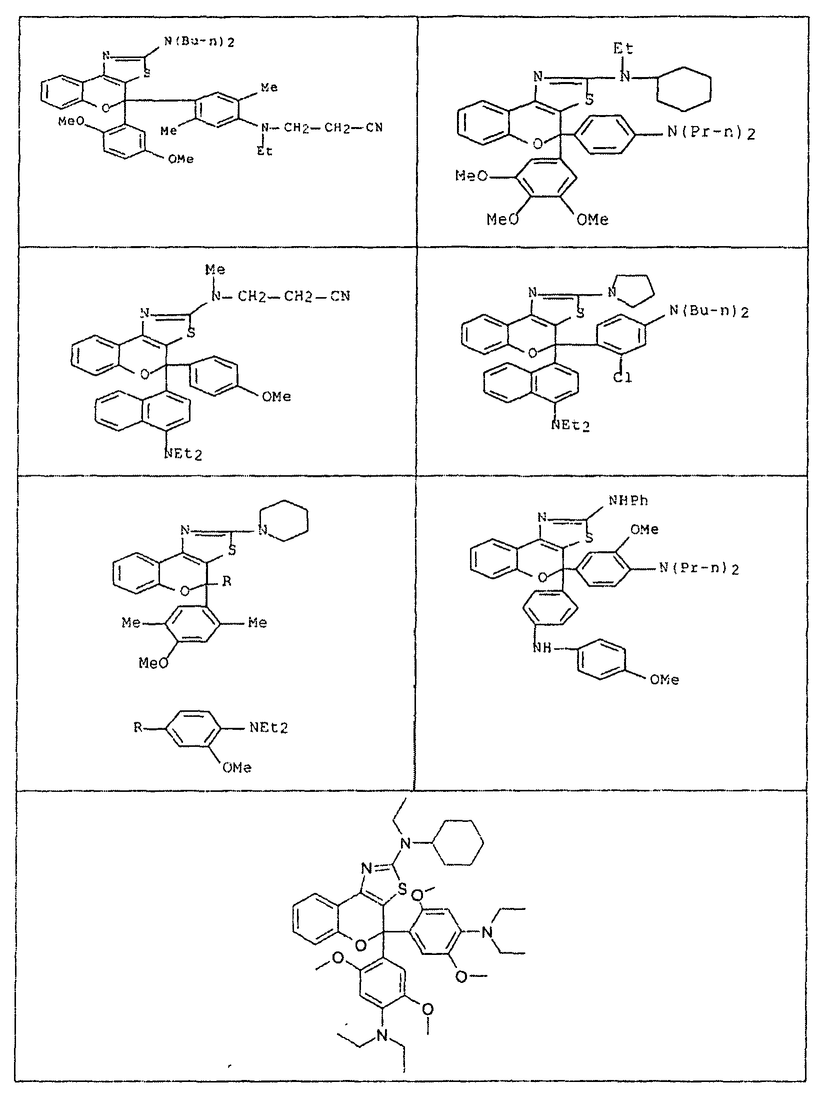

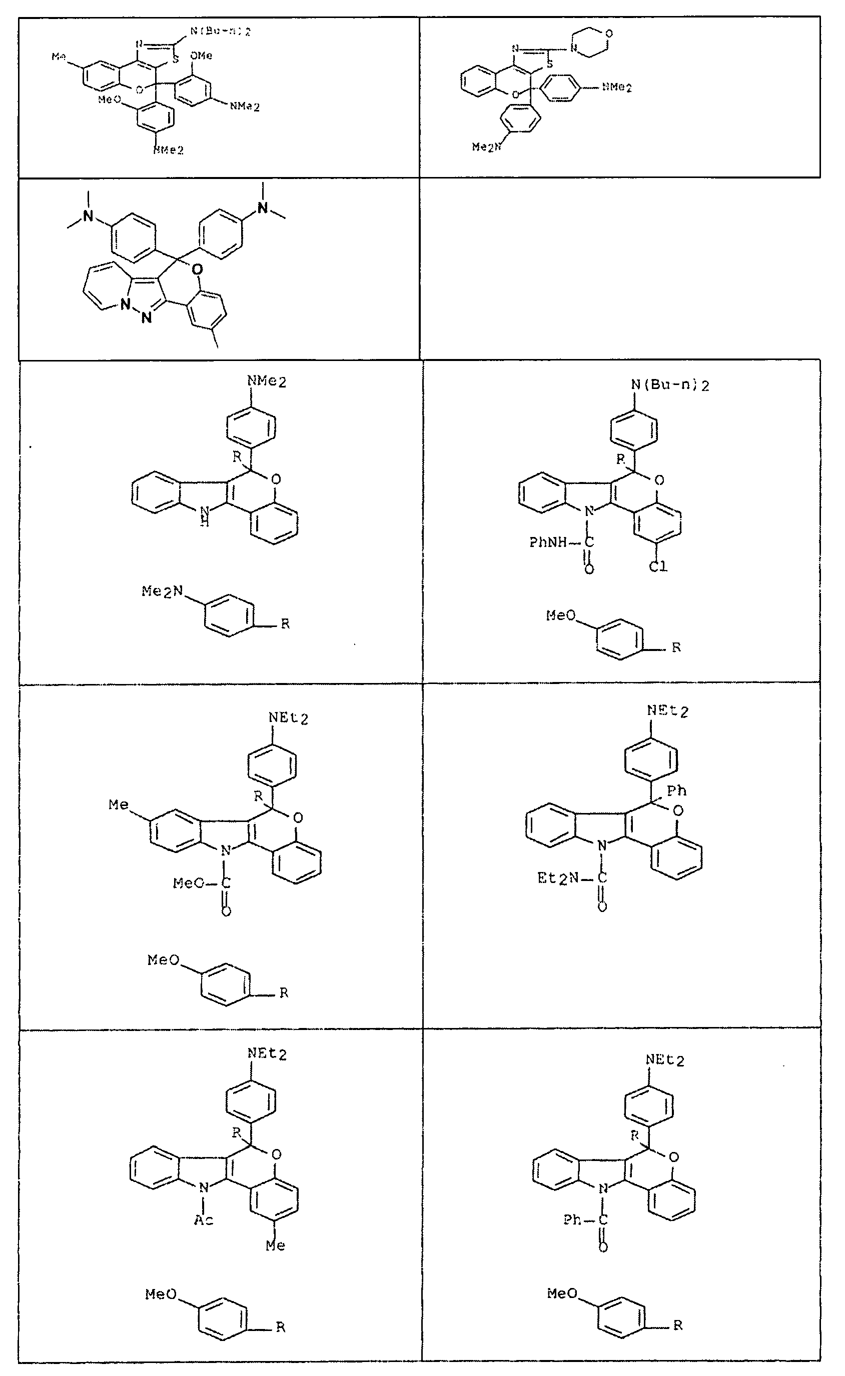

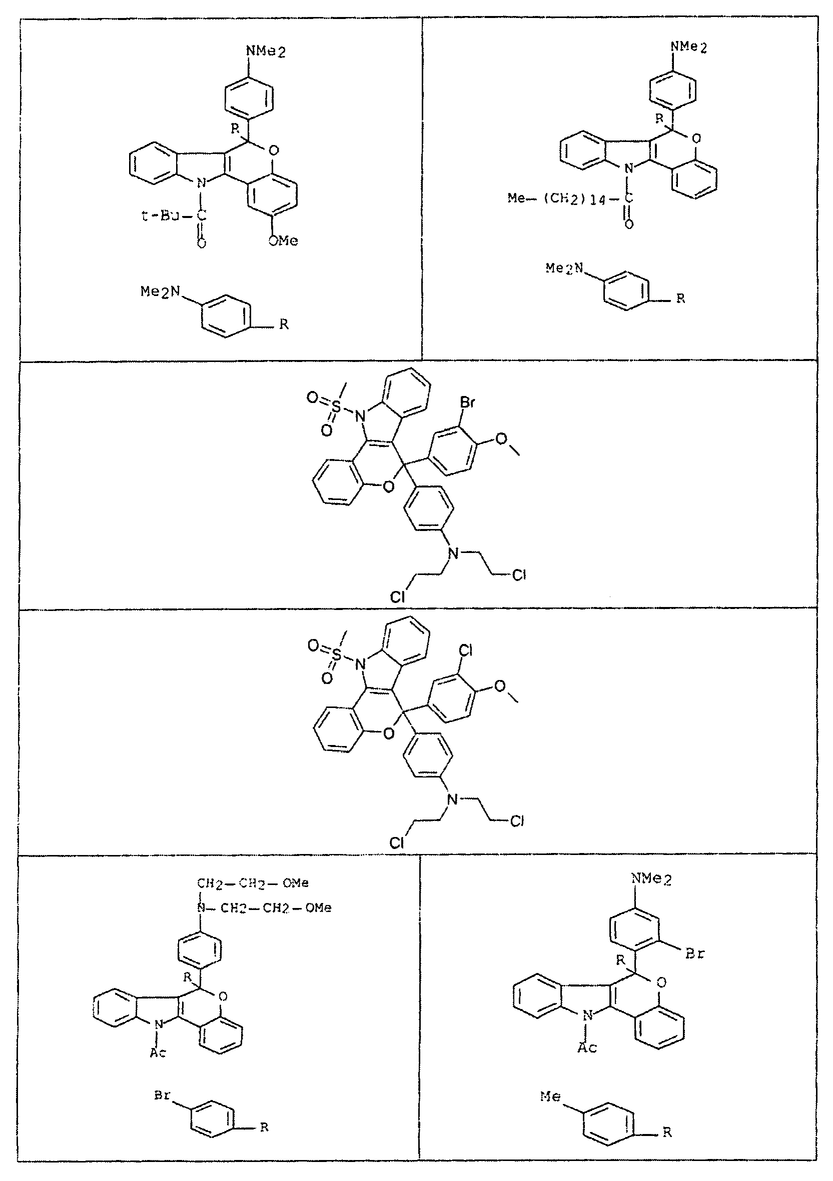

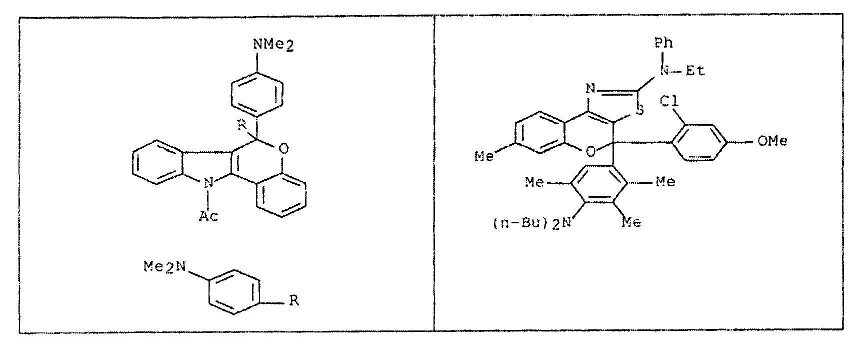

6. Use according to any one of the preceding claims, in which the compounds chosen from the compounds of formula (I), the dyes corresponding to the compounds of formula (I) in which the ring H is open and their addition salts represent from 0.001 to 10% by weight, with respect to the total weight of the composition.

7. Use according to any one of the preceding claims, in which the medium appropriate for dyeing is composed of water or of at least one organic solvent or of a mixture of water and of at least one organic solvent.

8. Use according to Claim 7, in which the medium appropriate for dyeing is composed of water or of a mixture of water and of at least one organic solvent and the pH is between 3 and 12.

9. Method for the treatment of keratinous fibres, in which the following stages are carried out:
a) application of a dyeing composition as defined in any one of Claims 1 to 8 for a sufficient development time, the pH being adjusted using an acidifying agent or a basifying agent according to the colouring desired;
b) optionally modification of the colouring of the keratinous fibres using an acidifying agent or a basifying agent applied to the keratinous fibres.

10. Method according to Claim 9, in which, in the first stage, the acidifying agent or the basifying agent is mixed with the dyeing composition before application to the keratinous fibres.

11. Method according to Claim 9, in which, in the first stage, the acidifying agent or the basifying agent is applied before or after the dyeing composition.

12. Method according to any one of Claims 9 to 11, in which the acidifying agent is chosen from inorganic acids, such as hydrochloric acid, nitric acid or sulphuric acid, or organic acids, such as compounds comprising at least one carboxylic acid functional group, one sulphonic acid functional group, one phosphonic acid functional group or one phosphoric acid functional group.

13. Method according to any one of Claims 9 to 12, in which the basifying agent is chosen from:
- basic amino acids;
- alkali metal or alkaline earth metal carbonates or bicarbonates;
- silicates or metasilicates;
- compounds of following formula (II):
X(OH)ₙ (II)
in which:
• X represents a potassium, lithium, sodium, ammonium or N⁺R₁₇R₁₈R₁₉R₂₀ ion with R₁₇, R₁₈, R₁₉ and R₂₀, which are identical or different, denoting a C₂-C₄ alkyl radical, when n is equal to 1;
• X represents a magnesium or calcium atom, when n is equal to 2;
- compounds of following formula (III): 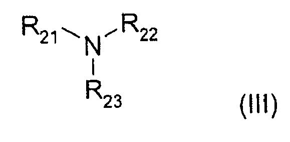 in which:
• R₂₁ represents a hydrogen atom, a C₁-C₆ alkyl radical, a C₁-C₆ monohydroxyalkyl radical or a C₂-C₆ polyhydroxyalkyl radical;
• R₂₂ and R₂₃, which are identical or different, represent a hydrogen atom, a C₁-C₆ alkyl radical, a C₁-C₆ monohydroxyalkyl radical or a C₂-C₆ polyhydroxyalkyl radical;
- compounds of following formula (IV): 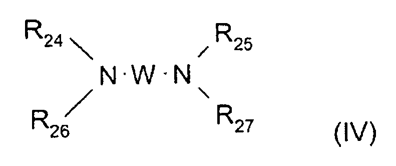 in which:
• W is a propylene residue optionally substituted by a hydroxyl group or a C₁-C₄ alkyl radical;
• R₂₄, R₂₅, R₂₆ and R₂₇, which are identical or different, represent a hydrogen atom, a C₁-C₉ alkyl radical or a C₁-C₄ hydroxyalkyl radical.

14. Composition as defined in any one of Claims 1 to 8, additionally comprising at least one surfactant and at least one thickening agent.

15. Composition according to Claim 14, in which the surfactant or surfactants are anionic, cationic, non-ionic, amphoteric or zwitterionic surfactants or their mixtures.

16. Composition according to Claim 14 or 15, in which the thickening agent or agents are associative or non-associative thickeners.

17. Multicompartment device comprising, in a first compartment, a composition as defined in any one of Claims 14 to 16 and, in a second compartment, an acidifying agent or a basifying agent.

18. Device according to Claim 17, comprising, in the second compartment, an acidifying agent, and in a third compartment, a basifying agent.

## Patentansprüche

1. Verwendung einer Zusammensetzung, die in einem zum Färben geeigneten Medium mindestens eine Verbindung enthält, die unter den Verbindungen der Formel (I), die eine cyclische Gruppe G enthalten, welche einen Ring H umfasst, der zur Öffnung befähigt ist, und den Farbstoffen, die den Verbindungen der Formel (I) entsprechen, deren Ring H geöffnet ist, und ihren Additionssalzen ausgewählt ist, zum Färben von Keratinfasern: 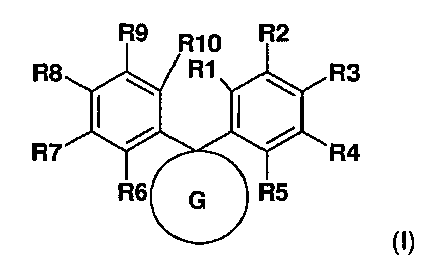 in der Formel:
die Gruppen R₁, R₂, R₃, R₄, R₅, R₆, R₇, R₈, R₉ und R₁₀ bedeuten unabhängig voneinander:
- ein Wasserstoffatom;
- ein Halogenatom;
- eine Hydroxygruppe,
- eine Nitrogruppe;
- eine Aminogruppe;
- eine Carboxygruppe;
- eine Aminocarbonylgruppe;
- eine Cyanogruppe;
- eine Gruppe, die von einer geradkettigen oder verzweigten, gesättigten oder ungesättigten Kohlenwasserstoffkette mit 1 bis 100 Kohlenstoffatomen abgeleitet ist, die acyclisch oder monocyclisch ist und bei der der Ring aromatisch oder nichtaromatisch vorliegt oder die polycyclisch ist und bei der die Ringe kondensiert oder nicht kondensiert, aromatisch oder nichtaromatisch sind, die durch ein oder mehrere Heteroatome, die unter Sauerstoff und Schwefel ausgewählt sind, oder durch eine oder mehrere Carbonylgruppen, SO-Gruppen, SO₂-Gruppen und NH-Gruppen unterbrochen sein oder an einem ihrer Enden abgeschlossen sein kann, die an ihrem anderen Ende durch eine Hydrogenocarbonylgruppe oder durch eine Gruppe abgeschlossen werden kann, die ein der mehrere Heteroatome enthält, die unter Stickstoff, Sauerstoff und Schwefel ausgewählt sind, wobei die Kohlenwasserstoffkette mit einem oder mehreren Substituenten substituiert sein kann, die unter den Substituenten Hydroxy, Halogen, Carboxy, Carboxyalkyl(C₁₋₉), Cyano, Amino, mit einer oder zwei C₁₋₄-Alkylgruppen substituierten Aminogruppen, Alkoxy(C₁₋₆), Aryl(C₆₋₁₈), Aryloxy-(C₁₋₆), deren Arylgruppe 6 bis 18 Kohlenstoffatome aufweist, Acyloxy(C₂₋₉) ausgewählt sind;
zwei der Gruppen R₁, R₂, R₃, R₄, R₅, R₆, R₇, R₈, R₉ und R₁₀, die von zwei angrenzenden Kohlenstoffatomen getragen werden, können gemeinsam und mit den Kohlenstoffatomen, an die sie gebunden sind, eine monocarbocyclische Gruppe, bei der der Ring aromatisch oder nichtaromatisch vorliegt, oder eine polycarbocyclische Gruppe bilden, bei der die Ringe kondensiert oder nicht kondensiert und aromatisch oder nichtaromatisch sind, wobei die Gruppe 5- bis 20-gliedrig ist, wobei ein oder mehrere Kohlenstoffatome durch ein Sauerstoffatom, Stickstoffatom, Schwefelatom oder Phosphoratom ersetzt sein können, wobei die mono- oder polycarbocyclische Gruppe unsubstituiert vorliegt oder mit einer oder mehreren Gruppen substituiert ist, die ausgewählt sind unter einem Halogenatom, einer Hydroxygruppe, einer Aminogruppe, einer Carboxygruppe, einer Aryl(C₆₋₁₈)gruppe, einer Cyanogruppe, einer Alkyl(C₁₋₉)-gruppe; einer Alkoxy(C₁₋₉)gruppe, einer Alkoxy(C₁₋₉)carbonylalkyl(C₁₋₉)aminogruppe; einer α-Naphthylalkylaminogruppe;
G bedeutet eine zweiwertige Gruppe einer der folgenden Formeln: 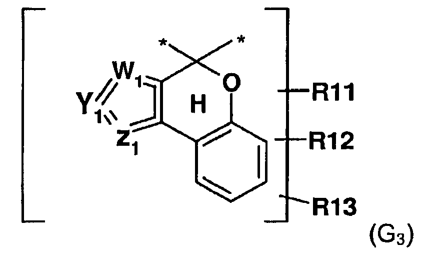 oder 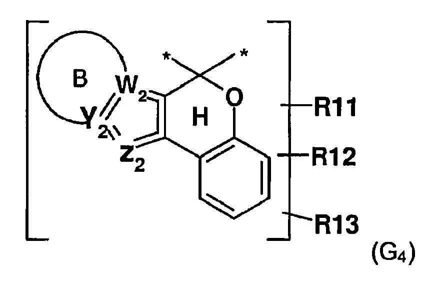 worin bedeuten:
Y₁, W₁ und Z₁ einerseits und Y₂, W₂ und Z₂ andererseits unabhängig voneinander ein Schwefelatom, ein Kohlenstoffatom, ein Stickstoffatom oder eine zweiwertige Gruppe CR₁₅ oder NR₁₅:
R₁₁, R₁₂, R₁₃ und R₁₅ unabhängig voneinander die für die Gruppen R₁ bis R₁₀ angegebenen Bedeutungen;
zwei der Gruppen R_{11,} R₁₂ und R₁₃, die von zwei aneinander angrenzenden Kohlenstoffatomen getragen werden, können gemeinsam und mit den Kohlenstoffatomen, an die sie gebunden sind, eine monocarbocyclische Gruppe, bei der der Ring aromatisch oder nichtaromatisch vorliegt, oder eine polycarbocyclische Gruppe bilden, bei der die Ringe kondensiert oder nicht kondensiert und aromatisch oder nichtaromatisch sind, wobei die Gruppe 5- bis 20-gliedrig ist, wobei ein oder mehrere Kohlenstoffatome durch ein Sauerstoffatom, Stickstoffatom, Schwefelatom oder Phosphoratom ersetzt sein können und wobei die mono- oder polycarbocyclische Gruppe unsubstituiert vorliegt oder mit einer oder mehreren Gruppen substituiert ist, die unter einem Halogenatom, einer Hydroxygruppe, einer Aminogruppe, einer Carboxygruppe, einer Aryl(C₆₋₁₈)gruppe, einer Cyanogruppe, einer Alkyl(C₁₋₉)gruppe; einer Alkoxy(C₁₋₉)-gruppe ausgewählt sind;
B bedeutet eine Aryl(C₆₋₁₈)gruppe oder eine 5- bis 12-gliedrige heterocyclische Gruppe, die gesättigt oder ungesättigt, substituiert oder unsubstituiert ist;
wobei die Aminogruppen unsubstituiert oder mit einer oder mit zwei gleichen oder voneinander verschiedenen Gruppen substituiert sind, die ausgewählt sind unter einer Alkyl(C₁₋₉)-gruppe; einer Hydroxyalkyl(C₁₋₉)gruppe; einer Alkenyl(C₂₋₉)-gruppe; einer Cycloalkyl(C₅₋₁₂)gruppe; einer Aryl(C₆₋₁₈)carbonylgruppe; einer Cycloalkyl(C₅₋₁₂)alkyl(C₁₋₉)gruppe; einer Alkyl(C₁₋₉)carbonylgruppe; einer Alkoxy(C₁₋₉)carbonylalkyl(C₁₋₉)-gruppe; einer α-Naphthylalkylgruppe; einer Halogenalkyl(C₁₋₉)-gruppe; einer Alkyl(C₁₋₉)carbonyloxyalkyl(C₁₋₉)gruppe; einer Cyanoalkyl(C₁₋₉)gruppe; einer Acyl(C₂₋₁₅)gruppe; einer Alkoxy(C₁₋₉)carbonylgruppe; einer Aryloxy(C₆₋₁₈)carbonylgruppe; einer Aryloxy(C₆₋₁₈)alkyl(C₁₋₉)carbonylgruppe; einer Aryl(C₆₋₁₈)-alkoxy(C₁₋₉)carbonylgruppe; einer Alkoxy(C₁₋₉)aryl(C₆₋₁₈)carbonylgruppe; einer Dialkyl(C₁₋₉)aminocarbonylgruppe; einer Dialkyl(C₁₋₉)aminosulfonylgruppe; einer Alkyl(C₁₋₉)aryl(C₆₋₁₈)-sulfonylgruppe; einer Alkyl(C₁₋₉)sulfonylgruppe; einer Dialkyl(C₁₋₉)aminoalkyl(C₁₋₉)gruppe; einer Alkoxy(C₁₋₉)alkyl(C₁₋₉)-gruppe; einer Aryl(C₆₋₁₈)gruppe oder einer Aryl(C₆₋₁₈)alkyl(C₁₋₉)-gruppe, die gegebenenfalls am Arylring mit einem oder mehreren Substituenten substituiert sein kann, die unter einem Halogenatom, einer Alkyl(C₁₋₉)gruppe, einer Nitrogruppe, einer Dialkyl(C₁₋₉)aminogmppe, einer Alkoxy(C₁₋₉)gruppe ausgewählt sind; wobei die beiden Gruppen gemeinsam und mit dem Stickstoffatom der Aminogruppe einen 5- bis 12-gliedrigen Ring bilden können, der gegebenenfalls ein weiteres Heteroatom enthält und gegebenenfalls mit einer Alkyl(C₁₋₉)gruppe substituiert sein kann.

2. Verwendung nach Anspruch 1, wobei die Verbindung(en) der Formel (I) so vorliegen, dass:
· R₅ bedeutet ein Wasserstoffatom;
· R₁ bedeutet:
· ein Wasserstoffatom;
· ein Halogenatom;
· eine Alkyl(C₁₋₉)gruppe;
· eine Alkoxy(C₁₋₉)gruppe;
· eine Nitrogruppe;
· eine Aminogruppe;
· eine Alkyl(C₁₋₉)thiogruppe;
· eine Acyloxy(C₂₋₉)alkoxy(C₁₋₉)gruppe;
· R₂ bedeutet ein Wasserstoffatom; eine Alkyl(C₁₋₉)gruppe; eine Alkoxy(C₁₋₉)gruppe; ein Halogenatom; eine Aminogruppe;
· R₃ bedeutet ein Wasserstoffatom; eine Aminogruppe; ein Halogenatom; eine Aryloxy(C₆₋₁₈)alkoxy(C₁₋₉)gruppe; eine Nitrogruppe; eine Alkoxy(C₁₋₉)gruppe;
· R₄ bedeutet ein Wasserstoffatom; ein Halogenatom; eine Alkyl(C₁₋₉)gruppe; eine Alkoxy(C₁₋₉)gruppe; eine Alkyl(C₁₋₉)thiogruppe;
· R₆ bedeutet ein Wasserstoffatom; ein Halogenatom; eine Alkyl(C₁₋₉)gruppe; eine Alkoxy(C₁₋₉)gruppe; eine Nitrogruppe; eine Aminogruppe; eine Acyloxy(C₂₋₉)alkoxy(C₁₋₉)gruppe;
· R₇ bedeutet ein Wasserstoffatom; ein Halogenatom; eine Alkyl(C₁₋₉)gruppe; eine Alkoxy(C₁₋₉)gruppe;
· R₈ bedeutet ein Wasserstoffatom; ein Halogenatom; eine Alkyl(C₁₋₉)gruppe; eine Alkoxy(C₁₋₉)gruppe; eine Nitrogruppe; eine Aminogruppe;
· R₉ bedeutet ein Wasserstoffatom; eine Alkyl(C₁₋₉)gruppe; eine Alkoxy(C₁₋₉)gruppe;
· R₁₀ bedeutet ein Wasserstoffatom; eine Alkoxy(C₁₋₉)gruppe;
· R₁₁bedeutet ein Wasserstoffatom; ein Halogenatom; eine Alkyl-(C₁₋₉)gruppe; eine Alkoxy(C₁₋₉)gruppe; eine Ar-yl(C₆₋₁₈)gruppe; eine Aminogruppe; eine Acyl(C₂₋₁₈)gruppe; eine Aryl(C₆₋₁₈)-sulfonylgruppe;
· R₁₂ bedeutet ein Wasserstoffatom; eine Alkyl(C₁₋₉)gruppe; eine Alkoxy(C₁₋₉)gruppe; eine Aminogruppe;
· R₁₃ bedeutet ein Wasserstoffatom;
· R₁ und R₂ und/oder R₂ und R₃ und/oder R₆ und R₇ und/oder R₇ und R₈ können gemeinsam und mit den Kohlenstoffatomen, an die sie gebunden sind, eine mono- oder polycarbocyclische Gruppe bilden, worin die Ringe 5- bis 20-gliedrig, aromatisch, kondensiert oder nicht kondensiert sind, wobei ein oder mehrere Kohlenstoffatome durch Sauerstoff, Stickstoff, Schwefel oder Phosphor, die substituiert oder unsubstituiert sind, ersetzt sein können.

3. Verwendung nach Anspruch 1, wobei die Verbindung(en) der Formel (I) unter den folgenden Verbindungen der Formel (I₁) ausgewählt sind: 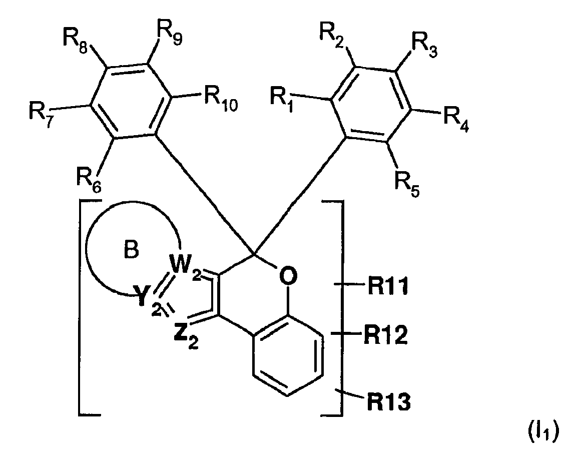 in der Formel:
· Y₂, W₂ und Z₂ bedeuten unabhängig voneinander ein Kohlenstoffatom, ein Stickstoffatom, ein Schwefelatom oder eine zweiwertige Gruppe CR₁₅ oder NR₁₅;
· B bedeutet eine Aryl(C₆₋₁₈)gruppe oder eine 5- bis 12-gliedrige heterocyclische Gruppe, die gesättigt oder ungesättigt, substituiert oder unsubstituiert ist;
· R₁bedeutet ein Wasserstoffatom; ein Halogenatom; eine Alkoxy(C₁₋₉)gruppe; eine Alkyl(C₁₋₉)gruppe; eine Nitrogruppe; eine Alkyl(C₁₋₉)thiogruppe;
· R₂ bedeutet ein Wasserstoffatom; ein Halogenatom; eine Alkyl(C₁₋₉)gruppe; eine Alkoxy(C₁₋₉)gruppe;
· R₃ bedeutet ein Wasserstoffatom; ein Halogenatom; eine Nitrogruppe; eine Aminogruppe; eine Alkoxy(C₁₋₉)gruppe;
· R₄ bedeutet ein Wasserstoffatom; eine Alkoxy(C₁₋₉)gruppe; eine Alkyl(C₁₋₉)gruppe; eine Alkyl(C₁₋₉)thiogruppe;
· R₅, R₁₀ und R₁₃ bedeuten ein Wasserstoffatom;
· R₆ bedeutet ein Wasserstoffatom; ein Halogenatom; eine Nitrogruppe; eine Alkoxy(C₁₋₉)gruppe; eine Alkyl(C₁₋₉)gruppe; eine Aminogruppe; eine Acyloxy(C₂₋₉)alkoxy(C₁₋₉)gruppe;
· R₇ bedeutet ein Wasserstoffatom; eine Alkoxy(C₁₋₉)gruppe;
· R₈ bedeutet ein Wasserstoffatom; ein Halogenatom; eine Alkoxy(C₁₋₉)gruppe; eine Alkyl(C₁₋₉)gruppe; eine Aminogruppe;
· R₉ bedeutet ein Wasserstoffatom; eine Alkoxy(C₁₋₉)gruppe; eine Alkyl(C₁₋₉)gruppe;
· R₁₁ bedeutet ein Wasserstoffatom; ein Halogenatom; eine Aminogruppe; eine Alkyl(C₁₋₉)gruppe; eine Dialkyl(C₁₋₉)aminocarbonylgruppe; eine Aryl(C₆₋₁₈)carbonylgruppe; eine Aryl(C₆₋₁₈)-sulfonylgruppe; eine Acyl(C₂₋₉)gruppe;
· R₁₂ bedeutet ein Wasserstoffatom; eine Alkyl(C₁₋₉)gruppe; eine Aryl(C₆₋₁₈)aminocarbonylgruppe; eine Acyl(C₂₋₂₀)gruppe; eine Alkoxy(C₁₋₉)carbonylgruppe;
· R₁ und R₂ einerseits und R₆ und R₇ andererseits können gemeinsam und mit den Kohlenstoffatomen, an die sie gebunden sind, einen aromatischen C₆₋₁₈-Ring bilden.

4. Verwendung nach Anspruch 1, wobei die Verbindung(en) der Formel (I) unter einer der folgenden Formeln (I₂) bis (I₆) ausgewählt sind: 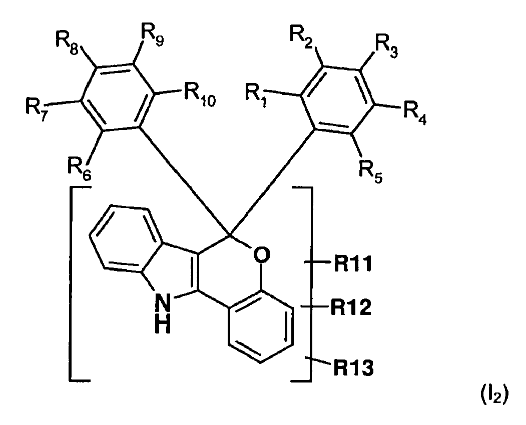 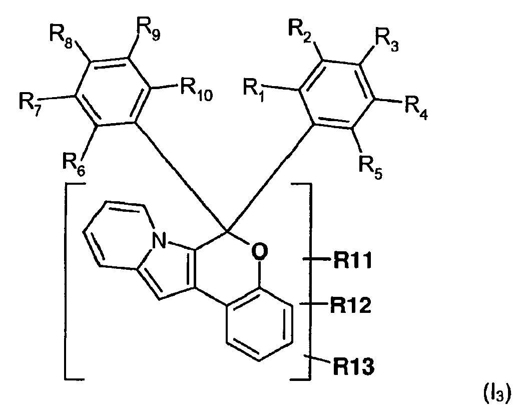 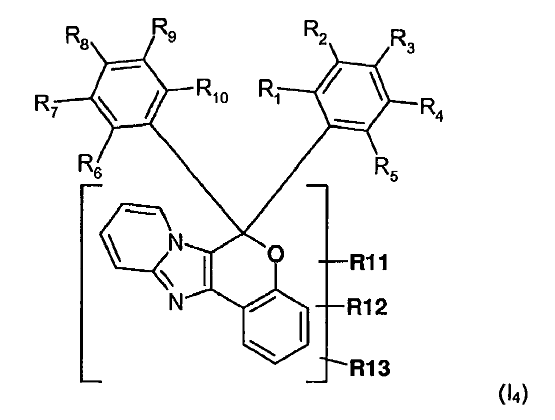 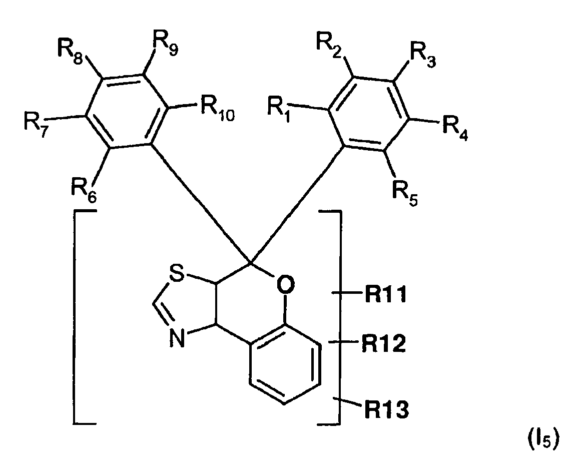 oder 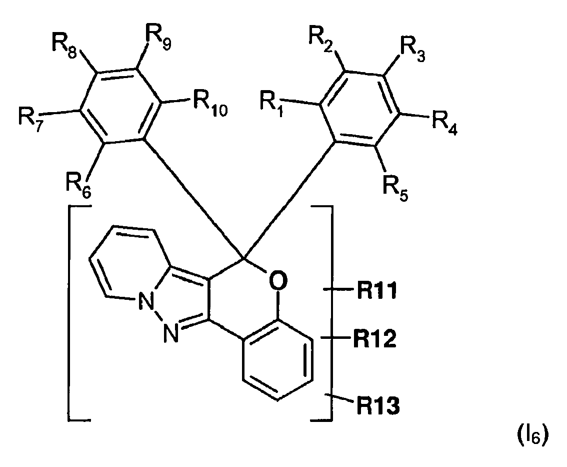 in den Formeln:
· R₁ bedeutet ein Wasserstoffatom; ein Halogenatom; eine Alkoxy(C₁₋₉)gruppe; eine Alkyl(C₁₋₉)gruppe; eine Nitrogruppe; eine Alkyl(C₁₋₉)thiogruppe;
· R₂ bedeutet ein Wasserstoffatom; ein Halogenatom; eine Alkyl(C₁₋₉)gruppe; eine Alkoxy(C₁₋₉)gruppe;
· R₃ bedeutet ein Wasserstoffatom; ein Halogenatom; eine Nitrogruppe; eine Aminogruppe; eine Alkoxy(C₁₋₉)gruppe;
· R₄ bedeutet ein Wasserstoffatom; eine Alkoxy(C₁₋₉)gruppe; eine Alkyl(C₁₋₉)gruppe; eine Alkyl(C₁₋₉)thiogruppe;
· R₅, R₁₀ und R₁₃ bedeuten ein Wasserstoffatom;
· R₆ bedeutet ein Wasserstoffatom; ein Halogenatom; eine Nitrogruppe; eine Alkoxy(C₁₋₉)gruppe; eine Alkyl(C₁₋₉)gruppe; eine Aminogruppe; eine Acyloxy(C₂₋₉)alkoxy(C₁₋₉)gruppe;
· R₇ bedeutet ein Wasserstoffatom; eine Alkoxy(C₁₋₉)gruppe;
· R₈ bedeutet ein Wasserstoffatom; ein Halogenatom; eine Alkoxy(C₁₋₉)gruppe; eine Alkyl(C₁₋₉)gruppe; eine Aminogruppe;
· R₉ bedeutet ein Wasserstoffatom; eine Alkoxy(C₁₋₉)gruppe; eine Alkyl(C₁₋₉)gruppe;
· R₁₁ bedeutet ein Wasserstoffatom; ein Halogenatom; eine Aminogruppe; eine Alkyl(C₁₋₉)gruppe; eine Dialkyl(C₁₋₉)aminocarbonylgruppe; eine Aryl(C₆₋₁₈)carbonylgruppe; eine Aryl(C₆₋₁₈)-sulfonylgruppe; eine Acyl(C₂₋₉)gruppe;
· R₁₂ bedeutet ein Wasserstoffatom; eine Alkyl(C₁₋₉)gruppe; eine Aryl(C₆₋₁₈)aminocarbonylgruppe; eine Acyl(C₂₋₂₀)gruppe; eine Alkoxy(C₁₋₉)carbonylgruppe;
· R₁ und R₂ einerseits und R₆ und R₇ andererseits können gemeinsam und mit den Kohlenstoffatomen, an die sie gebunden sind, einen aromatischen C₆₋₁₈-Ring bilden.

5. Verwendung nach einem der vorhergehenden Ansprüche, wobei die Verbindung der Formel (I) unter den folgenden Verbindungen ausgewählt ist:

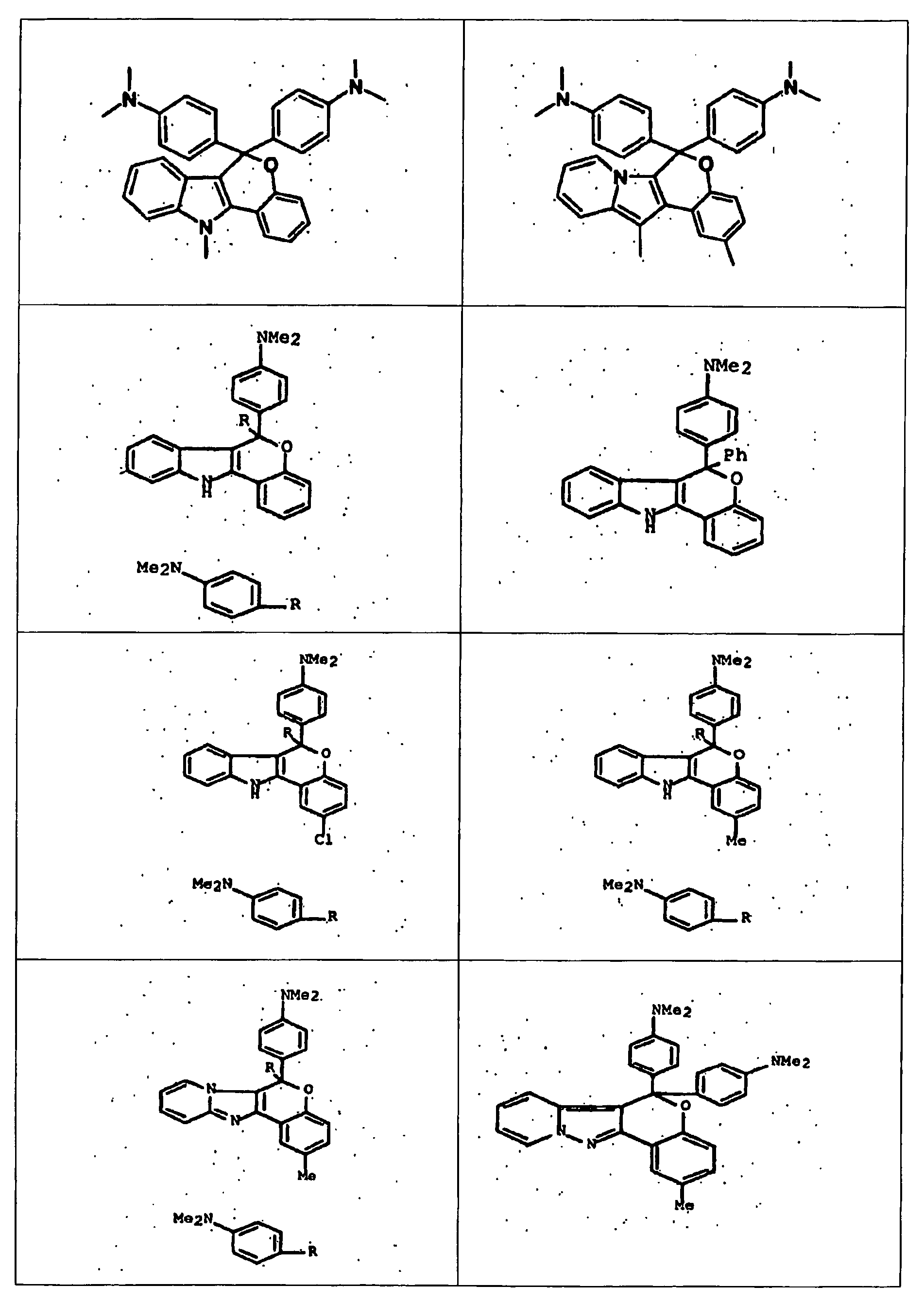

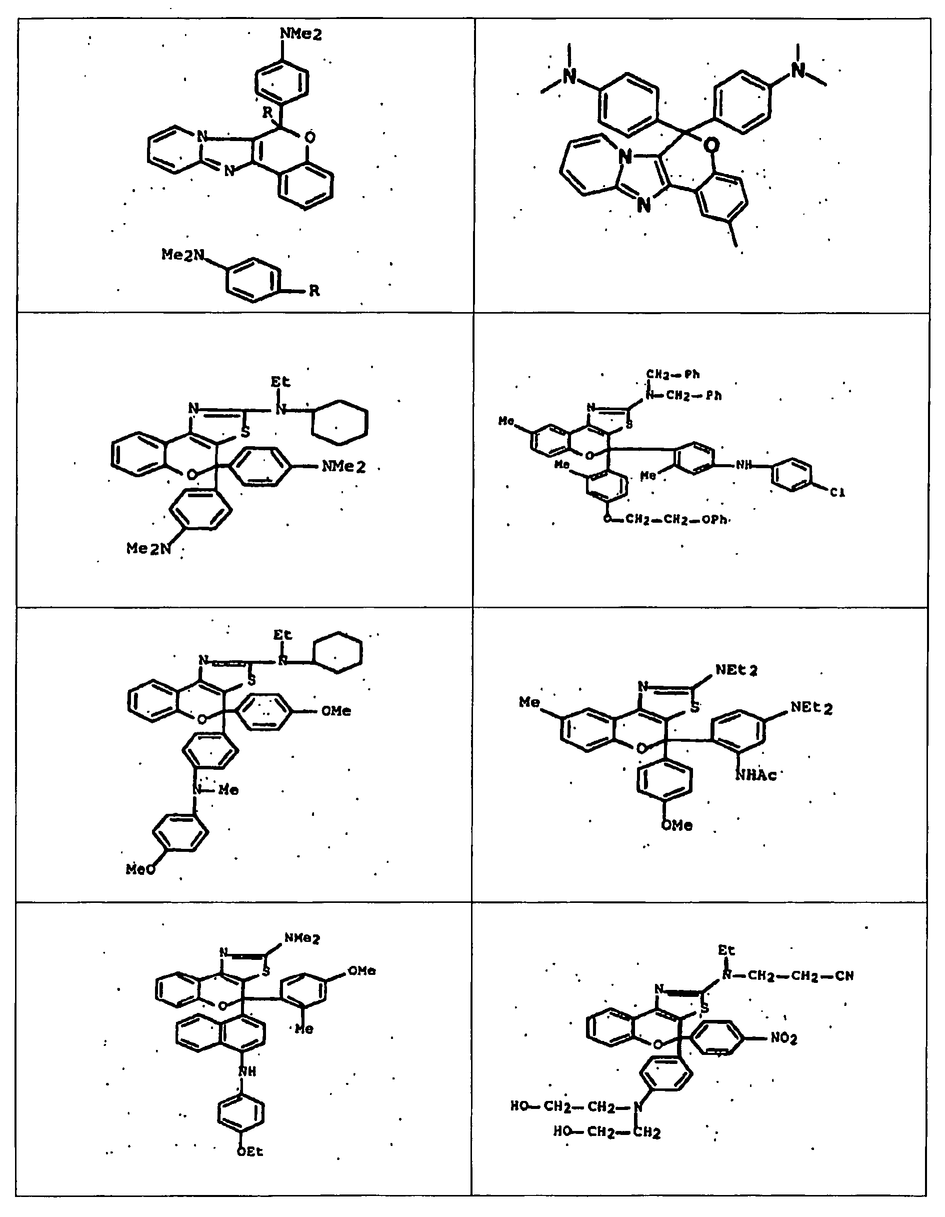

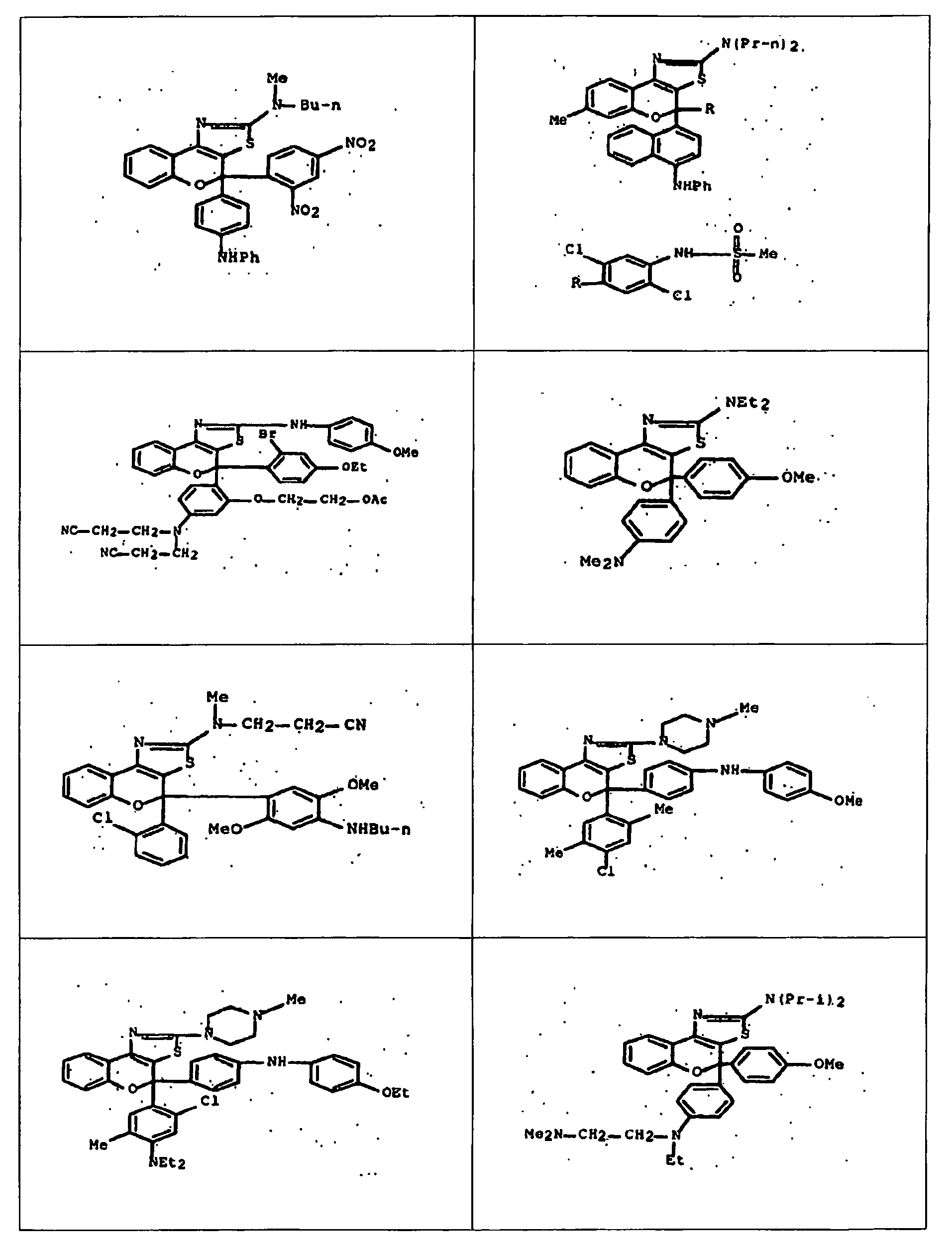

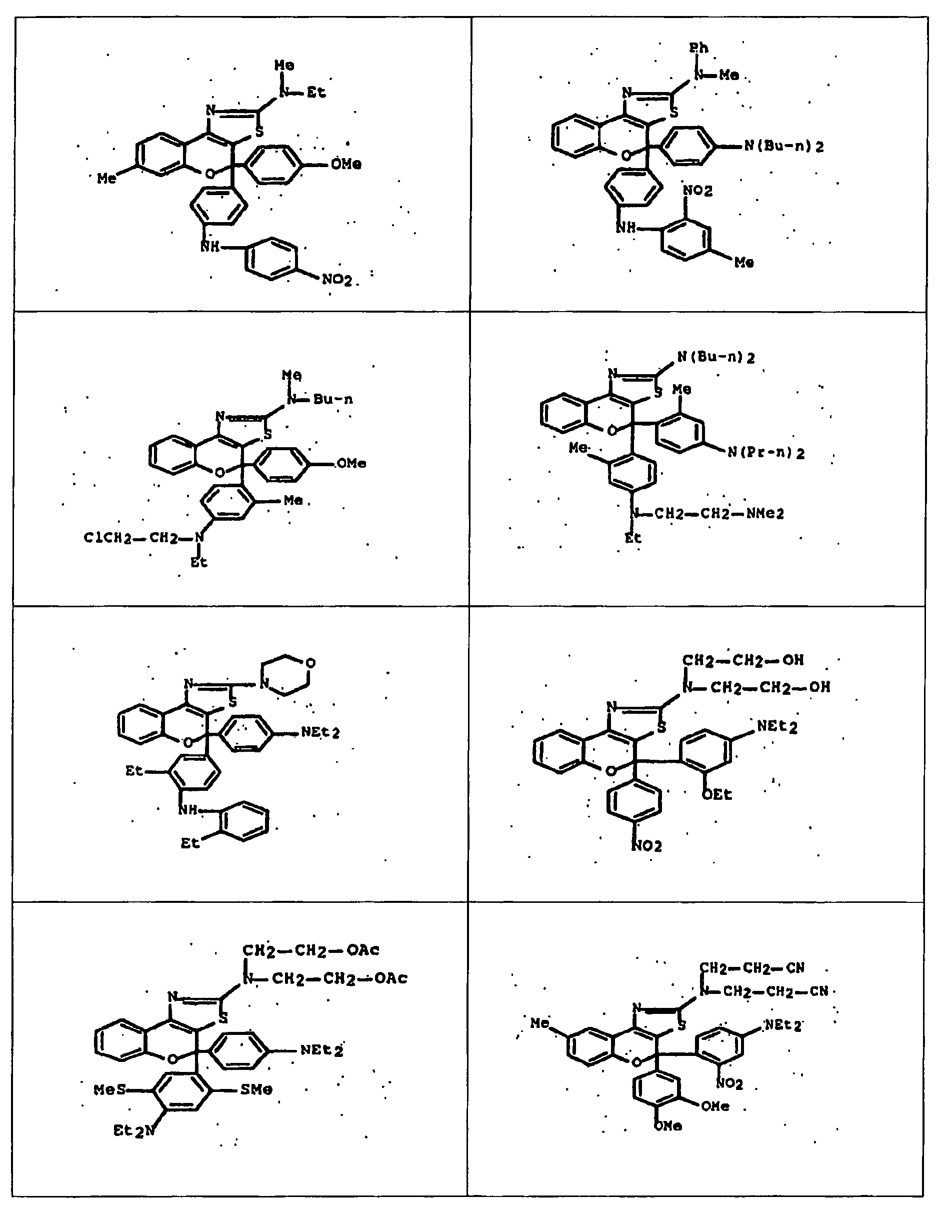

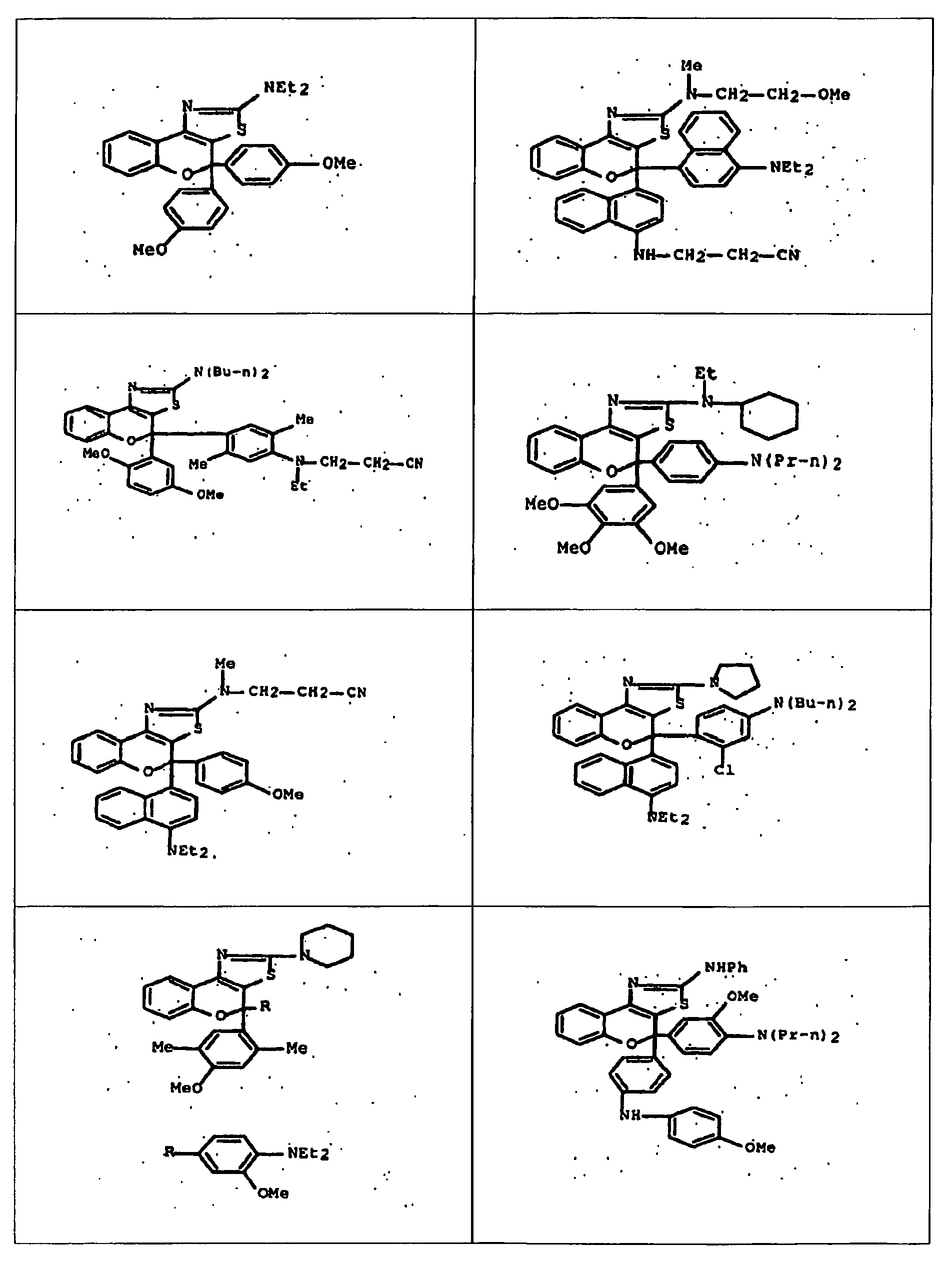

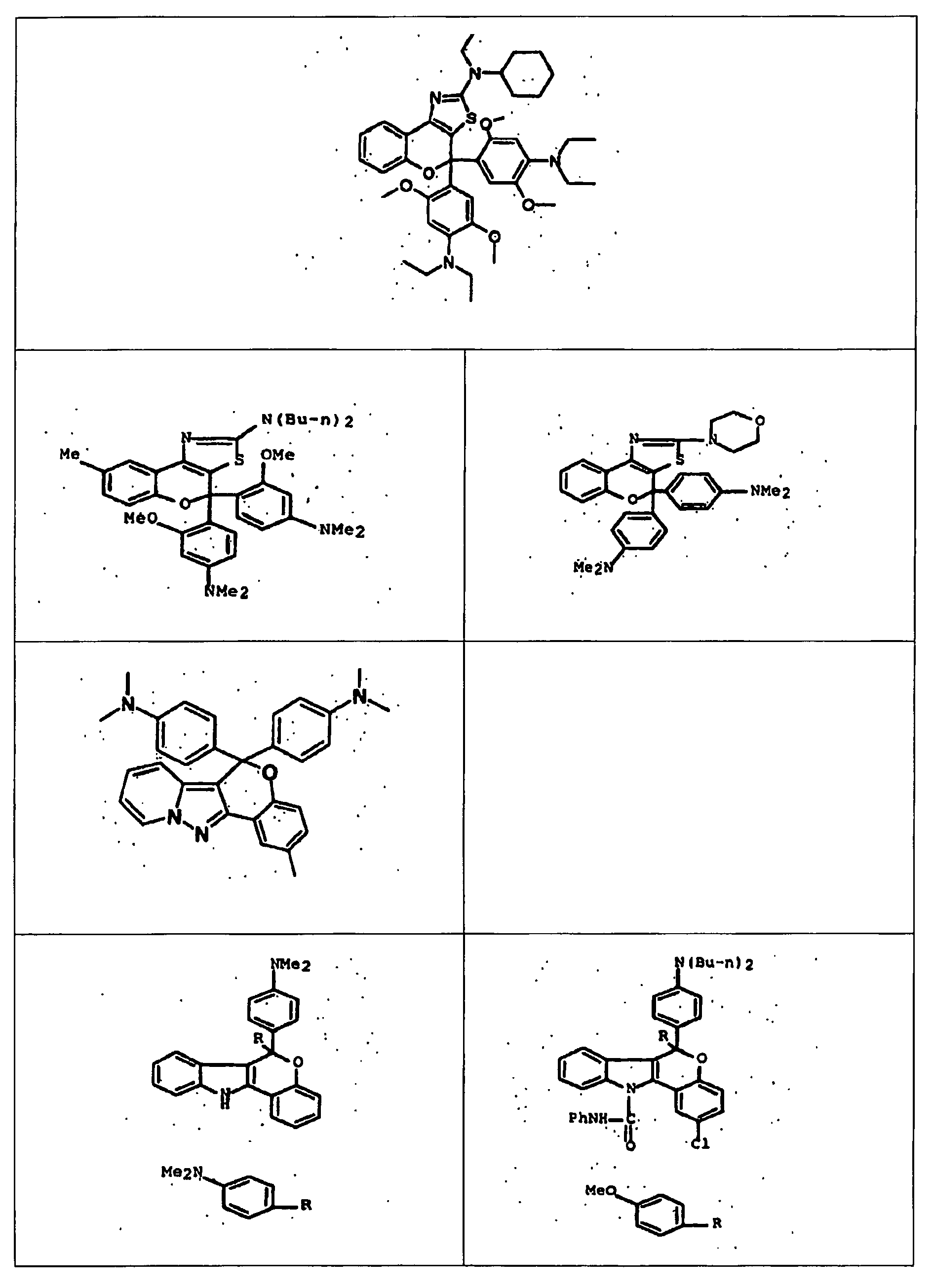

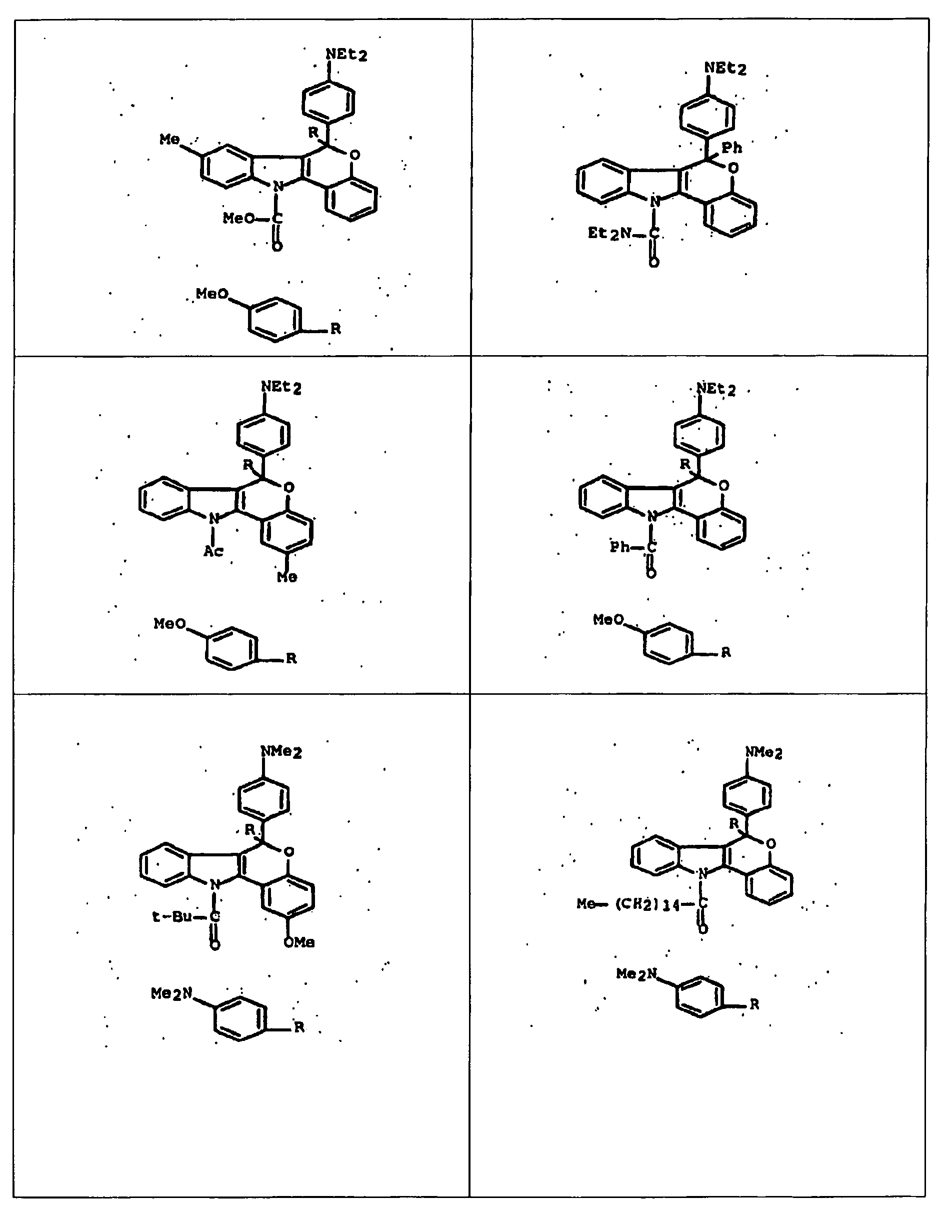

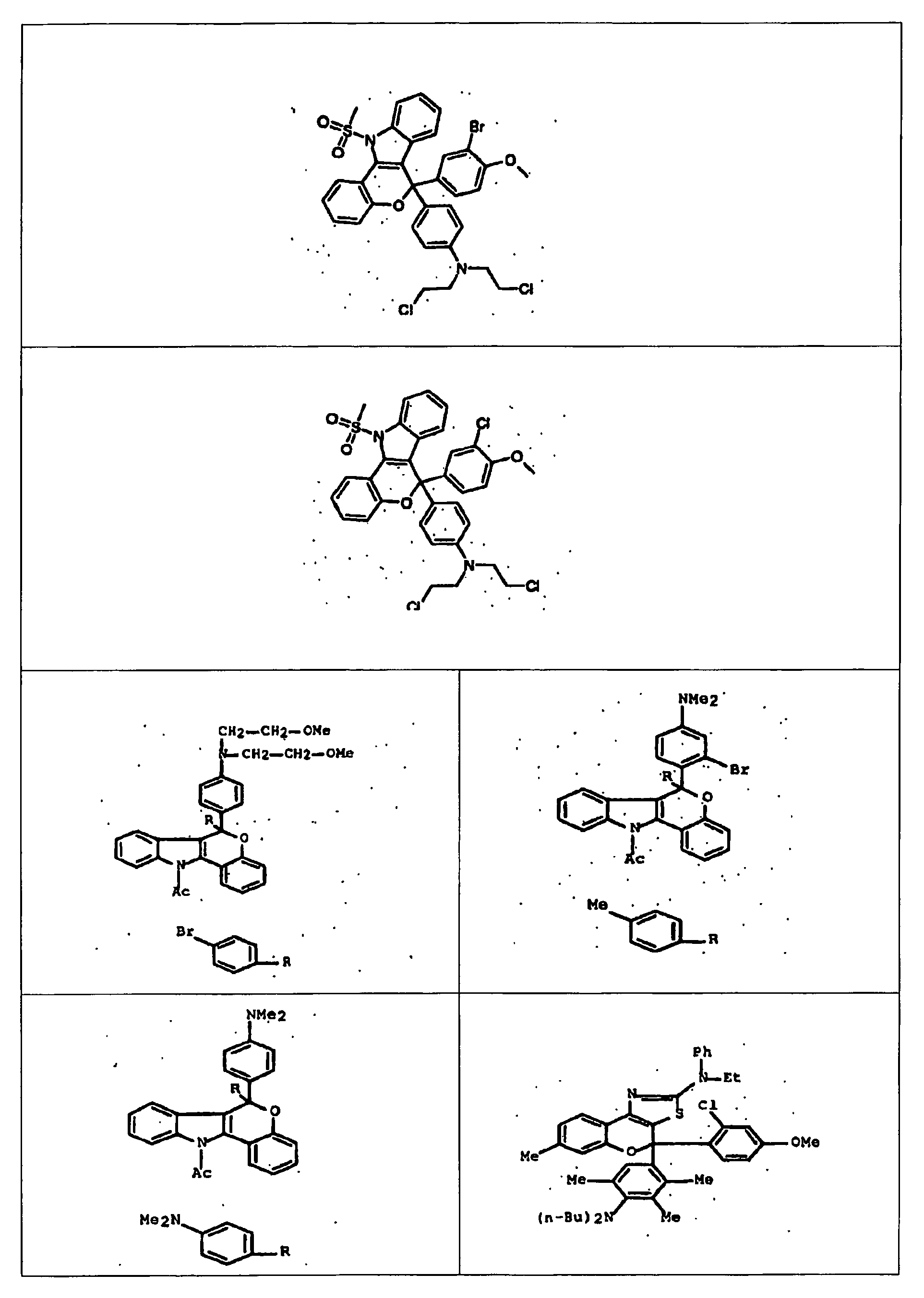

6. Verwendung nach einem der vorhergehenden Ansprüche, wobei die Verbindungen, die unter den Verbindungen der Formel (I), den Farbstoffen, die den Verbindungen der Formel (I) entsprechen und bei denen der Ring H geöffnet ist, und ihren Additionssalzen ausgewählt sind, 0,001 bis 10 Gew.-%, bezogen auf das Gesamtgewicht der Zusammensetzung, ausmachen.

7. Verwendung nach einem der vorhergehenden Ansprüche, wobei das zum Färben geeignete Medium aus Wasser oder mindestens einem organischen Lösungsmittel oder einem Gemisch aus Wasser und mindestens einem organischen Lösungsmittel besteht.

8. Verwendung nach Anspruch 7, wobei das zum Färben geeignete Medium aus Wasser oder einem Gemisch aus Wasser und mindestens einem organischen Lösungsmittel besteht und der pH-Wert im Bereich von 3 bis 12 liegt.

9. Verfahren zur Behandlung von Keratinfasern, bei dem die folgenden Schritte durchgeführt werden:
a) Aufbringen einer Farbmittelzusammensetzung, wie sie in einem der Ansprüche 1 bis 8 definiert ist, während einer ausreichenden Einwirkzeit, wobei der pH-Wert in Abhängigkeit von der gewünschten Färbung mit Hilfe eines Ansäuerungsmittel oder eines Alkalisierungsmittels eingestellt wird;
b) gegebenenfalls Modifikation der Färbung der Keratinfasern mit Hilfe eines Ansäuerungsmittel oder einer Alkalisierungsmittels, das auf die Keratinfasern aufgebracht wird.

10. Verfahren nach Anspruch 9, bei dem das Ansäuerungsmittel oder Alkalisierungsmittel in dem ersten Schritt vor dem Auftragen auf die Keratinfasern mit der Farbmittelzusammensetzung vermischt wird.

11. Verfahren nach Anspruch 9, bei dem das Ansäuerungsmittel oder Alkalisierungsmittel in dem ersten Schritt vor oder nach der Farbmittelzusammensetzung auf die Keratinfasern aufgetragen wird.

12. Verfahren nach einem der Ansprüche 9 bis 11, bei dem das Ansäuerungsmittel unter den anorganischen Säuren, wie Salzsäure, Salpetersäure oder Schwefelsäure, oder den organischen Säuren ausgewählt ist, wie den Verbindungen, die mindestens eine Carboxyfunktion, Sulfonsäurefunktion, Phosphonsäurefunktion oder Phosphorsäurefunktion aufweisen.

13. Verfahren nach einem der Ansprüche 9 bis 12, bei dem das Alkalisierungsmittel ausgewählt ist unter:
- basischen Aminosäuren;
- Carbonaten oder Bicarbonaten von Alkalimetallen oder Erdalkalimetallen;
- Silicaten oder Metasilicaten;
- Verbindungen der folgenden Formel (II):
X(OH)ₙ (II)
worin bedeuten:
· X ein Kaliumion, Lithiumion, Natriumion oder Ammoniumion N⁺R₁₇R₁₈R₁₉R₂₀, wobei die Gruppen R₁₇, R₁₈, R₁₉ und R₂₀, die gleich oder verschieden sind, eine C₂₋₄-Alkylgruppe bedeuten, wenn n 1 ist;
v X ein Magnesiumion oder Calciumion, wenn n 2 ist;
- Verbindungen der folgenden Formel (III): 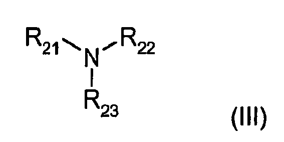 worin bedeuten:
· R₂₁ ein Wasserstoffatom; eine Alkyl(C₁₋₆)gruppe; eine Monohydroxyalkyl(C₁₋₆)gruppe; eine Polyhydroxyalkyl(C₂₋₆)gruppe;
· R₂₂ und R₂₃, die identisch oder verschieden sind, ein Wasserstoffatom; eine Alkyl(C₁₋₆)gruppe; eine Monohydroxyalkyl(C₁₋₆)gruppe; eine Polyhydroxyalkyl(C₂₋₆)gruppe;
- Verbindungen der folgenden Formel (IV): 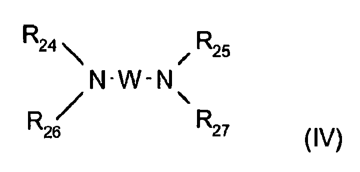 worin bedeuten:
· W einen Propylenrest, der gegebenenfalls mit einer Hydroxygruppe oder einer Alkyl(C₁₋₄)gruppe substituiert ist;
· R₂₄, R₂₅, R₂₆ und R₂₇, die gleich oder voneinander verschieden sind, ein Wasserstoffatom; eine Alkyl(C₁₋₄)gruppe; eine Hydroxyalkyl(C₁₋₄)gruppe.

14. Zusammensetzung, wie sie in einem der Ansprüche 1 bis 8 definiert ist, die ferner mindestens einen grenzflächenaktiven Stoff und mindestens ein Verdickungsmittel enthält.

15. Zusammensetzung nach Anspruch 14, wobei der oder die grenzflächenaktiven Stoffe unter den anionischen, kationischen, nichtionischen, amphoteren oder zwitterionischen grenzflächenaktiven Stoffen oder deren Gemischen ausgewählt sind.

16. Zusammensetzung nach Anspruch 14 oder 15, wobei das oder die Verdickungsmittel assoziative oder nicht assoziative, verdickende Polymere sind.

17. Vorrichtung mit mehreren Abteilungen, die in einer ersten Abteilung eine Zusammensetzung, wie sie in einem der Ansprüche 14 bis 16 definiert ist, und in einer zweiten Abteilung ein Ansäuerungsmittel oder ein Alkalisierungsmittel enthält.

18. Vorrichtung nach Anspruch 17, die in der zweiten Abteilung ein Ansäuerungsmittel und in einer dritten Abteilung ein Alkalisierungsmittel enthält.
